(19) Europäisches Patentamt
European Patent Office
Office européen des brevets

(11) Veröffentlichungsnummer: **0 114 787 B1**

(12) # EUROPÄISCHE PATENTSCHRIFT

(45) Veröffentlichungstag der Patentschrift: **25.09.91**

(51) Int. Cl.[5]: **C07K 5/06**, C07K 5/08, C07K 5/10, A61K 37/02

(21) Anmeldenummer: **84810030.1**

(22) Anmeldetag: **19.01.84**

(54) **Neue Peptidderivate.**

(30) Priorität: **25.01.83 CH 398/83**

(43) Veröffentlichungstag der Anmeldung:
**01.08.84 Patentblatt 84/31**

(45) Bekanntmachung des Hinweises auf die Patenterteilung:
**25.09.91 Patentblatt 91/39**

(84) Benannte Vertragsstaaten:
**AT BE CH DE FR GB IT LI LU NL SE**

(56) Entgegenhaltungen:
**EP-A- 0 000 330**
**EP-A- 0 005 682**
**EP-A- 0 014 815**
**GB-A- 2 053 231**
**GB-A- 2 053 232**

**CHEMICAL ABSTRACTS, Band 79, Nr. 1, 09.Juli 1973, Seite 149, Nr.1568j; Columbus, Ohio, US K.HANTKE et al.: "Covalent binding of lipid to protein. Diglyceride and amide-linked fatty acid at the N-terminal end of the mureinlipoprotein of the Escherichia coli outer membrane"**

**ANGEWANDTE CHEMIE, Band 24, Nr. 10, Okt. 1985, Seiten 872,873; VCH Verlaggsgesellschaft bH, Weinheim, De. G. JUNG et al.: "Increased production of specific antibodies by presentation of the antigen determinants with covalently coupled lipopeptide mitogens"**

(73) Patentinhaber: **CIBA-GEIGY AG**
**Klybeckstrasse 141**
**CH-4002 Basel(CH)**

(72) Erfinder: **Baschang, Gerhard, Dr.**
**Bückenweg 7**
**CH-4126 Bettingen(CH)**
Erfinder: **Hartmann, Albert, Dr.**
**Steingasse 21A**
**CH-7889 Grenzach(DE)**
Erfinder: **Wacker, Oskar, Dr.**
**Löwenbergstrasse 60**
**CH-4059 Basel(CH)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Lipopeptide und insbesondere Verbindungen der Formel I,

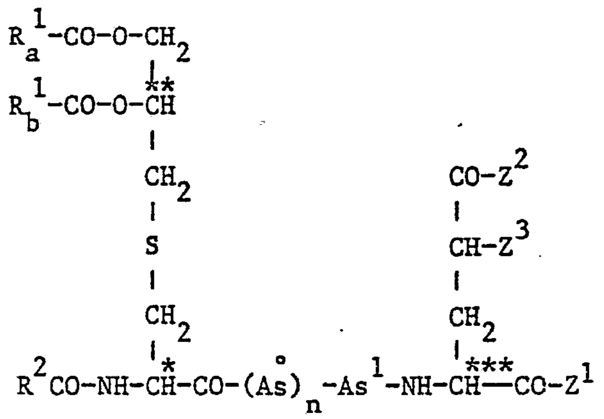

(I)

* = R

** = R oder S

*** = R

worin $R_a^1$ und $_b^1$ unabhängig voneinander je einen aliphatischen oder cycloaliphatisch-aliphatischen, gegebenenfalls durch Sauerstoffunktionen substituierten Kohlenwasserstoffrest mit 7 - 21 C-Atomen, oder der eine der Reste $R_a^1$ -CO und $R_b^1$ -CO Wasserstoff und der andere der Reste $R_a^1$ -CO und $R_b^1$ -CO einen Acylrest, worin $R_a^1$ beziehungsweise $R_b^1$ die obengenannte Bedeutung haben, $R^2$ einen aliphatischen oder cycloaliphatisch-aliphatischen, gegebenenfalls durch Sauerstoffunktionen substituierten Kohlenwasserstoffrest mit 1 - 21 C-Atomen, n = 0 oder 1, $As^°$ einen Rest der Formel -0-Kw-CO- oder -NH-Kw-CO-, worin Kw für einen aliphatischen Kohlenwasserstoffrest mit höchstens 12 C-Atomen steht, $As^1$ eine D- oder L-α-Aminosäure, $Z^1$ und $Z^2$ unabhängig voneinander Hydroxy oder den N-terminalen Rest einer D- oder L-α-Amino-carbonsäure, einer Amino-niederalkansulfonsäure oder eines Peptids mit maximal 6 Aminosäuren aus der Gruppe der D- oder L-α-Amino-carbonsäuren und der Amino-niederalkansulfonsäuren, und $Z^3$ Wasserstoff oder -CO-$Z^4$, worin $Z^4$für Hydroxy oder den N-terminalen Rest einer D- oder L-α-Aminosäure, einer Amino-niederalkansulfonsäure oder eines Peptids mit maximal 6 Aminosäuren aus der Gruppe der D- oder L-α-Amino-carbonsäuren und der Amino-niederalkansulfonsäuren steht, bedeuten, und die Amide und Ester von solchen Verbindungen, die Carboxylgruppen aufweisen, wobei die mit * bzw. ** bzw. *** bezeichneten Asymmetrie-Zentren die angegebenen absoluten Konfigurationen besitzen, und die Konfiguration an einem die Gruppe $Z^3$ tragenden asymmetrischen C-Atom R oder S sein kann, und entsprechende Diastereomerengemische, sowie Salze solcher Verbindungen mit mindestens einer salzbildenden Gruppe und gegebenenfalls Komplexsalze dieser Verbindungen. Die Erfindung betrifft sodann auch Verfahren zur Herstellung dieser Lipopeptide und pharmazeutische Präparate enthaltend eine oder mehrere dieser Verbindungen zusammen mit einem pharmazeutischen Trägermaterial und gegebenenfalls zusammen mit anderen pharmazeutischen Verbindungen. Im folgenden sollen, wenn es sich nicht anders aus dem Zusammenhang ergibt, unter dem Ausdruck "Lipopeptide der Formel I" auch alle ihre oben genannten Derivate, Diastereomerengemische und Salze verstanden werden.

Die Erfindung betrifft insbesondere die obengenannten Verbindungen, worin $R_a^1$ und $R_b^1$ die gleiche Bedeutung haben und je einen aliphatischen oder cycloaliphatisch-aliphatischen , gegebenenfalls durch Sauerstofffunktionen substituierten Kohlenwasserstoffrest mit 7 - 21 C-Atomen, $Z^1$ und $Z^2$ unabhängig voneinander Hydroxy oder den N-terminalen Rest einer D- oder L-α-Aminosäure oder eines Peptids mit maximal 6 D- oder L-α-Aminosäuren und $Z^3$ Wasserstoff oder -CO-$Z^4$, worin $Z^4$ für Hydroxy oder den N-terminalen Rest einer D- oder L-α-Aminosäure oder eines Peptids mit maximal 6 D- oder L-α-Aminosäuren steht, bedeuten.

α-Amino-säuren sind vorzugsweise die in der Natur vorkommenden L-α-Amino-carbonsäuren und ihre Antipoden der D-Reihe. Ohne nähere Angabe ist die L-Konfiguration gemeint. Eine Aminosäure $As^1$ ist vorzugsweise ausgewählt aus der Gruppe, bestehend aus Glycin (Gly), Alanin (Ala), α-Methyl-Alanin (αMeAla), N-Methyl-Alanin (MeAla), Serin (Ser), α-Amino-buttersäure (Abu), Valin (Val) und Leucin (Leu), eine Aminosäure im Rest $Z^1$ vorzugsweise aus der Gruppe Lysin (Lys), Ornithin (Orn), a,a'-Diamino-pimelinsäure (Dpm), Gly, Ala, D-Ala oder D-Asparagin (D-Asn) und eine Aminosäure in den Resten $Z^2$ oder $Z^4$ vorzugsweise aus der Gruppe Lys, Orn, Dpm, Lanthionin (Lan), Gly, Ala oder D-Ala, wobei ein Peptidrest $Z^1$ , $Z^2$ oder $Z^4$ vorzugsweise aus in solcher Weise ausgewählten Aminosäuren, vorzugsweise aus 2 solchen Aminosäuren, aufgebaut ist.

In Uebereinstimmung mit den international anerkannten Nomenklaturregeln bezeichnen in dieser Anmeldung die Abkürzungen für die Aminosäuren, z.B. die obengenannten Abkürzungen, die freie Säure und, wenn nicht anders angegeben, die L-Konfiguration. Die $\alpha$-Aminogruppe ist an der linken Seite der Abkürzung, die Carboxygruppe an der rechten Seite zu denken. Das Fehlen eines H-Atoms in der $\alpha$-Amino-Gruppe wird durch einen links an der Abkürzung für die Aminosäure stehenden Bindestrich, das Fehlen von zwei H-Atomen durch zwei links stehende Bindestriche gekennzeichnet. Das Fehlen einer HO-Gruppe in der Carboxygruppe wird durch einen rechts stehenden Bindestrich ausgedrückt. Substituenten in der Seitenkette von Aminosäuren werden unmittelbar hinter das Aminosäuresymbol in Klammern gesetzt. Somit steht z.B. Palmitoyl-Cys(2[R],3-di-lauroyloxy-propyl)-Ala-D-Glu(OtBu)-$NH_2$ für N-Palmitoyl-S-(2[R],3-di-lauroyloxy-propyl)-cysteinyl-alanyl-D-isoglutaminyl-tert.butylester.

Eine Amino-niederalkansulfonsäure ist insbesondere eine $\omega$-Amino-niederalkansulfonsäure, vorzugsweise eine $\omega$-Amino-$C_{2-3}$-alkansulfonsäure, wie in erster Linie Taurin oder daneben Homotaurin.

Gemäss der Erfindung sind Lipopeptide der Formel (I) bevorzugt, in denen $As^1$ ausgewählt ist aus der Gruppe Gly, Ala, Ser, Abu, Val, MeAla, $\alpha$MeAla und Leu, $Z^1$ und $Z^2$ unabhängig voneinander die Hydroxylgruppe oder den Rest einer in der Natur vorkommenden Aminosäure und $Z^3$ Wasserstoff oder den Rest einer natürlich vorkommenden Aminosäure bedeuten, und ihre Amide und Ester (Verbindungsgruppe IA), und insbesondere solche, worin ein Aminosäurerest $Z^1$ ausgewählt ist aus der Gruppe -Lys, -Orn, -Dpm, -Gly, -D-Ala, -D-Asn und -Ala, und die Aminosäurereste $Z^2$ und $Z^4$ unabhängig voneinander ausgewählt sind aus der Gruppe -Lys, -Orn, -Dpm, -Lan (Lanthionin), -Gly und -Ala, und ihre Ester und Amide (Verbindungsgruppe IB).

In der Europäischen Patentschrift 0 000 330 sind Lipopeptide der Formel

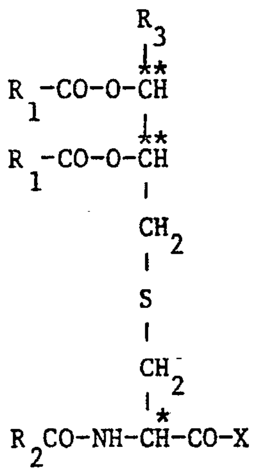

* = R

** = R oder S

beschrieben, worin $R_1$ und $R_2$ je einen gesättigten oder ungesättigten, aliphatischen oder gemischt aliphatisch-cycloaliphatischen, gegebenenfalls auch durch Sauerstoffunktionen substituierten, Kohlenwasserstoffrest mit 11-21 C-Atomen, $R_3$ Wasserstoff oder den Rest $R_1$-CO-O-$CH_2$-, wo $R_1$ die gleiche Bedeutung hat, und X eine peptidisch gebundene natürliche aliphatische Aminosäure mit freier, veresterter oder amidierter Carboxylgruppe, oder eine Aminosäurensequenz von 2-10 natürlichen aliphatischen Aminosäuren, deren terminale Carboxylgruppe in freier, veresterter oder amidierter Form vorliegt, bedeuten. Diese Verbindungen weisen immunpotenzierende Eigenschaften auf und sind auch bei hohen Konzentrationen nicht lymphocytotoxisch. Dabei besteht eine Peptidkette X aus einer beliebig gearteten Sequenz von natürlichen Aminosäuren, während in den durch Abbau aus Lipoproteinen der äusseren Zellwand von Esclierichia coli erhaltenen natürlichen Lipopeptiden (vgl. z.B. Eur. J. Biochem. 34, 284-296 (1973)) nur die Sequenz -Ser-Ser-Asn-Ala-Lys-OH an den "Glycerylcystein"-Teil gebunden ist.

Die in der genannten Europäischen Patentschrift die Peptidsequenz X aufbauenden Aminosäuren sind die natürlichen, der L-Reihe angehörenden, bekannten klassischen Bausteine der Peptide und Proteine. Wie aus obiger Formel (I) ersichtlich, enthält dagegen die entsprechende Sequenz in den Lipopeptiden gemäss der vorliegenden Erfindung als charakteristisches Merkmal eine D-Aminosäure, nämlich die D-Glutaminsäure (D-Glu) oder die D-$\gamma$-Carboxy-glutaminsäure (D-Gla) oder ihre Ester und Amide. Es wurde gemäss der vorliegenden Erfindung gefunden, dass solche Verbindungen eine gegenüber den Verbindungen der genannten Europäischen Patentschrift überlegene pharmakologische Wirkung aufweisen. So stimulieren die Verbindungen der Formel (I) und ihre genannten Derivate, Salze und Komplexsalze bereits bei einer Dosis von 0,01 $\mu$g/ml die in vitro durch Thymidin-Einbau bestimmte Proliferation von B-Lymphocyten der Maus

bis 100fach im Vergleich zu nicht-stimulierten Kontroll-Lymphocyten, während bei den Verbindungen des genannten Europäischen Patentes dies erst bei 0,5 µg/ml der Fall ist und das Ausmass der Stimulation nur das 20-50fache der Kontrollen erreicht. Die neuen Lipopeptide sind gemäss der vorliegenden Erfindung bereits in Konzentrationen aktiv, in denen bisher bekannte B-Zellmitogene (z.B. purified protein derivative = PPD oder Lipoprotein und Lipopolysaccharid von Escherichia coli) noch keine Wirksamkeit zeigen.

Ueberdies sind diese Verbindungen in der Lage, Ratten- und Maus-Alveolarmakrophagenin vitro zu aktivieren, so dass diese nach 24 stündiger Inkubation mit der Substanz Tumorzellen abtöten können. Werden diese Verbindungen inkorporiert in Liposomen (multilamellären Vesikeln) zu den Alveolarmakrophagen gegeben, sind die Verbindungen in der Lage, bereits bei 0,02 µg / 0,2 ml Kultur tumorizidale Makrophagen zu induzieren, während die Verbindungen der genannten Europäischen Patentschrift dies erst bei einer 100fach höheren Dosis vermögen.

Auch in in vivo Modellen zeichnen sich die neuen Verbindungen durch hohe biologische Aktivität aus: NMRI Mäuse werden durch intraperitoneale Injektion von 10 µg bovinem Serumalbumin (BSA) am Tag 0 immunisiert. 8, 18 und 28 Tage später werden Serumproben entnommen und auf ihren Gehalt an anti-BSA Antikörpern mit einer passiven Heamagglutinationstechnik untersucht. In der verwendeten Dosis ist das BSA für die Empfängertiere subimmunogen, d.h. es vermag keine oder nur eine geringfügige Produktion von Antikörpern auszulösen. In diesem Test sind nun die Verbindungen gemäss der Erfindung in der Lage, bei intraperitonealer Applikation von 0,1 mg/kg (appliziert am Tage der Immunisierung), die Antikörperproduktion gegen BSA signifikant zu steigern. Besonders hervorzuheben gegenüber den in der genannten Europäischen Patentschrift beschriebenen Lipopeptiden ist die gute Wirksamkeit der neuen Verbindungen bei s.c. Applikation, wo sie bereits bei 5 mg/kg wirksam sind.

Die neuen Lipopeptide gemäss der vorliegenden Erfindung sind wenig toxisch: auch 5malige intraperitoneale Applikation in einer Dosis von 10 mg/kg/Tag an fünf aufeinanderfolgenden Tagen werden von der Maus anscheinend symptomlos vertragen und bei subkutaner Verabreichung sind die Verbindungen bis zu Dosen von 300 mg/kg untoxisch. Da die für die Immunstimulation benötigten Dosen sehr gering sind, ist die therapeutische Breite der neuen Verbindungen sehr gross.

Die neuen Lipopeptide gemäss der vorliegenden Erfindung können als Adjuvantien in Mischung mit Impfstoffen dazu benützt werden, den Impferfolg zu verbessern und den durch humorale Antikörper und/oder zelluläre Immunität vermittelten Infektschutz gegenüber bakteriellen, viralen oder parasitären Erregern zu verbessern.

Die neuen Lipopeptide können dazu benützt werden, Immunreaktionen bei Mensch und Tier zu fördern. Die Verbindungen eignen sich demnach besonders für die Stimulation der körpereigenen Abwehr, z.B. bei Krebs, chronischen und akuten Infektionen oder bei selektiven (antigenspezifischen) immunologischen Defekten, sowie bei angeborenen, aber auch bei erworbenen allgemeinen (d.h. nicht antigenspezifischen) immunologischen Defektzuständen, wie sie im Alter, im Verlauf schwerer Primärerkrankungen und vor allem nach Therapie mit ionisierenden Strahlen oder mit immunosuppressiv wirkenden Pharmaka auftreten. Die genannten Verbindungen können auch in Kombination mit Antibiotika, Chemotherapeutika oder anderen Stoffen verabreicht werden, um immunologischen Schädigungen entgegenzuwirken. Schliesslich sind die beschriebenen Verbindungen auch zur allgemeinen Prophylaxe von Infektionskrankheiten bei Mensch und Tier geeignet.

In den Acylresten $R_a^1$ CO-, $R_b^1$ CO- und $R^2$CO- der Verbindungen der Formel I und ihrer Derivate sowie der anderen oben hervorgehobenen Verbindungs-gruppen, insbesondere der Gruppen (IA) und (IB), sind $R_a^1$ , $R_b^1$ und $R^2$ gesättigte oder ungesättigte, aliphatische oder cycloaliphatisch-aliphatische, gegebenenfalls auch durch Sauerstoffunktionen substituierte, Kohlenwasserstoffreste mit 7 - 21 bzw. (im Falle von $R^2$) 1 - 21 C-Atomen, d.h. die Acylreste leiten sich von gesättigten oder ungesättigten, aliphatischen oder cycloaliphatisch-aliphatischen , gegebenenfalls im Kohlenwasserstoffrest oxygenierten Carbonsäuren mit 8 - 22, bzw. 2 - 22, vorzugsweise mit 8 - 16 bzw. (im Falle von $R^2$-CO) 2 - 16, C-Atomen im aliphatischen Teil ab. Als solche sind z.B. die gesättigten oder ungesättigten Fettsäuren mit 2-22 C-Atomen, insbesondere mit gerader, d.h. unverzweigter, Kohlenstoffkette, zu nennen, wie Essigsäure, Propionsäure, Oenanthsäure, Buttersäure, Capronsäure, Caprylsäure, Pelargonsäure, Caprinsäure, Undecylsäure, Laurinsäure, Myristinsäure, Palmitinsäure, Margarinsäure, Stearinsäure, Arachinsäure, Behensäure, Oelsäure, Elaidinsäure, Linolsäure, α-und β-Eläostearinsäure, Stearolsäure, α-Linolensäure, ferner unter den cycloaliphatisch-aliphatischen Säuren der soeben genannten Art, in denen die Kohlenstoffkette an beliebiger Stelle durch einen Cycloalkyl- oder Cycloalkenyl-Ring mit vorzugsweise 3 - 8 C-Atomen substituiert oder durch einen Cycloalkylenoder Cycloalkenylen-Rest von 3 - 8 C-Atomen unterbrochen ist, z.B. die Dihydrosterkulsäure, Malvalsäure, Hydrocarpussäure und Chaulmoograsäure. Oxygenierte Säuren dieses Typus, welche ebenfalls für die Acylreste $R_a^1$ CO, $R_b^1$ CO und $R^2$CO in Betracht kommen, sind z.B. die durch Epoxydierung der oben genannten olefinischen Fettsäuren und cycloaliphatischaliphatischen Säuren sich ergebenden, z.B. die

δ,ι-Epoxystearinsäure, ferner Derivate der oben genannten Säuren, die z.B. eine oder mehrere Hydroxygruppen aufweisen, wie z.B. die Ricinolsäure.

Bevorzugterweise hat der Rest $R^2$ 7 - 21 C-Atome.

In der Formel (I) können die Gruppen $R_a^1$ , $R_b^1$ und R miteinander identisch oder voneinander verschieden sein.

Insbesondere sind Verbindungen gemäss Formel (I) und ihre Derivate und die anderen hervorgehobenen Verbindungsgruppen, insbesondere die Gruppen (IA) und (IB) bevorzugt, in denen $R_a^1$ und $R_b^1$ von $R^2$ verschieden sind, insbesondere solche, worin $R_a^1$ CO-, $R_b^1$ CO- und $R^2$CO- unabhängig voneinander Capryloyl, Caprinoyl, Lauroyl, Myristoyl, Palmitoyl oder Stearoyl bedeuten.

Eine weitere bevorzugte Gruppe von Verbindungen gemäss der Erfindung stellen die Ester der terminale und/oder Seitenketten-Carboxylgruppen enthaltenden Lipopeptide dar und unter diesen sind wiederum die Ester der oben näher spezifizierten Untergruppen, insbesondere der Verbindungsgruppen (IA) und (IB), hervorzuheben. Solche Ester leiten sich insbesondere von gegebenenfalls substituierten aliphatischen, araliphatischen, aromatischen oder heterocyclischen Alkoholen, insbesondere von niederaliphatischen Alkoholen mit 1-7 C-Atomen, wie Methyl-, Aethyl-, n-Propyl-, Isopropylalkohol oder den Butylalkoholen, ab. Substituenten sind vorzugsweise freie, veresterte oder verätherte Hydroxygruppen, wobei sich veresterte Gruppen vorzugsweise von Carbonsäuren mit 1-12 C-Atomen, vorzugsweise von aliphatischen Carbonsäuren mit 1-7 C-Atomen ableiten, und verätherte Gruppen sich von aliphatischen Alkoholen mit 1-7 C-Atomen ableiten und diese Gruppen sich in beliebiger Stellung, z.B. in α-, β- oder γ-Stellung zur Alkoholfunktion, befinden können. Araliphatische Alkohole sind insbesondere monocyclisch-niederaliphatische Alkohole mit 1-7 C-Atomen im aliphatischen Teil, wie z.B. Benzylalkohol. Aromatische Ester sind insbesondere solche von monocyclischen, vorzugsweise substituierten Phenolen, z.B. von nieder-Alkoxy-, insbesondere p-nieder-Alkoxy-, nieder-Alkylamino, insbesondere p-nieder-Alkylamino- oder -Dialkylaminophenolen, wobei nieder-Alkyl wiederum Gruppen mit 1 - 7 C-Atomen bedeutet, oder Halogen-phenolen, insbesondere p-Halogen-phenolen, wie Bromphenol, oder
schliesslich von durch eine $C_1$-$C_7$-Alkylgruppe, z.B. in p-Stellung, substituiertem Phenol. Heterocyclische Alkohole sind z.B. Tetrahydrofuranol oder Tetrahydropyranol. Estergruppen sind so z.B. Acetoxy, Propionyloxy, Butyryloxy oder Pivaloyloxy, Aethergruppen z.B Methoxy, Aethoxy, Propoxy oder Butoxy. Dabei können in der Alkoholkomponente der Estergruppe ein oder mehrere derartige Substituenten vorhanden sein, d.h. die betreffenden Estergruppen leiten sich z.B. von mehrwertigen Alkoholen oder ihren Halbestern oder Halbäthern mit den oben genannten Ester- bzw. Aethergruppen, ab. So können sich die Ester insbesondere von Acyloxymethanolen, wie z.B. von Alkanoyloxymethylalkoholen mit 1 - 7 C-Atomen in der Acylgruppe, z.B. von Pivaloyloxymethanol, Propylenglykol oder Glycerin, oder $C_{3-8}$-Cycloalkylcarbonyloxymethylalkoholen, oder ihren genannten Halbestern oder Halbäthern ableiten.

Es können sämtliche Carboxylgruppen der Peptidkette oder nur einzelne in veresterter Form vorliegen. Man kann somit je nach der Natur der Substituenten $Z^1$-$Z^3$ in Formel I, z.B. Mono-, Di-, Tri-, Tetra-, Penta-, Hexa- (im Falle, dass $Z^1 = Z^2 = Z^3$ = Dpm-OAcyl)oder Nonaester (im Falle, das $Z^1 = Z^2 = Z^3$ = Dpm-Dpm-OAcyl) herstellen. Vorzugweise stellt man Verbindungen her, in denen jeweils die α-Carboxylgruppe der betreffenden Aminosäure verestert ist.

Von den Amiden der Verbindungen gemäss Formel (I), wobei sowohl die terminalen oder die Seitenketten-Carboxylgruppen in der Peptidkette in amidierter Form vorliegen können, sind insbesondere die unsubstituierten Amide hervorzuheben, wobei man auch in diesem Falle Mono-, Di-, Tri-, Tetra-, Penta-, Hexa- und Nonaamide herstellen kann. Wie im Falle der Ester sind jene Amide bevorzugt, in denen jeweils die α-Carboxylgruppe amidiert ist. Ausser den unsubstituierten Amiden kommen substituierte in Betracht, die sich insbesondere von niederaliphatischen, cyclischen oder a-cyclischen, primären oder sekundären Aminen ableiten, in erster Linie von solchen, in denen die substituierenden Alkylreste je 1-7 C-Atome bzw. der substituierende Alkylenrest (im Falle der cyclischen Basen) 2-6 C-Atome aufweisen, wie z.B. Methylamin, Aethylamin, Diäthylamin, Propylamin, Isopropylamin, n-Butylamin, oder von Pyrrolidin, Piperidin oder Piperazin. Bevorzugt sind wiederum die eben hervorgehobenen Amide aus den spezifizierten Verbindungsgruppen, insbesondere den Gruppen (IA) und (IB).

Der oben im Zusammenhang mit der Erläuterung von As° erwähnte Kohlenwasserstoffrest Kw ist ein vorzugsweise unsubstituierter Alkylenrest mit vorzugsweise 2-6 C-Atomen, der gerade oder insbesondere verzweigt ist, wie ein Alkylidenrest, z.B. Methylen, Di-, Tri- oder Tetramethylen- und insbesondere Aethyliden, 2-Propyliden, 2,2-Dimethyläthyliden, 2-Butyliden, 3,3-Dimethylpropyliden, 2-Methyläthyliden, 2-Aethyläthyliden, Pentyliden. Der Rest -NH-Kw-CO ist im Falle Kw = Alkyliden der Rest von α-Aminosäuren, wie den natürlichen Aminosäuren Glycin, Alanin, α-Aminobuttersäure, Valin, Norvalin, Leucin, Isoleucin, Norleucin, umfasst aber auch die entsprechenden Verbindungen der D-Reihe, wie z.B. D-Alanin. Beim Rest -O-Kw-CO- für As° handelt es sich um die Reste von entsprechenden Oxycarbonsäuren, insbesondere von α-

Oxycarbonsäuren, so z.B. den Rest der Glykolsäure und der Milchsäure. Auch in diesem Fall können sowohl Reste, die sich von der L- wie von der D-Reihe ableiten als Rest As° vorhanden sein, z.B. der Rest der L- oder D-Milchsäure. Bevorzugt sind Verbindungen der oben spezifizierten Verbindungsgruppen, insbesondere der Gruppen (IA) und (IB), mit den oben hervorgehobenen Beispielen für die Gruppe As°.

Die neuen Lipopeptide gemäss der vorliegenden Erfindung sind dadurch charakterisiert, dass in der Peptidsequenz auf die Aminosäure $As^1$ die D-Glutaminsäure (D-Glu) oder die $\gamma$-Carboxy-D-glutaminsäure (D-Gla), oder deren Amide, wie die Monoamide Glutamin [Glu($NH_2$)], Isoglutamin [Glu-$NH_2$], Gla($NH_2$) oder Gla-$NH_2$ oder die Di- oder Triamide, z.B. Glu($NH_2$)-$NH_2$ oder Gla[($NH_2$)$_2$]-$NH_2$ folgen.

Die terminalen Seitenketten-Carboxlgruppen dieser Aminosäuren können gegebenenfalls insbesondere verestert oder amidiert oder auch durch eine Peptidbindung mit weiteren Aminosäuren gemäss der Definition von $Z^1$ und/oder $Z^2$ bzw. $Z^4$ verknüpft sein.

Bevorzugte Peptidsequenzen in Verbindungen der Formel (I) und ihren Derivaten, sowie in den spezifizierten Verbindungsklassen, insbesondere den Verbindungsgruppen (IA) und (IB), sind solche, in denen n = O ist, d.h. solche, in denen das Verbindungsglied As° zwischen Glycerylcystein-Teil und der Aminosäure $As^1$ fehlt, und unter diesen insbesondere die Sequenzen

-Ala-D-Glu
-Ala-D-Glu-$NH_2$
-Ala-D-Glu($NH_2$)
-Ala-D-Glu-D-Ala-$NH_2$
-Ala-D-Glu($NH_2$)-$NH_2$
-Ala-D-Glu(Ala)-OH
-Ala-D-Glu($NH_2$)-D-Ala-$NH_2$
-Ala-D-Glu(Ala)-$NH_2$
-Ala-D-Glu(Gly-taurin)-$NH_2$

und entsprechende Sequenzen, in denen -Gly-, -Ser-, -Abu-, -Leu-, -$\alpha$MeAla-oder -Val- anstelle des ersten Alanin-Restes stehen, ferner entsprechende Sequenzen mit einem vorangehenden As , welches z.B. der Rest des D-oder L-Alanins, der D- oder L-Milchsäure, der Glykolsäure oder des Glycins sein kann, oder einer der oben für -NH-Kw-CO- bzw. -O-Kw-CO-genannten Reste.

Von besonderem Interesse sind Lipopeptide der Verbindungsgruppe (IB) mit R-Konfiguration am ** Asymmetriezentrum, mit Resten $R^1_a$ -CO- und $R^1_b$ -CO- mit 8 - 16 C-Atomen und Resten $R^2$-CO- mit 2 - 16 C-Atomen und mit den oben angegebenen, bevorzugten Peptidketten. Bevorzugterweise sind die Acylreste $R^1_a$ -CO- und $R^1_b$ -CO- von $R^2$-CO- verschieden und $R^1_a$ -CO-, $R^1_b$ -CO- und $R^2$-CO- stellen insbesondere den Rest der Capryl-, Caprin-, Laurin-, Myristin-, Palmitin-, Stearin- oder der Oelsäure dar.

Besonders wichtige Lipopeptide gemäss der Anmeldung sind

Palmitoyl-Cys(2[R],3-dilauroyloxy-propyl)-Ala-D-Glu-$NH_2$,
Palmitoyl-Cys(2[R],3-dipalmitoyloxy-propyl)-Ala-D-Glu(Ala)-$NH_2$,
Palmitoyl-Cys(2[R],3-dilauroyloxy-propyl)-Ala-D-Glu($NH_2$),
Palmitoyl-Cys(2(R),3-dilauroyloxy-propyl)-Abu-D-Glu-$NH_2$,
Palmitoyl-Cys(2[R],3-dilauroyloxy-propyl)-Ala-D-Glu($NH_2$)-OnBu,
Palmitoyl-Cys(2(R],3-dilauroyloxy-propyl)-Ala-D-Glu(OnBu)-$NH_2$,
Palmitoyl-Cys(2[R],3-dilauroyloxy-propyl)-D-Ala-Ala-D-Glu-$NH_2$,

$$\text{Palmitoyl-Cys(2[R],3-dilauroyloxy-propyl)-O-}\underset{CH_3}{\overset{(D)}{C}}\text{H-CO-Ala-D-Glu-}NH_2,$$

Palmitoyl-Cys(2[R],3-dilauroyloxy-propyl)-O-$CH_2$CO-Ala-D-Glu-$NH_2$,
Palmitoyl-Cys(2[R],3-dilauroyloxy-propyl)-Ala-D-Glu($NH_2$)-O-$CH_2$-CO-C($CH_3$)$_3$,
Palmitoyl-Cys(2[R],3-dilauroyloxy-propyl)-Ser-D-Glu($OCH_3$)-$OCH_3$,
Palmitoyl-Cys(2[R],3-dilauroyloxy-propyl)-Ala-D-Gla-$NH_2$,
Palmitoyl-Cys(2[R],3-dilauroyloxy-propyl)-Abu-D-Glu,
Palmitoyl-Cys(2[R],3-dilauroyloxy-propyl)-Val-D-Glu-$NH_2$,
Palmitoyl-Cys(2[R],3-dilauroyloxy-propyl)-$\alpha$MeAla-D-Glu-$NH_2$,
Palmitoyl-Cys(2[R],3-dilauroyloxy-propyl)-Ala-D-Glu-(Lys-$OCH_3$)-$NH_2$,
Palmitoyl-Cys(2[R],3-dilauroyloxy-propyl)-Ala-D-Glu(Lys-Lys-$OCH_3$)-$NH_2$,
Palmitoyl-Cys(2[R],3-dilauroyloxy-propyl)-Ala-D-Glu(Arg),
dessen Mono- und Dimethylester und dessen Mono- und Diamid,

```
                                                                  (D)
Palmitoyl-Cys(2[R],3-dilauroyloxy-propyl)-Ala-D-Glu(Lys-OCH-COOH),
                                                                  |
                                                                  CH3
```

dessen Mono- und Dimethylester und dessen Mono- und Diamid,
Palmitoyl-Cys(2[R],3-dilauroyloxy-propyl)-Ala-D-Glu(Tly),
(Tly = 4-Thia-Lysin), dessen Mono- und Dimethylester und dessen Mono- und Diamid,
und entsprechende Lipopeptide, in denen anstelle von Palmitoyl am Stickstoff des Cysteinrestes Lauroyl, Caprinoyl, Capryloyl oder Myristoyl vorhanden sind, und diesen und den oben angeführten Lipopeptiden entsprechende Verbindungen, in denen im Diacyloxypropyl-Rest anstelle von Lauroyl-Resten die Reste der Palmitin-, der Capryl-, der Caprin- und der Myristinsäure vorhanden sind, sowie die all diesen Lipopeptiden entsprechenden Verbindungen, in denen die Konfiguration am chiralen Atom des Diacyloxypropylrestes S statt R ist, und entsprechende DiastereomerenGemische von R und S-Verbindungen, wie z.B.

Lauroyl-Cys(2[R,S],3-didecanoyloxy-propyl)-Ala-D-Glu-$NH_2$,
Decanoyl-Cys(2(R,S],3-dilauroyloxy-propyl)-Ala-D-Glu-$NH_2$,
Myristoyl-Cys(2[R,S),3-dilauroyloxy-propyl)-Ala-D-Glu-$NH_2$,
Palmitoyl-Cys(2[R,S),3-didecanoyloxy-propyl)Ala-D-Glu-$NH_2$,
Palmitoyl-Cys(2[R,S],3-didecanoyloxy-propyl)-Abu-D-Glu($OCH_3$)-$OCH_3$,

sowie gegebenenfalls deren unsubstituierte Amide, deren substituierte Amide, die sich von niederen aliphatischen Aminen mit $C_{1-7}$-Alkylresten ableiten, insbesondere von Methylamin oder Aethylamin, oder von Pyrrolidin, Piperidin oder Piperazin, sowie Ester von aliphatischen Alkoholen mit 1 - 7 C-Atomen, ferner Ester, die sich von substituierten ein- und mehrwertigen Alkoholen, wie von $C_{1-7}$-Alkanoyloxymethylalkoholen, $C_{1-7}$-Alkanoyloxyäthylalkoholen, ($C_{3-8}$-Cycloalkyl)-carbonyoxymethylalkoholen oder ($C_{3-8}$-Cycloalkyl)-carbonyloxy-äthylalkoholen oder von den oben genannten substituierten Phenolen ableiten. Als spezifische Ester dieses Typs der Lipopeptide gemäss der Erfindung seien z.B. die Methyl-, Aethyl-, Butyl- und Propylenglykol-Ester der oben angeführten und der in den illustrativen Beispielen beschriebenen spezifischen Lipopeptide erwähnt. Von den erfindungsgemässen Lipopeptiden mit einem N-Acylrest $R^2$-CO, der sich von einer niederen Carbonsäure ableitet, sei z.B. das Acetyl-Cys(2-[R],3-dilauroyloxy-propyl)-Ala-D-Glu-$NH_2$ erwähnt.

Die Erfindung betrifft in allererster Linie die in den Beispielen genannten Verbindungen der Formel I.

Die vorliegenden neuen Lipopeptide sind je nach der Art ihrer Substituenten neutrale, saure oder basische Verbindungen. Falls überschüssige saure Gruppen vorhanden sind, bilden sie Salze mit Basen, wie Ammoniumsalze oder Salze mit Alkali- oder Erdalkalimetallen, z.B. Natrium, Kalium, Calcium oder Magnesium; sind jedoch überschüssige basische Gruppen vorhanden, bilden sie Säureadditionssalze.

Säureadditionssalze sind besonders pharmazeutisch verwendbare, nichttoxische Säureadditionssalze, wie solche mit anorganischen Säuren, z.B. Chlorwasserstoff-, Bromwasserstoff-, Salpeter-, Schwefel- oder Phosphorsäuren, oder mit organischen Säuren, wie organischen Carbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 4-Phenoxy-benzoesäure, 2-Acetoxy-benzoesäure, Embonsäure, Nicotinsäure oder Isonicotinsäure, oder organischen Sulfonsäuren, z.B. Methansulfonsäure, Aethansulfonsäure, 2-Hydroxy-äthansulfonsäure, Aethan-1,2-disulfonsäure, Benzolsulfonsäure, p-Toluolsulfonsäure oder Naphthalin-2-sulfonsäure, ferner auch andere Säureadditionssalze, die z.B. als Zwischenprodukte, z.B. zur Reinigung der freien Verbindungen oder in der Herstellung von anderen Salzen, sowie zur Charakterisierung verwendet werden können, wie z.B. solche mit Pikrin-, Pikrolon-, Flavian-, Phosphorwolfram-, Phosphormolybdän-, Chlorplatin-, Reinecke- oder Perchlorsäure.

Komplexsalze sind die mit Metallsalzen, z.B. mit Schwermetallsalzen, wie Kupfer-, Zink-, Eisen- oder Kobaltsalzen entstehenden Verbindungen. Zur Bildung solcher Komplexe werden vorzugsweise die Phosphate, Pyrophosphate und Polyphosphate dieser Metalle verwendet, gegebenenfalls in Kombination mit sauren organischen Stoffen, z.B. saure Gruppen enthaltenden Polysacchariden, wie Carboxymethylcellulose, Gerbsäure, Polyglutaminsäure oder teilweise hydrolysierte Gelatine, ferner Alkalimetallpolyphosphate wie z.B. "Calgon*N", "Calgon*322", "Calgon*188" oder "Plyron*B 12" (* eingetragene Warenzeichen).

Die neuen Lipopeptide können nach an sich bekannten Methoden hergestellt werden. Nach einem bevorzugten Verfahren werden die Verbindungen der Formel (I), ihre Amide und/oder Ester oder ihre Diastereomeren-Gemische und ihre Salze und Komplexsalze dadurch hergestellt, dass man

a) in einer der Formel (I) entsprechenden Verbindung oder einem Salz derselben, worin die Substituenten die obengenannten Bedeutungen haben mit der Massgabe, dass die Peptidkette

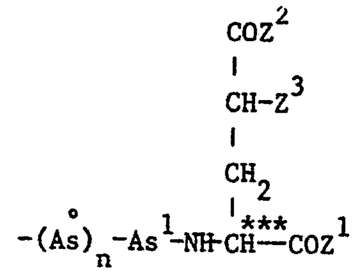

(II)

mindestens eine geschützte funktionelle Gruppe enthält, die Schutzgruppe(n) abspaltet, oder

b) eine Verbindung der Formel I, worin mindestens einer der Reste $R_a^1$ -CO-, $R_b^1$ -CO- und $R^2$-CO für Wasserstoff steht und die übrigen Substituenten die obengenannten Bedeutungen haben mit der Massgabe, dass in diesem Ausgangsmaterial vorhandene freie funktionelle Gruppen mit Ausnahme der an der Reaktion teilnehmenden Hydroxy- und/oder Aminogruppe(n), wenn nötig, in geschützter Form vorliegen, oder ein Salz derselben, mit einer Säure $R_a^1$ -COOH, $R_b^1$ -COOH beziehungsweise $R^2$-COOH oder einem reaktionsfähigen Carbonsäurederivat davon acyliert und vorhandene Schutzgruppen abspaltet, oder

c) eine Amidbindung einer Verbindung der Formel I durch Umsetzung eines entsprechenden Bruchstücks einer Verbindung der Formel I mit einer freien Carboxylgruppe oder eines reaktionsfähigen Säurederivats davon mit einem komplementierenden Bruchstück mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen, wenn nötig, in geschützter Form vorliegen, herstellt und vorhandene Schutzgruppen abspaltet, oder

d) dass man in einer Verbindung der Formel (I), worin zumindestens eine freie Carboxylgruppe vorhanden ist, die freie(n) Carboxylgruppe(n) verestert oder amidiert und/oder in einer Verbindung der Formel (I), worin zumindestens eine Estergruppe vorhanden ist, die Estergruppe(n) verseift, oder

e) eine Verbindung der Formel III,

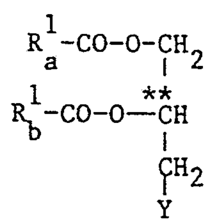

** = R oder S

(III)

worin $R_a^1$ und $R_b^1$ die obengenannten Bedeutungen haben und Y für eine nucleofuge Gruppe steht, mit einer Verbindung der Formel IV,

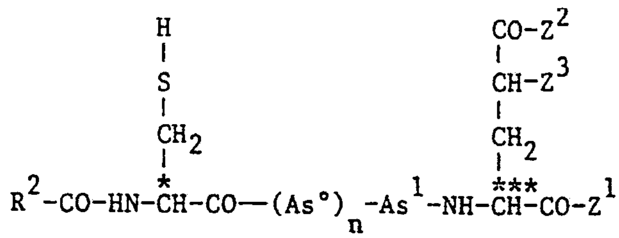

* = R

*** = R

(IV)

worin die Substituenten die obengenannten Bedeutungen haben, wobei freie funktionelle Gruppen mit Ausnahme der an der Reaktion teilnehmenden Mercaptogruppe, wenn nötig, durch leicht abspaltbare Schutzgruppen geschützt sind, oder mit einem reaktionsfähigen Derivat einer Verbindung der Formel IV umsetzt und vorhandene Schutzgruppen abspaltet, oder

(f) eine Verbindung der Formel V,

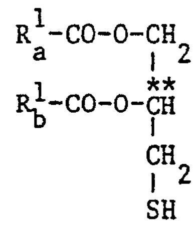

** = R oder S

(V)

worin $R_a^1$ und $R_b^1$ die obengenannten Bedeutungen haben, oder ein reaktionsfähiges Derivat dieser Verbindung mit einer Verbindung der Formel VI,

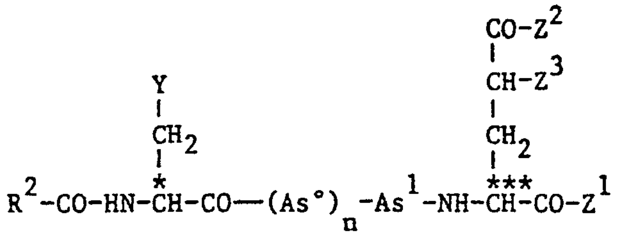

* = R

*** = R (VI)

worin Y für eine nucleofuge Gruppe steht und die übrigen Substituenten die obengenannten Bedeutungen haben, wobei freie funktionelle Gruppen, wenn nötig, in geschützter Form vorliegen, umsetzt und vorhandene Schutzgruppen abspaltet, und, wenn erwünscht, nach Durchführung einer der Verfahrensvarianten a - f) eine erhaltene Verbindung der Formel I mit mindestens einer salzbildenden Gruppe in ein Salz oder Komplexsalz überführt oder ein erhaltenes Salz oder Komplexsalz in die freie Verbindung überführt und, wenn erwünscht, erhaltene Isomerengemische auftrennt.

Verfahren a):

Die Schutzgruppen in den Ausgangsstoffen für das Verfahren gemäss Variante a) sind besonders die für den Schutz von Amino-, Carboxy- oder Hydroxygruppen von der Synthese von Peptiden her bekannten, die z.B. durch Hydrolyse, Reduktion, Aminolyse oder Hydrazinolyse abgespalten werden können.

So sind z.B. Schutzgruppen für Aminogruppen Acyl- oder Aralkylgruppen wie Formyl-, Trifluoracetyl-, Phthaloyl-, Benzolsulfonyl-, p-Toluolsulfonyl-, o-Nitrophenylsulfenyl-, 2,4-Dinitrophenylsulfenylgruppen, gegebenenfalls substituierte, wie z.B. durch Niederalkoxygruppen, besonders o- oder p-Methoxygruppen substituierte Benzyl , oder Diphenyl- oder Triphenylmethylgruppen oder von der Kohlensäure sich ableitende Gruppen, wie gegebenenfalls in den aromatischen Ringen, z.B. durch Halogenatome wie Chlor oder Brom, Nitrogruppen, Niederalkyl- oder Niederalkoxygruppen oder farbgebende Gruppen, z.B. Azogruppen substituierte Arylmethyloxycarbonylgruppen, in denen die Methylengruppe durch einen weiteren Arylrest und/oder einen oder gegebenenfalls zwei niedere Alkylreste substituiert sein kann, wie Benzyl-, Benzhydryl- oder 2-Phenyl-isopropyloxycarbonylgruppen, z.B. Carbobenzoxy, p-Brom- oder p-Chlorcarbobenzoxy, p-Nitrocarbobenzoxy oder p-Methoxycarbobenzoxy, p-Phenylazo-benzyloxycarbonyl und p-(p'-Methoxy-phenylazo)-benzyloxycarbonyl, 2-Tolyl-isopropyloxycarbonyl und insbesondere 2-(p-Biphenylyl)-isopropyloxycarbonyl, sowie aliphatische Oxycarbonylgruppen wie Adamantyloxycarbonyl, Cyclopentyloxycarbonyl, Trichloräthyloxycarbonyl, tert. Amyloxycarbonyl oder vor allem tert.-Butyloxycarbonyl.

Die Aminogruppen können auch durch Bildung von Enaminen, erhalten durch Reaktion der Aminogruppe mit 1,3-Diketonen, z.B. Benzoylaceton, Acetylaceton oder Dimedon, geschützt werden.

Carboxylgruppen sind beispielsweise durch Amid- oder Hydrazidbildung oder durch Veresterung geschützt. Die Amid- und Hydrazidgruppen sind vorzugsweise substituiert, die Amidgruppe z.B. durch die 3,4-Dimethoxybenzyl- oder bis-(p-Methoxyphenyl)-methylgruppe, die Hydrazidgruppe z.B. durch die Benzyloxycarbonyl-, die Trichloräthyloxycarbonylgruppe, die Trifluoracetylgruppe, die Tritylgruppe, die tert.-Butyloxycarbonylgruppe oder die 2-(p-Biphenylyl)-isopropyloxycarbonylgruppe. Zur Veresterung geeignet sind z.B. niedere gegebenenfalls substituierte Alkanole wie Methanol, Aethanol, Cyanmethylalkohol, Benzoylmethylalkohol oder insbesondere tert.-Butanol, ferner Aralkanole wie Arylniederalkanole, z.B. gegebenenfalls durch Niederalkyl- oder Niederalkoxygruppen oder Halogenatome substituierte Benzylalkohole oder Benzhydrole wie Benzhydrol, p-Nitrobenzylalkohol, p-Methoxybenzylalkohol, 2,4,6-Trimethylbenzylalkohol, gegebenenfalls durch elektronenanziehende Substituenten substituierte Phenole und Thiophenole wie Thiophenol, Thiokresol, p-Nitrothiophenol, 2,4,5- und 2,4,6-Trichlorphenol, p-Cyanophenol oder p-Methansulfonylphenol, weiter z.B. N-Hydroxysuccinimid, N-Hydroxyphthalimid, N-Hydroxypiperidin, 8-Hydroxychinolin.

Hydroxygruppen, z.B. in Serin- oder Threoninresten, können z.B. durch Veresterung oder Verätherung geschützt sein.

Als Acylreste bei der Veresterung sind vor allem von der Kohlensäure sich ableitende Reste wie Benzyloxycarbonyl oder Aethyloxycarbonyl geeignet. Zur Verätherung geeignete Gruppen sind z.B. Benzyl-, Tetrahydropyranyl- oder tert.-Butylreste. Ferner eignen sich zum Schutz der Hydroxylgruppen die in Chem. Ber. 100 (1967), 3838-3849 beschriebenen 2,2,2-Trifluor-1-tert.-butyloxycarbonylamino- oder -1-benzyloxycarbonylaminoäthylgruppen (Weygand).

Besondere Schutzgruppen für Carboxygruppen, welche man unter neutralen Bedingungen abspalten kann, sind die in der Deutschen Offenlegungsschrift 27 06 490 beschriebenen Hydrocarbyl-silyl-äthyl-Gruppen, wie z.B. die 2-(Trimethylsilyl)-äthyl-Gruppe.

Eine Mercaptogruppe, wie z.B. in Cystein, kann insbesondere durch S-Alkylierung mit gegebenenfalls substituierten Alkylresten, Thioacetalbildung, S-Acylierung oder durch das Erstellen asymmetrischer Disulfid-Gruppierungen geschützt werden. Bevorzugte Mercaptoschutzgruppen sind z.B. gegebenenfalls im Phenylrest, z.B. durch Methoxy oder Nitro, substituiertes Benzyl, wie 4-Methoxybenzyl, gegebenenfalls im Phenylteil, z.B. durch Methoxy, substituiertes Diphenylmethyl, wie 4,4'-Dimethoxydiphenyl-methyl, Triphenylmethyl, Trimethylsilyl, Benzyl-thiomethyl, Tetrahydropyranyl, Acylaminomethyl, Benzoyl, Benzyloxycarbonyl oder Aminocarbonyl, wie Ethylaminocarbonyl.

Die verfahrengemässe Abspaltung der Schutzgruppen erfolgt in an sich bekannter Weise, z.B. nach üblichen Methoden der Peptidchemie. So können die oben genannten Aminoschutzgruppen, wie Acylschutzgruppen, durch saure Hydrolyse, z.B. mit Trifluoressigsäure, Chlorwasserstoff oder Bromwasserstoff, in einem geeigneten Lösungsmittel, wie einem Ester, z.B. Essigester, oder einem chlorierten aliphatischen Kohlenwasserstoff, wie Chloroform, Methylenchlorid oder Aethylenchlorid abgespalten werden. Im Falle von Sulfenylgruppen kann die Abspaltung auch durch Einwirkung von nucleophilen Reagenzien, z.B. Sulfiten oder Thiosulfiten, erlangt werden. Aralkylgruppen werden vorzugsweise durch katalytische Hydrierung, z.B. mit Palladium-Katalysatoren, wie Palladium/Bariumsulfat, Palladium-Mohr oder aber auch mit Rhodium-Katalysator, entfernt, wobei man die aus der Literatur bekannten Lösungsmittel verwendet, z.B. cyclische Aether, wie Tetrahydrofuran, gegebenenfalls im Gemisch mit andern inerten Lösungsmitteln, wie z.B. einem nieder-aliphatischen Säureamid, wie Dimethylformamid.

Die Abspaltung von Carboxylschutzgruppen kann ebenfalls hydrolytisch (unter den angegebenen neutralen oder milden sauren Bedingungen) durchgeführt werden, z.B. mit den gleichen sauren Mitteln, wie oben für die Entfernung der Aminoschutzgruppen besprochen. Aralkylester, wie z.B. Benzylester, können aber auch durch katalytische Hydrierung, wie im Falle der oben genannten Spaltung von Arylalkylaminen, gespalten werden. Die oben genannte 2-(Trimethylsilyl)-äthyl-Gruppe kann unter neutralen Bedingungen, z.B. durch Einwirkung eines Salzes der Fluorwasserstoffsäure, wie insbesondere des Salzes der Fluorwasserstoffsäure einer quaternären Stickstoffbase, z.B. Tetraäthylammoniumfluorid, in einem geeigneten Lösungsmittel, abgespalten werden.

Eine durch eine geeignete Acylgruppe, eine organische Silylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z.B. durch basische Hydrolyse, eine durch tert.-Niederalkyl oder durch einen 2-oxa- oder 2-thia-aliphatischen oder -cycloaliphatischen Kohlenwasserstoffrest verätherte Hydroxy- bzw. Mercaptogruppe durch Acidolyse, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, freigesetzt werden.

In den Ausgangsstoffen gemäss der Variante a) sind durch Veresterung geschützte Carboxylgruppen insbesondere tert.-Butyloxycarbonyl- oder Benzyloxycarbonyl-Gruppen.

Aminogruppen von Seitenketten, wie von Ornithin oder Lysin, sind in erster Linie durch die tert.-Butyloxycarbonylgruppe (Boc) geschützt. Hydroxygruppen, z.B. in Serin in $As^1$, werden vor allem durch die tert.-Butyläthergruppe geschützt. Die Abspaltung dieser Gruppen erfolgt zweckmässig durch Behandlung mit den genannten sauren Mitteln unter den bekannten Bedingungen, z.B. mit Trifluoressigsäure, bei Zimmertemperatur, so dass man in einem Schritt die Schutzgruppen sowohl der Carboxylgruppen, wie von Aminogruppen und eventuell des Serins entfernen kann.

Verfahren b):

Freie funktionelle Gruppen in dem Ausgangsmaterial, die vorzugsweise in geschützter Form vorliegen, sofern sie nicht an der Reaktion teilnehmen sollen, sind insbesondere Amino-, Mercapto-, Hydroxy- und Carboxygruppen. Die Veresterung mit der freien Carbonsäure findet in Gegenwart eines geeigneten wasserentziehenden Mittels statt. Reaktionsfähige Carbonsäurederivate sind insbesondere die Anhydride, wie z.B. gemischte oder innere Anhydride, z.B. diejenigen mit Halogenwasserstoffsäuren, d.h. die entsprechenden Säurehalogenide, insbesondere Chloride, ferner mit Cyanwasserstoffsäure oder dann diejenigen mit geeigneten Kohlensäurehalbderivaten, wie entsprechenden -halbestern (wie die z.B. mit einem Halogenameisensäure-niederalkyl, wie Chlorameisensäure-äthylester oder -isobutylester, gebildeten gemischten Anhydride) oder mit gegebenenfalls substituierten, z.B. Halogen, wie Chlor, enthaltenden Niederalkancarbonsäuren (wie die mit Pivalinsäurechlorid oder Trichloressigsäurechlorid gebildeten gemischten Anhydride). Innere Anhydride sind z.B. diejenigen von organischen Carbonsäuren, d.h. Ketene, wie Keten oder Diketen, oder diejenigen von Carbamin- oder Thiocarbaminsäuren, d.h. Isocyanate oder Isothiocyanate. Weitere reaktionsfähige, als Acylierungsmittel verwendbare Derivate von organischen Carbonsäuren sind aktivierte Ester, wie geeignet substituierte Niederalkylester, z.B. Cyanmethylester, oder geeignet substituierte Phenylester, z.B. Pentachlorphenyl- oder 4-Nitrophenylester. Die Veresterung kann, wenn notwendig, in Gegenwart von geeigneten Kondensationsmitteln, bei Verwendung von freien Carbonsäuren, z.B. in Gegenwart von Carbodiimidverbindungen, wie Dicyclohexylcarbodiimid, oder Carbonylverbindungen, wie Diimidazolylcarbonyl, und bei Verwendung von reaktionsfähigen Säurederivaten z.B. in Gegenwart von basischen Mitteln, wie Triniederalkylaminen, z.B. Triäthylamin, oder heterocyclischen Basen, z.B. Pyridin oder 4-Dimethylaminopyridin, durchgeführt werden. Die Acylierungsreaktion kann in Abwesenheit oder in Gegenwart eines Lösungsmittels oder Lösungsmittelgemisches, unter Kühlen, bei Raumtemperatur oder unter Erwärmen, und, wenn notwendig, in einem geschlossenen Gefäss und/oder in einer Inertgas-, z.B. Stickstoffatmosphäre, durchgeführt werden. Geeignete Lösungsmittel sind z.B. gegebenenfalls substituierte, insbesondere gegebenenfalls chlorierte, aliphatische, cycloaliphatische oder aromatische Kohlenwasserstoffe, wie Benzol oder Toluol, wobei man geeignete Veresterungsreagentien, wie Essigsäureanhydrid, auch als Verdünnungsmittel verwenden kann.

Schutzgruppen sind z.B. die bei Verfahren a) genannten.

Verfahren c):

Ein Bruchstück einer Verbindung der Formel I mit einer freien Carboxylgruppe ist z.B. eine Carbonsäure der Formeln VII, VIII, IX, X oder XI,

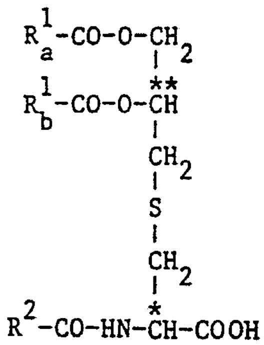

(VII)

* = R

** = R oder S

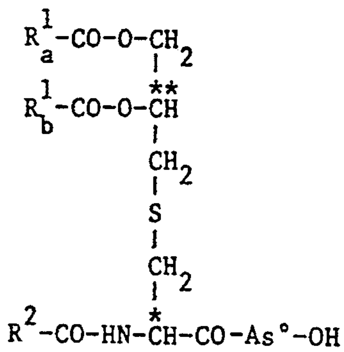

(VIII)

* = R

** = R oder S

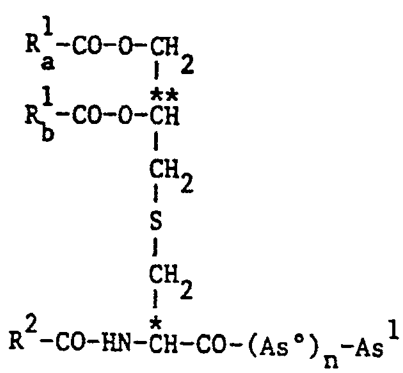

(IX)

* = R

** = R oder S

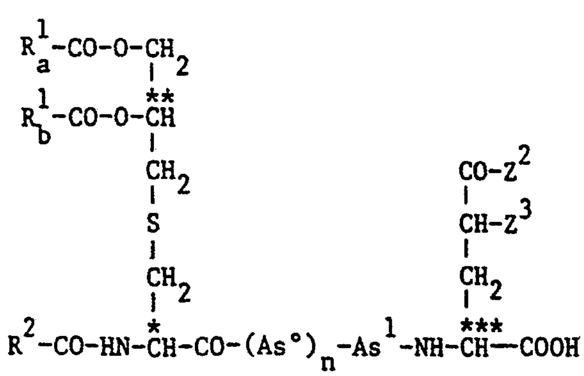

(X)

* = R

** = R oder S

*** = R

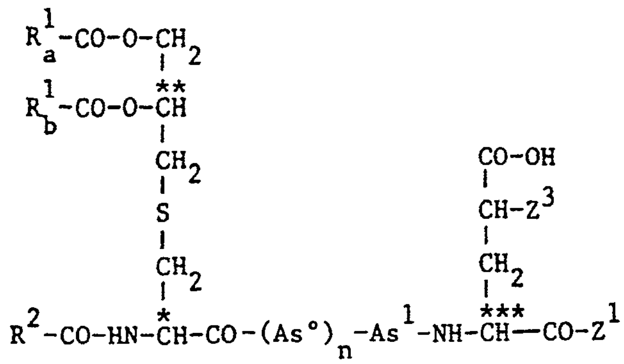

* = R

** = R oder S

*** = R

(XI)

worin die Substituenten die obengenannten Bedeutungen haben, wobei freie funktionelle Gruppen mit Ausnahme der an der Reaktion teilnehmenden Carboxylgruppe, wenn nötig, in geschützter Form vorliegen. Funktionelle Gruppen in einem solchen Bruchstück, die vorzugsweise in geschützter Form vorliegen, sind vor allem Amino- oder weitere Carboxygruppen, daneben z.B. auch Hydroxy- oder Mercaptogruppen. Ein komplementierendes Bruchstück mit einer freien Aminogruppe ist z.B. im Falle einer Carbonsäure der Formel VII eine Aminoverbindung der Formel XII, im Falle einer Carbonsäure der Formel VIII eine Aminoverbindung der Formel XIII, im Falle einer Carbonsäure der Formel IX eine Aminoverbindung der Formel XIV, im Falle einer Carbonsäure der Formel X eine Aminoverbindung der Formel XV und im Falle einer Carbonsäure der Formel XI eine Aminoverbindung der Formel XVI,

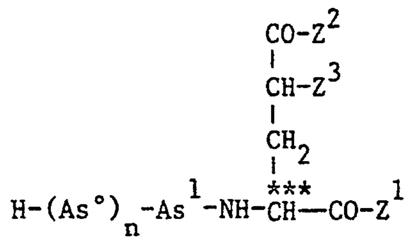

*** = R

(XII)

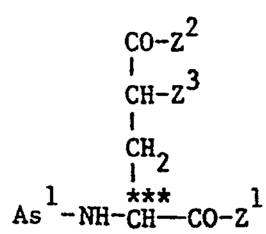

*** = R

(XIII)

$$\begin{array}{l} \qquad\qquad CO-Z^{2} \\ \qquad\qquad | \\ \qquad\qquad CH-Z^{3} \\ \qquad\qquad | \\ \qquad\qquad CH_{2} \\ \qquad\qquad | \\ \qquad\qquad *** \\ H-NH-CH—CO-Z^{1} \end{array}$$

*** = R

(XIV)

$$H-Z^{1}$$

(XV)

$$H-Z^{2}$$

(XVI)

wobei die Substituenten in den Formeln XII, XIII, XIV, XV und XVI die obengenannten Bedeutungen haben mit der Massgabe, dass in den Verbindungen der Formeln XII bis XVI vorhandene freie funktionelle Gruppen mit Ausnahme der an der Reaktion teilnehmenden Aminogruppe, wenn nötig, in geschützter Form vorliegen.

Funktionelle Gruppen in einem solchen komplementierenden Bruchstück, die vorzugsweise in geschützter Form vorliegen, sind vor allem Carboxyl- oder weitere Aminogruppen, daneben z.B. auch Hydroxy- oder Mercaptogruppen.

Eine bevorzugte Ausführungsform der Verfahrensvariante c) ist die Umsetzung eines reaktionsfähigen Säurederivats mit dem komplementierenden Bruchstück mit freier Aminogruppe, wobei die Aktivierung der Säure auch in situ erfolgen kann. Daneben kann man auch die Säure mit dem komplementierenden Bruchstück, dessen Aminogruppe in aktivierter Form vorliegt, umsetzen.

Ein reaktionsfähiges Säurederivat ist z.B. ein Säureazid, -anhydrid, -imidazolid, -isoxazolid oder ein aktivierter Ester, wie Cyanmethylester, Carboxymethylester, Thiophenylester, p-Nitrothiophenylester, Thiokresylester, p-Methansulfonylphenylester, p-Nitrophenylester, 2,4-Dinitrophenylester, 2,4,5- oder 2,3,6-Trichlorphenylester, Pentachlorphenylester, ein Ester mit N-Hydroxy-succinimid, N-Hydroxy-phthalimid, 8-Hydroxy-chinolin, 2-Hydroxy-1,2-dihydro-1-äthoxycarbonyl-chinolin oder N-Hydroxypiperidin oder ist ein Enolester, der mit N-Aethyl-5-phenyl-isoxazolium-3-sulfonat gewonnen wird [Woodward Reagens], oder kann durch Reaktion der Säure mit einem Carbodiimid (gegebenenfalls unter Zusatz von N-Hydroxysuccinimid) oder einem unsubstituierten oder z.B. durch Halogen, Methyl oder Methoxy substituierten 1-Hydroxybenzotriazol, 3-Hydroxy-4-oxo-3,4-dihydrobenzo[d]-1,2,3-triazin oder N,N'-Carbonyldiimidazol, gegebenenfalls in situ, gebildet werden.

Ein reaktionsfähiges Derivat mit aktivierter Aminogruppe kann beispielsweise durch Reaktion der Aminoverbindung mit einem Phosphit gebildet werden.

Als gebräuchlichste Methoden der Kondensation sind zu nennen: die Methode nach Weygand-Wünsch (Carbodiimid in Gegenwart von N-[Hydroxysuccinimid), die Azidmethode, die Methode der N-Carboxyanhydride oder N-Thiocarboxyanhydride, die Methode der aktivierten Ester und die Anhydridmethode. Insbesondere können diese Kondensationen auch nach der Merrifield-Methode vollzogen werden.

Verfahren d):

Nach Verfahrensvariante d) können die terminalen Carboxylgruppen der Aminosäurensequenz der Verbindungen der Formel (I) amidiert oder verestert werden. Diese Umetzungen werden in konventioneller,

an sich bekannter Weise durchgeführt. So kann man Amide z.B. durch Umsetzen der Carbonsäuren mit Ammoniak oder einem Amin, oder eines reaktiven Derivates der Carbonsäure, wie eines Säurehalogenids oder Säureesters, mit den genannten Reagenzien herstellen. Insbesondere werden die in der Peptidchemie üblichen Amidierungsmethoden angewendet, z.B. die Umsetzung eines aktivierten Carbonsäurederivats mit dem gewünschten Amin oder mit Ammoniak nach den oben für Verfahrensvariante c) erörterten Methoden. Die Veresterung erfolgt z.B. nach an sich bekannten Methoden. Man setzt z.B. die freie Säure mit einem reaktiven funktionellen Derivat des betreffenden Alkohols, wie einem Alkylhalogenid, z.B. einem Alkylbromid oder -chlorid, oder einem Dialkylsulfat, wie Dimethylsulfat, in Gegenwart einer Base, wie Pyridin oder Natriumbicarbonat um, oder man setzt direkt mit dem Alkohol unter Zusatz eines geeigneten dehydratisierenden Mittels um. Man kann die Säuren auch mit einem Diazoalkan, z.B. Diazomethan, vorzugsweise in einem Aether und bei Temperaturen zwischen -5° und +30°, oder mit dem betreffenden O-Alkyl-N,N'-dicyclohexyl-isothioharnstoff, vorzugsweise in einem aprotischen Mittel und bei Temperaturen zwischen 25 und 100°, in an sich bekannter Weise umsetzen. Man kann aber sehr vorteilhaft auch von den Metallsalzen der Carbonsäuren, insbesondere den Alkalisalzen ausgehen, und diese mit dem dem herzustellenden Ester entsprechenden Halogenkohlenwasserstoff umsetzen: man verwendet z.B. ein Halogenalkyl, wie Methylbromid, Aethylchlorid oder Benzylchlorid oder ein Dialkylsulfat, wie Dimethylsulfat, und arbeitet vorzugsweise in einem polaren Lösungsmittel, wie z.B. Aceton, Methyläthylketon oder Dimethylformamid, vorzugsweise bei Temperaturen zwischen 25° und 100°. Als Metallsalze verwendet man vorzugsweise diejenigen des Natriums oder Kaliums oder insbesondere auch die des Caesiums. Anstelle der genannten Halogenalkyle kann man vorteilhaft auch ihre Anlagerungsprodukte an tertiäre Amine, d.h. quaternäre Tetraalkylammoniumsalze verwenden.

Schliesslich kann man die Ester auch aus funktionellen Derivaten der Carbonsäuren, z.B. gegebenenfalls aus ihren Halogeniden, durch Umsetzung mit dem gewünschten Alkohol, oder aus anderen Estern durch Umesterung herstellen.

Die nach Verfahrensvariante d) vorzunehmende Umwandlung von Estergruppen in die Carbonsäure-Gruppen geschieht ebenfalls nach an sich bekannten Methoden.

Zur Verseifung können hydrolytische Verfahren mit sauren oder basischen Mitteln oder gegebenenfalls reduktive Methoden verwendet werden: so können z.B. Benzylester durch katalytische Reduktion in an sich bekannter Weise, z.B. mit Palladium-Katalysatoren zur Carbonsäure gespalten werden.

Verfahren e):

Eine nucleofuge Gruppe Y ist eine Abgangsgruppe bei einer nucleophilen Substitution, z.B. eine vorzugsweise veresterte Hydroxygruppe, wobei die Hydroxygruppe insbesondere mit starken anorganischen oder organischen Säuren, z.B. Mineral- oder Sulfonsäuren, verestert ist. Eine nucleofuge Gruppe Y ist somit z.B. Chlorid, Bromid, Jodid, Mono- oder Dialkylsulfat oder Toluolsulfonat.

Fuktionelle Gruppen in einer Verbindung der Formel IV, die vorzugsweise durch leicht abspaltbare Schutzgruppen geschützt werden, sind vor allem andere Mercaptogruppen, aber auch Hydroxy, Amino oder Carboxylgruppen. Ein reaktionsfähiges Derivat einer Verbindung der Formel IV ist eine Verbindung, in der die Nucleophilie des an der Reaktion teilnehmenden Schwefelatoms erhöht ist, z.B. durch Abspaltung des Protons der Mercaptogruppe. Ein solches reaktionsfähiges Derivat kann gegebenenfalls auch in situ gebildet werden.

Die Reaktion kann z.B. analog durchgeführt werden wie beschrieben in der Europäischen Patentschrift 0 000 330 oder in K.H. Wiesmüller, W. Bessler und G. Jung, Hoppe-Seyler's Z. Physiol.Chem. 364, 593-606 (1983).

Verfahren f):

Ein reaktionsfähiges Derivat einer Verbindung der Formel V ist eine Verbindung, in der die Nucleophilie des an der Reaktion teilnehmenden Schwefelatoms erhöht ist, z.B. durch Abspaltung des Protons der Mercaptogruppe. Ein solches reaktionsfähiges Derivat kann gegebenenfalls auch in situ gebildet werden.

Eine nucleofuge Gruppe Y ist z.B. eine der bei Verfahren e) genannten Gruppen.

Funktionelle Gruppen in einer Verbindung der Formel VI, die vorzugsweise in geschützter Form vorliegen, sind vor allem Mercapto-, daneben auch Hydroxy-, Amino- und Carboxygruppen.

Wenn vorstehend nichts anders angegeben, werden die Verfahren a) bis f) in einem inerten Lösungsmittel oder Lösungsmittelgemisch bei einer Temperatur zwischen etwa -20°C und etwa +120°C, und, wenn nötig, unter Schutzgas durchgeführt.

Die Ausgangsstoffe für die oben beschriebenen Verfahren gemäss der vorliegenden Erfindung sind

bekannt, z.B. aus der Europäischen Patentschrift 0 000 330, oder können in an sich bekannter Weise, z.B. analog den obengenannten Verfahren, hergestellt werden.

Ausgangsstoffe, worin in Formel I der Rest $R^2$-CO für Wasserstoff steht, können z.B. durch Umsetzen des an der Aminogruppe mit einer leicht abspaltbaren Schutzgruppe geschützten Glycerylcysteins, dessen Carboxylgruppe in aktivierter Form vorliegt, mit dem gewünschten Peptid gemäss dem gleichen Verfahren wie für Verfahrensvariante c), und nachträgliche Entfernung der Aminoschutzgruppe erhalten werden. Alternativ kann man auch so vorgehen, dass man zunächst an der Aminogruppe mit einer leicht abspaltbaren Schutzgruppe versehenes S-(2,3-Dihydroxypropyl)-cystein acyliert, dann mit dem Peptid kondensiert und hernach die Aminoschutzgruppe abspaltet. Diese Abspaltung kann vorteilhaft in leicht saurem oder neutralem Medium geschehen.

Die Ausgangsstoffe für das Verfahren a) können nach denselben Methoden wie für das Verfahren c) erhalten werden, mit dem Unterschied, dass zumindest eine der funktionellen Gruppen in den Aminosäuren, d.h. die Amino-, Carboxyl- oder Hydroxygruppe in geschützter Form vorliegt.

Die Peptide gemäss Formel (XII) oder ihre Fragmente können nach bekannten Methoden der Peptidchemie, insbesondere nach den oben für Verfahren c) angegebenen, hergestellt werden. Deren Bausteine, insbesondere auch die D-Glutaminsäure oder D-$\gamma$-Carboxy-Glutaminsäure und ihre Amide sind bekannte Verbindungen. Bei den als Ausgangsstoffe zu verwendenden N-Acyl-Glyceryl-cysteinen oder deren auch im Glycerinrest acylierten Derivaten handelt es sich um Abkömmlinge des natürlichen L-Cysteins (Konfiguration R). Gemische von Diastereomeren können, wenn erwünscht, in an sich bekannter Weise in die einzelnen Diastereomere aufgetrennt werden.

Terminale- oder Seitenketten-Carboxylgruppen können auf beliebiger Stufe bei diesen Herstellungsverfahren für Ausgangsstoffe in der gewünschten Weise nach an sich bekannten Methoden verändert, z.B. verestert oder amidiert werden.

Die erhaltenen Lipopeptide können in an sich bekannter Weise in ihre Salze übergeführt werden, z.B. durch Umsetzen erhaltener saurer Verbindungen mit Alkali- oder Erdalkalihydroxyden oder erhaltener basischer Verbindungen mit Säuren. Die Salze können aus ihren Lösungen z.B. durch Lyophilisation in eine für die pharmazeutische Verwendung geeignete Form gebracht werden.

Infolge der engen Beziehung zwischen den neuen Verbindungen in freier Form und in Form ihrer Salze und Komplexsalze kann man im Vorausgegangenen und nachfolgend statt der freien Verbindungen sinn- und zweckgemäss gegebenenfalls auch die entsprechenden Salze einsetzen oder verwenden.

Erhaltene Isomerengemische können auf Grund der physikalisch-chemischen Unterschiede der Bestandteile in bekannter Weise aufgetrennt werden, beispielsweise durch Chromatographie und/oder fraktionierte Kristallisation. Vorteilhafterweise isoliert man das wirksamere der Isomeren.

Die beschriebenen Verfahren werden z.B. nach an sich bekannten Methoden durchgeführt, in Abwesenheit oder vorzugsweise in Anwesenheit von Verdünnungs- oder Lösungsmitteln, wenn notwendig, unter Kühlen oder Erwärmen, unter erhöhtem Druck und/oder in einer Inertgas-, wie Stickstoffatmosphäre. Dabei sind unter Berücksichtigung aller im Molekül befindlichen Substituenten, wenn erforderlich, insbesondere bei Anwesenheit leicht hydrolysierbarer O-Acylreste, besonders schonende Reaktionsbedingungen, wie kurze Reaktionszeiten, Verwendung von milden sauren Mitteln in niedriger Konzentration stöchiometrische Mengenverhältnisse, Wahl geeigneter Katalysatoren, Lösungsmittel, Temperatur und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Die vorliegende Erfindung betrifft ebenfalls pharmazeutische Präparate, welche die beschriebenen neuen Lipopeptide gemäss der Erfindung, ihre Gemische, Salze oder Komplexsalze enthalten. Bei den erfindungsgemässen pharmazeutischen Präparaten handelt es sich um solche zur enteralen, wie oralen oder rektalen, und insbesondere parenteralen oder topischen, z.B. nasalen oder vaginalen Verabreichung an Warmblüter, welche den pharmakologischen Wirkstoff allein oder zusammen mit einem pharmazeutisch anwendbaren Trägermaterial enthalten. Die Dosierung des Wirkstoffes hängt von der Warmblüter-Spezies, dem Alter und dem individuellen Zustand, sowie von der Applikationweise ab. So werden z.B. zur Erzielung eines Immunopotenzierungs-Effekts an Warmblüter mit geringem Körpergewicht, z.B. Mäuse, bei subcutaner Applikation Dosen im Bereiche von ca. 1-30 mg/kg Körpergewicht und bei intraperitonealer Applikation Dosen im Bereiche von 0,03-3 mg/kg Körpergewicht verabreicht. Wegen der schwach ausgeprägten Dosis-Wirkungsbeziehung beträgt die Dosierung bei Warmblütern mit höherem Körpergewicht, z.B. Menschen mit

einem Körpergewicht von etwa 70 kg, 0,01 bis etwa 5 mg/Mensch. Diese Dosis wird je nach der zu behandelnden Krankheit entweder nur einmal während der Krankheitsdauer oder etwa zweimal pro Woche über einen Zeitraum von etwa 4 Wochen verabreicht, z.B. subcutan.

Die oralen bzw. rektalen Formen der neuen pharmazeutischen Präparate enthalten von etwa 1 % bis etwa 95 %, vorzugsweise von etwa 10 % bis etwa 95 %, insbesondere von etwa 20 % bis etwa 90 % des Wirkstoffs: sie können z.B. in Dosiseinheitsform, wie Dragées, Tabletten, Kapseln, Suppositorien oder Ampullen vorliegen und können in an sich bekannter Weise, z.B. mittels konventioneller Misch-, Granulier-, Dragier-, Lösungs- oder Lyophilisierungsverfahren hergestellt werden.

Geeignete Trägerstoffe für die oralen Formen sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindmittel, wie Stärkekleister unter Verwendung z.B. von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls magensaftresistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzugen, Lösungen von geeigneten Cellulospräparaten, wie mikrokristalline Cellulose (Avicel), Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol, Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viksositätserhöhende Stoffe, z.B. Natrium-Carboxymethylcellulose, Sorbit und/oder Dextran und gegebenenfalls auch Stabilisatoren enthalten.

Die pharmazeutischen Präparate für die parenterale Anwendung enthalten vorzugsweise zwischen 0,1% und 75%, insbesondere zwischen 1% und 50% des aktiven Stoffes.

Als topisch anwendbare Präparate kommen z.B. Crèmen, Salben, Pasten, Schäume, Tinkturen und Lösungen in Betracht, die vorzugsweise von etwa 0,02% bis etwa 2% des Wirktoffs enthalten.

Crèmen sind Oel-in-Wasser-Emulsionen, die mehr als 50% Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechend nichtionische Emulgatoren, z.B. Fettsäureester von Polyalkoholen oder Aethylenoxidaddukte davon, wie Polyglycerinfettsäureester oder Polyoxyäthylensorbitan-fettsäureester (Tweens), ferner Polyoxyäthylen-fettalkoholäther oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Crèmen vermindern, z.B. Polyalkohole, wie Glycerin, Sorbit, Propylenglykol und/oder Polyäthylenglykole, ferner Konservierungsmittel, Riechstoffe etc.

Salben sind Wasser-in-Oel-Emulsionen, die bis zu 70%, vorzugsweise jedoch von etwa 20% bis etwa 50% Wasser oder wässerige Phase enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs, enthalten. Emulgatoren sind entsprechende lipophile Substanzen,

wie Sorbitan-fettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyäthylenglykol, sowie Konservierungsmittel, Riechstoffe, etc.

Fettsalben sind wasserfrei und enthalten als Grundlage insbesondere Kohlenwasserstoffe, z.B. Paraffin, Vaseline und/oder flüssige Paraffine, ferner natürliche oder partialsynthetische Fette, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmeflüssigkeit steigernden Fettalkohole, Emulgatoren und/oder Zusätze.

Pasten sind Crèmen und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluoräthan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyäthylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässerig-äthanolische Grundlage auf, der u.a. Polyalkohole, z.B. Glycerin, Glykole, und/oder Polyäthylenglykol, als Feuchthaltemittel zur Herabsetzung der Verdunstung, und rückfettende Substanzen, wie Fettsäureester mit niedrigen Polyäthylenglykolen, d.h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Besonders vorteilhaft ist die Verwendung von pharmazeutischen Präparaten in Liposomenform. Das Lipopeptid wird bei der Bildung der Liposomen zugegeben. Die Herstellung von Liposomen und der Einschluss des Wirkstoffs kann auf verschiedene Weise erfolgen und ist in dem Uebersichtsartikel von Kaye, St. B., Cancer Treatment Reviews (1981) 8, 27-50, beschrieben. Weitere Verfahren zur Herstellung von Liposomen als Träger von Wirkstoffen sind ebenfalls durch Barenholz et al. in Biochemistry, Vol. 16, No. 12, 2806-2810, sowie in den Deutschen Offenlegungsschriften (DOS) 28 19 655, 29 02 672, 25 32 317 und 28 42 608, in der US-Patentschrift 4,053,585 und in der Europäischen Patentanmeldung 36 676 beschrieben.

Man löst beispielsweise die Lipidkomponenten, z.B. Phospholipide, z.B. Phosphatidsäure, Lecithin oder Kephalin, und gegebenenfalls neutrale Lipide, z.B. Cholesterin, zusammen mit dem Lipopeptid in einem organischen Lösungsmittel, z.B. Chloroform/Methanol, auf. Nach dem Eindampfen bleibt eine homogene Filmschicht zurück. Man dispergiert diese in einer wässrigen Phase, z.B. durch Schütteln. Man erhält so multilamellare Liposomen. Bei der anschliessenden Behandlung mit Ultraschall können sich je nach Beschallungsdauer unilamellare Liposomen bilden, die den Wirkstoff enthalten. Die Liposomen-Suspensionen können insbesondere für die parenterale, z.B. subcutane oder intraperitoneale Applikation oder auch topisch, z.B. intranasal, insbesondere bei der Verwendung der neuen Lipopeptide als antivirale Mittel, verwendet werden.

Zum Gegenstand der vorliegenden Erfindung gehört insbesondere auch die Verwendung der neuen Lipopeptide gemäss Formel (I) und ihrer genannten Derivate in einem Verfahren zur Erzielung eines immunstimulierenden Effektes, oder als Prophylaktika oder Therapeutika gegen Infektionskrankheiten, oder als antivirale Mittel bei Mensch und Tier, wobei die neuen Verbindungen vorzugsweise in Form der oben beschriebenen pharmazeutisch Präparate verabreicht werden.

Die vorliegende Erfindung umfasst sodann auch Kombinationen bestehend aus einer oder mehreren Verbindungen gemäss Formel (I) oder ihrer Derivate, und insbesondere Lipopeptiden der Verbindungsgruppen (IA) oder (IB) und einem oder mehreren Antibiotika. Solche Kombinationen sind z.B., wie schon oben erwähnt, zur Erzielung eines erhöhten antibiotischen Effektes bei verschiedenen infektiösen Zuständen indiziert. Diese Kombinationen können Antibiotika aus der Gruppe der ß-Lactame, Aminoglycoside, Tetracycline, Macrolide, Lincomycine, der Polyen- oder Polypeptidantibiotika, der Anrhracycline, der Chlor- oder Thiamphenicole, der Cycloserine, Rifamycine oder der Fusidinsäure enthalten. Solche Kombinationen

können vorzugsweise in Form von pharmazeutischen Präparaten verwendet werden können, die die beiden Komponenten zusammen mit pharmazeutischen Trägermaterialien, wie die oben besonders hervorgehobenen, enthalten.

Sodann betrifft die Erfindung ein Verfahren zur Steigerung der antibiotischen Wirksamkeit von Antibiotika, bei dem man ein Antibiotikum zusammen mit einem Lipopeptid der Formel (I) oder einem der genannten Derivate verabreicht, wobei die Verabreichung getrennt oder simultan erfolgen kann, z.B. in Form der oben erwähnten Kombinationspräparate. Man verwendet in diesem Verfahren eine wirksame oder subwirksame Dosis des Antibiotikums, je nach Art des letzteren, z.B. von etwa 50 bis etwa 500 mg pro Einzeldosis. Die Lipopeptide gemäss der vorliegenden Erfindung werden bei diesem Verfahren in Einzeldosen von etwa 5 mg bis ungefähr zur halben Antibiotika-Menge verwendet. Dabei kann das Lipopeptid bis zu 24 Stunden vor oder nach, vorzugsweise jedoch etwa gleichzeitig mit dem Antibiotikum verabreicht werden.

Bei diesem Verfahren kann man einzelne Antibiotika wie auch Antibiotikagemische verwenden. Als bevorzugte Antibiotika seien unter den $\beta$-Lactamen die Penicilline, Cephalosporine, Peneme. Nocardicine, Thienamycine und Clavulansäuren genannt. Penicillin-Antibiotika sind in erster Linie Amoxycillin, Ampicillin, Carbenicillin, Cloxacillin, Cyclacillin, Dicloxacillin, Mecillinam, Methicillin, Penicillin G, Penicillin V, Pivampicillin, Sulbenicillin, Azlocillin, Ticarcillin, Mezlocillin, Pivmecillinam oder 6-(4-endo-Azatricyclo-[5.2.2.$0^{2,6}$]-undec-8-enyl)-methylenaminopenicillansäure genannt. Aus der Gruppe der Cephalosphorine können z.B. Cefaclor, Cefazaflur, Cefazolin, Cefadroxil, Cefocitin, Cefuroxim, Cephacetril, Cephalexin, Cephaloglycin, Cephaloridine, Cephalotin, Cefamandol, Cephanon, Cephapirin, Cefatrizin, Cephradin, Cefroxadin (7$\beta$-[D-2-amino-2-(1,4-cyclohexadienyl)-acetamido]-3-methoxy-3-cephem-4-carbonsäure Cefsulodin, Cefotaxim, Cefotiam, Ceftezol oder Cefazedon genannt werden. Unter den Nocardicinen ist z.B. Nocardicin A und unter den Thienamycinen und Clavulansäuren z.B. Thienamycin bzw. Clavulansäure zu erwähnen. Unter den Aminoglycosiden sind insbesondere Streptomycine, z.B. Streptomycin und Streptomycin A, Neomycine, z.B. Neomycin B, Tobramycine, z.B. Tobramycin oder Dibekacin, Kanamycine (z.B. Gemische von Kanamycin A, B und C), sowie Amicacine, Gentamycine (z.B. Gemische von Gentamycin A, $C_1$, $C_2$ oder $C_{1a}$), oder Sisomicine, wie Sisomicin oder Netilmicin, ferner Lividomycin, Ribocamycin und Paromomycin zu erwähnen. Als Tetracycline sind insbesondere Tetracyclin, Doxycyclin, Chlortetracyclin, Oxytetracyclin oder Methacyclin zu nennen. Als Macrolide sind z.B. Maridomycin, Spiramycine, wie Spiramycin I, II und III, Erythromycine, z.B. Erythromycin, Oleandomycine, z.B. Oleandomycin und Tetraacetyloleandomycin, und als Lincomycine z.B. Lincomycin und Clindamycin Zu erwähnen.

Als Polyen-Antibiotika sind besonders Amphotericin B und dessen Methylester oder Nystatin zu nennen. Als Polypeptid-Antibiotika können z.B. Colistrin, Gramicidin S, Polymyxin B, Virginamycin, Tyrothricin, Viomycin oder Vancomycin besonders genannt werden. Als Rifamycine kommen in erster Linie das Rifamycin S, Rifamycin SV oder Rifamycin B oder deren halbsynthetische Derivate, insbesondere das Rifampicin, in Frage.

Die nachfolgenden Beispiele illustrieren die oben beschriebene Erfindung; sie sollen jedoch diese in ihrem Umfang in keiner Weise einschränken. Temperaturen werden in Celsiusgraden angegeben. $R_F$-Werte werden auf Kieselgel-Dünnschichtplatten (Merck, Darmstadt, Deutschland) ermittelt. Die Zusammensetzung von Lösungsmittelgemischen ist, wenn nicht anders vermerkt, in Volumenteilen angegeben. Die Konzentration, c, der Substanz im Lösungsmittel(gemisch) ist im Falle der optischen Drehung in Prozent (Gweicht/Volumen) angegeben.

Abkürzungen:

| | |
|---|---|
| Boc | tert. Butyloxycarbonyl |
| Bz | Benzyl |
| DMA | Dimethylacetamid |
| DMF | Dimethylformamid |
| EEDQ | 2-Aethoxy-N-äthoxycarbonyl-1,2-dihydro-chinolin |
| Et | Ethyl |
| Me | Methyl |
| nBu | n-Butyl |
| Rotavap | Rotationsverdampfer |
| RT | Raumtemperatur |
| Smp. | Schmelzpunkt |
| Su | Succinimidyl |
| tBu | tert.Butyl |
| Z | Benzyloxycarbonyl |

Beispiel 1

a) 4,1 g (3,89mMol) Palmitoyl-Cys(2[R],3-dilauroyloxypropyl)-Ala-D-Glu(OtBu)-$NH_2$ löst man in einer Mischung aus 18 ml Trifluoressigsäure und 42 ml Methylenchlorid. Nach 6 Stunden bei Raumtemperatur dampft man unter Vakuum zum Sirup ein, verreibt mit Aether und erhält einen farblosen, kristallinen Rückstand; dieser wird nochmals mit Aether extrahiert und zweimal aus Aethanol umkristallisiert, worauf man Palmitoyl-Cys(2[R],3-dilauroyloxy-propyl)-Ala-D-Glu-$NH_2$ erhält; $R_F$ = 0,23 ($CHCl_3$ : EtOH = 9:1), Smp. 158-161°, $[\alpha]_D^{20}$ = -12° ($CHCl_3$ : MeOH = 1:1; c = 1,04).

Das Ausgangsmaterial erhält man wie folgt:

b) 5,4 g (6,67 mMol) Palmitoyl-Cys(2[R],3-dilauroyloxy-propyl), 2,2 g (7,1 mMol) Ala-D-Glu(OtBu)-$NH_2$ x HCl und 1,1 g (8,1 mMol) N-Hydroxy-benztriazol löst man in einer Mischung aus 120 ml absolutem Dimethylformamid und 180 ml Methylenchlorid, gibt 1,67 g (8,1 mMol) Dicyclohexylcarbodiimid zu und stellt den pH-Wert der Lösung mit 1 ml Triäthylamin auf etwa 7 ein. Nach 16 Stunden bei Raumtemperatur wird im Vakuum zur Trockne eingedampft. Der Rückstand wird mit Wasser extrahiert, die Extrakte verworfen, und der Rückstand zweimal aus Methanol umkristallisiert. Man erhält farblose Kristalle von Palmitoyl-Cys(2[R],3-dilauroyloxy-propyl)-Ala-D-Glu(OtBu)-$NH_2$; Smp. 196-198°, $[\alpha]_D^{20}$ = -11° ($CHCl_3$ : MeOH = 1:1; c = 0,98), $R_F$ = 0,44 ($CHCl_3$ : EtOH = 9:1).

c) Das als Ausgangsmaterial verwendete Palmitoyl-Cys(2[R],3-dilauroyloxy-propyl) erhält man aus dem entsprechenden Benzhydrylester mit Trifluoressigsäure:

8 g Benzhydrylester lässt man in einer Mischung aus 20 ml Trifluoressigsäure und 80 ml Methylenchlorid 3 Stunden bei Raumtemperatur stehen, dampft im Vakuum zum Sirup ein und extrahiert diesen mit Eiswasser. Man reinigt die Substanz durch Chromatographie an 300 g Kieselgel, Merck, mit den Elutionsmitteln

Methylenchlorid/Aethanol = 95:5

Methylenchlorid/Aethanol = 9:1

Chloroform/Methanol = 7:3

Vor Aufgabe der Substanz auf die Säule wird der pH-Wert mit Triäthylamin auf etwa 5 gebracht. Die nach dem Eindampfen zunächst sirupösen Fraktionen der Reinsubstanz mit $R_F$ = 0,22 ($CHCl_3$:MeOH = 9:1) kristallisieren bei Zugabe von Methanol, worauf man Palmitoyl-Cys(2[R],3-dilauroyloxy-propyl) erhält; Smp. 52-55°, $[\alpha]_D^{20}$ = -7° (Dioxan; c = 0,65).

Beispiel 2

a) 300 mg Palmitoyl-Cys(2[R],3-dipalmitoyloxy-propyl)-Ala-D-Glu(Ala-OBz)-$NH_2$ hydriert man in 50 ml Dimethylformamid-Tetrahydrofuran(7:3) mit 200 mg Pd-Mohr 50 Stunden bei 45°. Man filtriert vom Katalysator ab und extrahiert mit 30 ml warmem Lösungsmittelgemisch. Die Lösungen werden im Vakuum eingedampft und der Rückstand an 10 g Kieselgel, Merck, mit Chloroform-Methanol (9:1) als Elutionsmittel gereinigt. Man erhält nach Eindampfen der reinen Fraktionen farblose Kristalle von Palmitoyl- Cys(2[R],3-dipalmitoyloxy-propyl)-Ala-D-Glu(Ala)-$NH_2$ mit dem Zersetzungsbereich 224-229°, $[\alpha]_D^{20}$ = -12° ($CHCl_3$: MeOH = 1:1; c = 0,91), $R_F$ = 0,31 ($CHCl_3$ :MeOH = 8:2).

Den als Ausgangsmaterial verwendeten Benzylester erhält man wie folgt:

b) 0,6 g (0,659 mMol) Palmitoyl-Cys(2[R],3-dipalmitoyloxy-propyl), 257,6 mg (0,659 mMol) Ala-D-Glu(Ala-OBz)-$NH_2$ x HCl, 106,9 mg (0,79 mM ol) N-Hydroxy-benztriazol und 92 µl Triäthylamin löst man in einer Mischung aus 20 ml absolutem Dimethylformamid und 50 ml Methylenchlorid und gibt dazu 164 mg (0,79 mMol) Dicyclohexylcarbodiimid. Nach 16 Stunden bei Raumtemperatur dampft man die Reaktionslösung im Vakuum zur Trockne ein und verrührt den Rückstand mit 50 ml Methanol bei 50°. Man erhält eine erste Kristallfraktion, aus der Mutterlauge kristallisiert eine zweite Fraktion. Beide Fraktionen werden nochmals aus Essigester umkristallisiert. Man erhält so farblose Kristalle von Palmitoyl-Cys(2[R],3-dipalmitoyloxy-propyl)-Ala-D-Glu(Ala-OBz-$NH_2$; Smp. 180-184°, $R_F$ = 0,61 ($CHCl_3$:EtOH = 9:1).

c) Das zur Kupplung mit dem Tripeptid verwendete Palmitoyl-Cys(2[R],3-dipalmitoyloxy-propyl) erhält man analog Beispiel 1c aus dem entsprechenden Benzhydrylester als farblose, kristalline Substanz mit dem Smp. 71-75°, $R_F$ = 0,45 ($CHCl_3$:MeOH = 9:1), $[\alpha]_D^{20}$ = -4° (Dioxan; c = 0,89).

Beispiel 3

3 mg des Lipopeptids Palmitoyl-Cys(2[R],3-dilauroyloxy-propyl)-Ala-D-Glu-$NH_2$ (vgl. Beispiel 1) werden zusammen mit 27 mg Lecithin in einem Gemisch von Chloroform und Methanol 2:1 gelöst. Die Mischung wird sodann in einem Rotationsverdampfer im Vakuum eingedampft, wobei man einen Lipid-Film erhält. Zu

diesem gibt man 0,2 ml einer sterilen pyrogenfreien 0,9 %igen NaCl-Lösung "flec-Flac" der Firma VIFOR S.A. in Genf. Man beschallt die Lösung bei Raumtemperatur 2 Minuten lang, wobei eine Suspension von Lipidvesikeln (Liposomen) mit einem Durchmesser von ca. 1 bis 5 Mikron, die das Lipopeptid enthalten, entsteht. Diese Suspension kann z.B. subcutan oder intraperitoneal Mäusen in Dosen von 0,1 ml pro 10 g Körpergewicht verabreicht werden.

Aehnliche pharmazeutische Präparate für die Verabreichung am Menschen können in analoger Weise hergestellt werden.

Beispiel 4: 500 mg (0,34 mMol) Palmitoyl-Cys(2[R],3-dilauroyloxypropyl)-Ala-D-Glu{Lys(Boc)-Lys(Boc)-OMe}-$NH_2$ löst man in einer Mischung aus 5,3 ml 1,7 N Salzsäure in absolutem Essigsaureathylester und 12 ml absol. Methylenchlorid. Nach 1 Stunde bei Raumtemperatur dampft man zur Trockene und wiederholt diesen Vorgang dreimal mit je 10 ml Methyl-tert.-butyläther. Den Rückstand verreibt man mit Aceton, saugt den Niederschlag ab und wiederholt diesen Vorgang mit warmem Essigsäureäthylester, dem man vor dem Absaugen noch den gleichen Volumteil Aceton zusetzt, worauf man Palmitoyl-Cys(2[R],3-dilauroyloxy-propyl)-Ala-D-Glu{Lys-Lys-OMe}-$NH_2$ x 2 HCl in Form farbloser Kristalle mit 0,5 Mol Kristallwasser erhält; Smp. 250-260° (Zers.), $[\alpha]_D^{20}$ = -18° ± 1° (c = 0,849; $CHCl_3$: MeOH = 1:1), $R_F$ = 0,22 ($CHCl_3$:MeOH:$H_2O$:Essigsäure = 75:25:2:2).

Das Ausgangsmaterial gewinnt man wie folgt:

Stufe 4.1: 1 g (1,002 mMol) Palmitoyl-Cys(2[R],3-dilauroyloxy-propyl)-Ala-D-Glu-$NH_2$, 0,15 g N-Hydroxy-benztriazol, 0,15 g N-Hydroxy-succinimid und 0,5 g Dicyclohexylcarbodiimid rührt man 3 Stunden bei Raumtemperatur in einer Mischung aus 5 ml Dimethylformamid, 20 ml Chloroform und 5 ml Acetonitril. Danach gibt man 0,55 g (1,05 mMol) Lys(Boc)-Lys(Boc)-OMe x HCl und 0,3 ml Triäthylamin zu. Nach 40 Stunden bei Raumtemperatur dampft man im Vakuum ein, verrührt den Rückstand mit 20 ml Wasser und saugt ab. Dies wiederholt man mit Methanol und kristallisiert den verbleibenden Niederschlag aus MeOH um. Man erhält so farblose Kristalle von Palmitoyl-Cys(2[R],3-dilauroyloxy-propyl)-Ala-D-Glu{Lys(Boc)-Lys-(Boc)-OMe}-$NH_2$; Smp. 180-182°, $[\alpha]_D^{20}$ = -25° ± 1° (c = 1,395; $CHCl_3$), $R_F$ = 0,69 ($CHCl_3$:MeOH = 9:1).

Beispiel 5: 1 g (1,002 mMol) Palmitoyl-Cys(2[R],3-dilauroyloxy-propyl)-Ala-D-Glu-$NH_2$, 0,23 g N-Hydroxy-succinimid, 0,07 g N-Hydroxy-benztriazol und 0,52 g Dicyclohexylcarbodiimid löst man in 15 ml absol. Dimethylacetamid und lässt 5 Stunden bei Raumtemperatur stehen. Danach gibt man eine Lösung von 0,274 g Glycyltaurin und 173 µl Tetramethylguanidin in 10 ml Dimethylacetamid zu. Nach 24 Stunden bei Raumtemperatur gibt man weitere 100 mg Dicyclohexylcarbodiimid, 100 mg N-Hydroxy-succinimid, 50 mg Glycyltaurin und 90 µl Tetramethylguanidin zu. Nach weiteren 24 Stunden bei Raumtemperatur wird aufgearbeitet. Eindampfen im Vakuum, Waschen des Rückstandes mit 50 ml Hexan, dann mit 30 ml Acetonitril, danach 3maliges Digerieren mit je 20 ml gesättigter NaCl-Lösung bei 40° ergibt einen Rückstand, der in $CHCl_3$-MeOH-Wasser (85:15:1) bei 40° gelöst und über Kieselgel chromatographiert wird. Nach Elution mit $CHCl_3$-MeOH-$H_2O$ (70:30:3) und Eindampfen im Vakuum erhält man Palmitoyl-Cys(2-[R],3-dilauroyloxy-propyl)-Ala-D-Glu(Gly-taurinnatriumsalz)-$NH_2$ x 0,82 $H_2O$; Zersetzungspunkt 250°, $[\alpha]_D^{20}$ = -24° (c = 1,052; $CHCl_3$:MeOH = 1:1), $R_F$ = 0,256 ($CHCl_3$:MeOH = 8:2).

Das verwendete Glycyltaurin erhält man in bekannter Weise durch Hydrolyse von N-(Boc-Gly)-taurin-natriumsalz mit Trifluoressigsäure. N-(Boc-Gly)-taurin-Na-salz seinerseits wird erhalten aus Boc-Gly-O-Su und Taurin-natriumsalz in 90proz.-wässrigem Methanol.

Beispiel 6: 521 mg (0,588 mMol) Octanoyl-Cys(2[R,S],3-didecanoyloxy-propyl)-Ala-D-Glu(OtBu)-$NH_2$ werden bei Raumtemperatur in 5 ml Methylenchlorid, enthaltend 20 Vol.-% Trifluoressigsäure, 6 Stunden gerührt. Anschliessend wird das Lösungsmittel am Rotationsverdampfer bei 40° Badtemperatur abdestilliert, der Rückstand mit Wasser angerieben, abgenutscht, auf der Nutsche neutral gewaschen und bei 40°/0,1 Torr 4 Stunden getrocknet. Das so erhaltene Rohprodukt wird aus Aethylmethylketon umkristallisiert, worauf man Octanoyl-Cys(2[R,S],3-didecanoyloxy-propyl)-Ala-D-Glu-$NH_2$ erhält; Smp. 170-171°, $[[\alpha]_D^{20}$ = -13° (c = 1,723; $CHCl_3$:Methanol = 1:1), $R_F$ = 0,5 ($CHCl_3$:Methanol = 9:1).

Das Ausgangsprodukt erhält man folgendermassen:

Stufe 6.1: Zu 20 g (0,16 Mol) L-Cystein in 150 ml Pyridin und 120 ml Methylenchlorid werden bei 20-25° unter Stickstoffatmosphäre 26,8 g (0,16 Mol) Caprylsäurechlorid in 30 ml Methylenchlorid zugetropft, anschliessend wird 17 Stunden bei Raumtemperatur gerührt. Aus dem Reaktionsgemisch destilliert man bei ca. 40° im Vakuum das Methylenchlorid ab und versetzt mit 150 ml Pyridin, 150 ml DMA und 150 ml Wasser, wobei eine homogene Lösung entsteht. Das Reaktionsgemisch wird nun mit 30proz. NaOH auf pH = 9 gestellt, 2 Stunden bei Raumtemperatur gerührt, mit 2N Salzsäure auf pH = 5 gestellt, mit Essigsäureäthylester extrahiert, die organische Phase über Natriumsulfat getrocknet und am Rotationsverdampfer bei 40-45° eingedampft, wobei Octanoyl-Cys als gelbes, viskoses Oel zurückbleibt; $R_F$ = 0,31 (Chloroform:Methanol = 7:3), $[\alpha]_D^{20}$ = -16° (c = 1,06; $CHCl_3$:MeOH = 1:1).

Stufe 6.2: 40,4 g (0,163 Mol) Octanoyl-Cys und 52,8 g (0,383 Mol) Kaliumcarbonat in 375 ml Aethanol

versetzt man unter Stickstoff mit 13,9 g (0,189 Mol) Glyceringlycid. Unter schwachem Stickstoffstrom wird 4 Stunden bei 75-80° gerührt, auf Raumtemperatur gekühlt, die Suspension mit 150 ml Wasser versetzt, mit 2N HCl auf etwa pH = 3 gestellt und mit Essigsäureäthylester extrahiert. Die organische Phase trocknet man über Natriumsulfat, filtriert und dampft das Filtrat ein, wobei Octanoyl-Cys(2[R,S],3-dihydroxy-propyl) als gelbes Oel zurückbleibt. $R_F$ = 0,23 ($CHCl_3$:MeOH:$H_2O$ = 65:35:2), $[\alpha]_D^{20}$ = -6° (c = 0,995; $CHCl_3$:MeOH = 1:1).

Stufe 6.3: 2,01 g (6,32 mMol) Octanoyl-Cys(2[R,S],3-dihydroxy-propyl) in 40 ml Dimethylacetamid versetzt man mit 2,28 g (9,22 mMol) EEDQ, 1,95 g (6,32 mMol) Ala-D-Glu(OtBu)-$NH_2$ x HCl und 0,77 ml (6,32 mMol) Triäthylamin, worauf das Reaktionsgemisch pH = 7 aufweist. Nach 16stündigem Rühren bei 40-45° wird das Lösungsmittel bei 45-50° im Hochvakuum abdestilliert. Der Rückstand wird 3mal mit Diäthyläther und 3mal mit Essigsäureäthylester verrieben und jeweils die flüssige Phase abdekantiert. Der unlösliche Rückstand wird 1 Stunde bei 40° und 1 Torr getrocknet, in 30 ml THF-$H_2O$ (9:1) bei Raumtemperatur gelöst und mit 100 ml $H_2O$ unter Rühren wieder gefällt. Nach Absaugen und Trocknen dieses Niederschlags bei 40° und 1 Torr erhält man Octanoyl-Cys(2[R,S],3-dihydroxy-propyl)-Ala-D-Glu-(OtBu)-$NH_2$; Smp. 184-186°, $R_F$ = 0,61 (Methylenchlorid:Methanol = 8:2), $[\alpha]_D^{20}$ = -4° (c = 0,479; $CHCl_3$:MeOH = 1:1).

Stufe 6.4: 2,5 g (3,6 mMol) Octanoyl-Cys(2[R,S],3-dihydroxy-propyl)-Ala-D-Glu(OtBu)-NH in einer Mischung aus 18,9 ml (0,23 Mol) Pyridin und 18,9 ml Tetrachlorkohlenstoff werden auf 55-60° erwärmt und bei dieser Temperatur 37,8 mg (0,31 mMol) 4-Dimethylaminopyridin und 1,7 ml (8,1 mMol) Caprinsäurechlorid zugegeben. Das Reaktionsgemisch wird 20 Stunden bei 55-60° gerührt, abgekühlt, mit 200 ml Methylenchlorid verdünnt, 3mal mit je 50 ml 2N HCl geschüttelt, dann mit $H_2O$ neutral gewaschen, die org. Phase über $Na_2SO_4$ getrocknet und eingedampft. Der braune, harzige Rückstand wird durch Niederdruckchromatographie über Kieselgel (0,040-0,64 mm) mit dem Laufmittel $CH_2Cl_2$:Methanol = 96:4 gereinigt, worauf man Octanoyl-Cys(2[R,S],3 didecanoyloxy-propyl)-Ala-D-Glu(OtBu)-$NH_2$ erhält; Smp. 144-146°, $[\alpha]_D^{20}$ = -10° (c = 1,017; $CHCl_3$:Methanol = 1:1), $R_F$ = 0,64 ($CHCl_3$:Methanol = 9:1).

Beispiel 7: 0,972 g (1,03 mMol) Octanoyl-Cys(2[R,S],3-didodecanoyloxy-propyl)-Ala-D-Glu(OtBu)-$NH_2$ werden analog Beispiel 1a zu Octanoyl-Cys(2[R,S],3-didodecanoyloxy-propyl)-Ala-D-Glu-$NH_2$ umgesetzt; Smp. 162°, $[\alpha]_D^{20}$ = +12° (c = 0,855; Chloroform:Methanol = 1:1), $R_F$ = 0,2 (Chloroform:Methanol = 92:8).

Das Ausgangsprodukt erhält man folgendermassen:

Stufe 7.1: 1,1 g (1,90 mMol) Octanoyl-Cys(2[R,S],3-dihydroxy-propyl)-Ala-D-Glu(OtBu)-$NH_2$ werden analog Stufe 6.4 mit Laurinsäurechlorid zu Octanoyl-Cys(2[R,S],3-didodecanoyloxy-propyl)-Ala-D-Glu(OtBu)-$NH_2$ umgesetzt; Smp. 140-141°, $[\alpha]_D^{20}$ = -9° (c = 1,352; Chloroform:Methanol = 1:1), $R_F$ = 0,37 (Chloroform:Methanol = 92:8).

Beispiel 8: 900 mg (0,902 mMol) Octanoyl-Cys(2[R,S],3-ditetradecanoyloxy-propyl)-Ala-D-Glu(OtBu)-$NH_2$ werden analog Beispiel 1a zu Octanoyl-Cys(²[R,S],3-ditetradecanoyloxy-propyl)-Ala-D-Glu-$NH_2$ umgesetzt; Smp. 166°, $[\alpha]_D^{20}$ = -11° (c = 1,457; $CHCl_3$:Methanol = 1:1), $R_F$ = 0,11 ($CHCl_3$:Methanol = 92:8).

Das Ausgangsmaterial ist folgendermassen erhältlich:

Stufe 8.1: 1,1 g (1,90 mMol) Octanoyl-Cys(2[R,S],3-dihydroxy-propyl)-Ala-D-Glu(OtBu)-$NH_2$ werden mit 1,03 g (4,18 mMol) Myristoylchlorid analog Stufe 6.4 zu Octanoyl-Cys(2[R,S],3-ditetradecanoyloxy-propyl)-Ala-D-Glu(OtBu)-$NH_2$ umgesetzt; Smp. 140-141°, $[\alpha]_D^{20}$ = -10° (c = 0,648; $CHCl_3$:Methanol = 1:1), $R_F$ = 0,5 ($CHCl_3$:Methanol = 92:8).

Beispiel 9: 995,3 mg (0,971 mMol) Decanoyl-Cys(2[R,S],3-ditetradecanoyloxy-propyl)-Ala-D-Glu(OtBu)-$NH_2$ werden analog Beispiel 1a zu Decanoyl-Cys(2[R,S],3-ditetradecanoyloxy-propyl)-Ala-D-Glu-$NH_2$ umgesetzt; Smp. 154-155°, $[\alpha]_D^{20}$ = -12° (c = 0,922; $CHCl_3$:Methanol = 1:1), $R_F$ = 0,41 ($CHCl_3$:Methanol = 1:1).

Das Ausgangsprodukt ist folgendermassen erhältlich:

Stufe 9.1: 20 g L-Cystein (0,1652 Mol) werden mit 28,2 g (0,1487 Mol) Caprinsäurechlorid analog Stufe 6.1 zu Decanoyl-Cys umgesetzt; Smp. 57-58°, $R_F$ = 0,21 ($CHCl_3$:Methanol = 85:15), $[\alpha]_D^{20}$ = -15° (c = 0,814; $CHCl_3$:MeOH = 1:1).

Stufe 9.2: 6,1 g (22,2 mMol) Decanoyl-Cys werden analog Stufe 6.2 zu Decanoyl-Cys(2[R,S],3-dihydroxy-propyl) umgesetzt. Man erhält ein viskoses, gelbes Oel mit $R_F$ = 0,25 ($CHCl_3$:MeOH = 65:35), $[\alpha]_D^{20}$ = -4° (c = 0,946; $CHCl_3$:MeOH = 1:1).

Stufe 9.3: 2,62 g (7,49 mMol) Decanoyl-Cys(2[R,S],3-dihydroxy-propyl) werden mit 2,31 g (1,49 mMol) Ala-D-Glu(OtBu)-$NH_2$ x HCl analog Beispiel 1b zu Decanoyl-Cys(2[R,S],3-dihydroxy-propyl)-Ala-D-Glu-(OtBu)-$NH_2$ umgesetzt; Smp. 166-167°, $[\alpha]_D^{20}$ = -27° (c = 0,804; $CHCl_3$:Methanol = 1:1), $R_F$ = 0,48 ($CH_2Cl_2$:Methanol = 85:15).

Stufe 9.4: 907 mg (1,5 mMol) Decanoyl-Cys(2[R,S],3-dihydroxy-propyl)-Ala-D-Glu(OtBu)-$NH_2$ werden

mit 814 mg (3,3 mMol) Myristoylchlorid zu Decanoyl-Cys(2[R,S],3-ditetradecanoyloxy-propyl)-Ala-D-Glu-(OtBu)-$NH_2$ umgesetzt; Smp. 138-139°, $[\alpha]_D^{20}$ = -9° (c = 1,234; $CHCl_3$:Methanol 1:1), $R_F$ = 0,51 ($CHCl_3$:Methanol = 9:1).

Beispiel 10: 987 mg Decanoyl-Cys(2[R,S],3-didodecanoyloxy-propyl)-Ala-D-Glu(OtBu)-$NH_2$ werden analog Beispiel 1a zu Decanoyl-Cys(2[R,S],3-didodecanoyloxy-propyl)-Ala-D-Glu-$NH_2$ umgesetzt; Smp. 180°, $[\alpha]_D^{20}$ = -12° (c = 1,066; $CHCl_3$:Methanol = 1:1), $R_F$ = 0,4 ($CHCl_3$:Methanol = 92:8).

Das Ausgangsprodukt ist folgendermassen erhältlich:

Stufe 10.1: 604 mg (1 mMol) Decanoyl-Cys(2[R,S],3-dihydroxy-propyl]-Ala-D-Glu(OtBu)-$NH_2$ werden mit 480 mg (2,2 mMol) Laurinsäurechlord analog Stufe 6.4 zu Decanoyl-Cys(2[R,S],3-didodecanoyloxy-propyl)-Ala-D-Glu(OtBu)-$NH_2$ umgesetzt; Smp. 146-147°, $[\alpha]_D^{20}$ = -8° (c = 1,246; $CHCl_3$:Methanol = 1:1), $R_F$ = 0,62 ($CHCl_3$:Methanol = 9:1).

Beispiel 11: 1,5 g (1,75 mMol) Decanoyl-Cys(2[R,S],3-dioctanoyloxy-propyl)-Ala-D-Glu(OtBu)-$NH_2$ werden analog Beispiel 1a zu Decanoyl-Cys(²[R,S],3-dioctanoyloxy-propyl)-Ala-D-Glu-$NH_2$ umgesetzt; Smp. 154-155°, $[\alpha]_D^{20}$ = -15° (c = 0,958; $CHCl_3$:Methanol = 1:1), $R_F$ = 0,375 ($CHCl_3$:Methanol:$H_2O$ = 80:20:1).

Das Ausgangsmaterial ist folgendermassen erhältlich:

Stufe 11.1: 955,5 mg (1,58 mMol) Decanoyl-Cys(2[R,S],3-dihydroxy-propyl)-Ala-D-Glu(OtBu)-$NH_2$ werden analog Stufe 6.4 mit 565 mg (3,475 mMol) Caprylsäurechlorid zu Decanoyl-Cys(2[R,S],3-dioctanoyloxy-propyl)-Ala-D-Glu(OtBu)-$NH_2$ umgesetzt; Smp. 165-167°, $[\alpha]_D^{20}$ = -12° (c = 0,930; $CHCl_3$:Methanol = 1:1), $R_F$ = 0,454 (Toluol:Aethanol = 85:15).

Beispiel 12: 2,912 g (2,841 mMol) Tetradecanoyl-Cys(2[R,S],3-didodecanoyloxy-propyl)-Ala-D-Glu-(OtBu)-$NH_2$ werden analog Beispiel 1a zu Tetradecanoyl-Cys(2[R,S],3-didodecanoyloxy-propyl)-Ala-D-Glu-$NH_2$ umgesetzt; Smp. 160-162°, $[\alpha]_D^{20}$ = -12° (c = 1,333; $CHCl_3$:Methanol = 1:1), $R_F$ = 0,39 ($CHCl_3$:MeOH:$H_2O$ = 80:20:1).

Das Ausgangsmaterial erhält man folgendermassen:

Stufe 12.1: 20 g (0,1652 Mol) Cys werden mit 36,6 g (0,1487 Mol) Myristoylchlorid analog Stufe 6.1 zu Tetradecanoyl-Cys umgesetzt; Smp. = 63-65°, $R_F$ = 0,13 ($CH_2Cl_2$:Aceton = 8:2).

Stufe 12.2: 31,0 g (93,5 mMol) Tetradecanoyl-Cys werden analog Stufe 6.2 mit 8,3 g (108 mMol) Glyceringlycid zu Tetradecanoyl-Cys(2[R,S],3-dihydroxy-propyl) (Harz) umgesetzt; $R_F$ = 0,19 ($CHCl_3$:Methanol = 65:35), $[\alpha]_D^{20}$ = +3° (c = 1,183; $CHCl_3$:MeOH = 1:1).

Stufe 12.3: 3,0 g (7,39 mMol) Tetradecanoyl-Cys(2[R,S],3-dihydroxy-propyl) werden mit 2,28 g (7,39 mMol) Ala-D-Glu(OtBu)-$NH_2$ x HCl analog Stufe 6.3 zu Tetradecanoyl-Cys(2[R,S],3-dihydroxy-propyl)-Ala-D-Glu(OtBu)-$NH_2$ umgesetzt; Smp. 161-162°, $R_F$ = 0,52 (Toluol:Essigsäureäthylester:Isopropanol:2N Essigsäure = 10:35:35:20), $[\alpha]_D^{20}$ = -19° (c = 1,112; $CHCl_3$:MeOH = 1:1).

Stufe 12.4: 3,29 g (5,21 mMol) Tetradecanoyl-Cys(2[R,S],3-dihydroxy-proypl]-Ala-D-Glu(OtBu)-$NH_2$ werden mit 2,5 g (11,4 mMol) Laurinsäurechlorid analog Stufe 6.4 zu Tetradecanoyl-Cys(2[R,S],3-didodecanoyloxy-propyl)-Ala-D-Glu(OtBu)-$NH_2$ umgesetzt; Smp. 129-131°, $[\alpha]_D^{20}$ = -7° (c = 1,194; $CHCl_3$:Methanol = 1:1), $R_F$= 0,55 ($CHCl_3$:Methanol = 9:1).

Beispiel 13: 1,02 g (1,06 mMol) Tetradecanoyl-Cys(2[R,S],3-didecanoyloxy-propyl)-Ala-D-Glu(OtBu)-$NH_2$ werden analog Beispiel 1a zu Tetradecanoyl-Cys(2[R,S],3-didecanoyloxy-propyl)-Ala-D-Glu-$NH_2$ umgesetzt; Smp. 166-167°, $[\alpha]_D^{20}$ = -13° (c = 1,13; $CHCl_3$:Methanol = 1:1), $R_F$ = 0,3 ($CHCl_3$:Methanol:$H_2O$ = 75:25:1).

Der Ausgangsstoff wird folgendermassen hergestellt:

Stufe 13.1: 997,7 mg (1,5 mMol) Tetradecanoyl-Cys(2[R,S],3-dihydroxy-propyl)-Ala-D-Glu(OtBu)-$NH_2$ werden analog Stufe 6.4 mit 629 mg (3,3 mMol Caprinsäurechlorid zu Tetradecanoyl-Cys(2[R,S],3-di decanoyloxy-propyl)-Ala-D-Glu(OtBu)-$NH_2$ umgesetzt, Smp. 132-133°, $[\alpha]_D^{20}$ = -10° (c = 0,943; $CHCl_3$:Methanol = 1:1), $R_F$ = 0,64 ($CHCl_3$:Methanol = 9:1).

Beispiel 14: 1,311 g (1,41 mMol) Tetradecanoyl-Cys(2[R,S],3-dioctanoyloxy-propyl)-Ala-D-Glu(OtBu)-$NH_2$ werden analog Beispiel 1a zu Tetradecanoyl-Cys(2[R,S],3-dioctanoyloxy-propyl)-Ala-D-Glu-$NH_2$ umgesetzt; Smp. 170°, $[\alpha]_D^{20}$ = -13° (c = 1,201; $CHCl_3$:Methanol = 1:1), $R_F$ = 0,43 ($CHCl_3$:Methanol:$H_2O$ = 80:20:0,5).

Der Ausgangsstoff wird folgendermassen hergestellt:

Stufe 14.1: 998,2 mg (1,58 mMol) Tetradecanoyl-Cys(2[R,S],3-dihydroxy-propyl)-Ala-D-Glu-$NH_2$ werden mit 565 mg (3,47 mMol) Caprylsäurechlorid analog Stufe 6.4 zu Tetradecanoyl-Cys(2[R,S],3-dioctanoyloxy-propyl)-Ala-D-Glu(OtBu)-$NH_2$ umgesetzt; Smp. 136-138°, $[\alpha]_D^{20}$ = -9° (c = 0,990; $CHCl_3$:Methanol = 1:1), $R_F$ = 0,54 ($CHCl_3$:Methanol =9:1).

Beispiel 15: 565 mg (0,659 mMol) Tetradecanoyl-Cys(2[R,S],3-dihexanoyloxy-propyl)-Ala-D-Glu(OtBu)-$NH_2$ werden analog Beispiel 1a zu Tetradecanoyl-Cys(2[R,S],3-dihexanoyloxy-propyl)-Ala-D-Glu-$NH_2$ umgesetzt; Smp. 176-177°, $[\alpha]_D^{20}$ = -14° (c = 1,130; $CHCl_3$:Methanol = 1:1), $R_F$ = 0,31 ($CHCl_3$:Methanol =

8:2).

Der Ausgangsstoff wird folgendermassen hergestellt:

Stufe 15.1: l g (1,58 mMol) Tetradecanoyl-Cys(2[R,S],3-dihydroxy-propyl)-Ala-D-Glu(OtBu)-$NH_2$ werden analog Stufe 6.4 mit 466 mg (3,48 mMol) Capronsäurechlorid zu Tetradecanoyl-Cys(2[R,S],3-dihexanoyloxy-propyl)-Ala-D-Glu(OtBu)-$NH_2$ umgesetzt; Smp. 144-145°, $[\alpha]_D^{20}$ = -12° (c = 1,281; $CHCl_3$:Methanol = 1:1), $R_F$ = 0,43 ($CHCl_3$:Methanol = 95:5).

Beispiel 16: Analog Beispiel 1a verseift man Palmitoyl-Cys(2[R],3-dilauroyloxy-propyl)-Abu-D-Glu(OtBu)-$NH_2$ mit einer Lösung von 3 Vol.-Teilen Trifluoressigsäure in 7 Vol-Teilen Methylenchlorid bei Raumtemperatur während 3 Stunden. Nach analoger Aufarbeitung erhält man Palmitoyl-Cys(2[R],3-dilauroyloxy-propyl)-Abu-D-Glu-$NH_2$ in Form farbloser Kristalle; Smp. 176-179°, $[\alpha]_D^{20}$ = -15° (c = 0,818, $CHCl_3$:MeOH = 1:1), $R_F$ = 0,155 ($CHCl_3$:MeOH = 9:1).

Der Ausgangsstoff wird folgendermassen hergestellt:

Stufe 16.1: Analog Beispiel 1b erhält man aus Abu-D-Glu(OtBu)-$NH_2$ x HCl und Palmitoyl-Cys(2[R],3-dilauroyloxy-propyl) mit EEDQ Palmitoyl-Cys(2[R],3-dilauroyloxy-propyl)-Abu-D-Glu(OtBu)-$NH_2$ in Form farbloser Kristalle; Smp. 139-141°, $[\alpha]_D^{20}$ = -14° (c = 0,865; $CHCl_3$:MeOH = 1:1), $R_F$ = 0,7 ($CHCl_3$:MeOH = 9:1).

Beispiel 17: Analog Beispiel 1a verseift man Palmitoyl-Cys(2[R],3-dilauroyloxy-propyl)-Val-D-Glu(OtBu)-$NH_2$ mit einer Lösung von 3 Volumenteilen Trifluoressigsäure und 7 Volumenteilen Methylenchlorid bei Raumtemperatur während 3 Stunden. Nach analoger Aufarbeitung erhält man Palmitoyl-Cys(2[R],3-dilauroyloxy-propyl)-Val-D-Glu-$NH_2$ in Form farbloser Kristalle; Smp. 173-175°, $[\alpha]_D^{20}$ = -16° (c = 0,985; $CHCl_3$:MeOH = 1:1), $R_F$ = 0,25 ($CHCl_3$:MeOH = 9:1).

Der Ausgangsstoff wird folgendermassen hergestellt:

Stufe 17.1: Analog Beispiel 1b erhält man aus Val-D-Glu(OtBu)-$NH_2$ X HCl und Palmitoyl-Cys(2[R],3-dilauroyloxy-propyl) mit EEDQ Palmitoyl-Cys(2[R],3-dilauroyloxy-propyl)Val-D-Glu(OtBu)-$NH_2$ in Form farbloser Kristalle; Smp. 142-144°, $[\alpha]_D^{20}$ = -10° (c = 0,791; $CHCl_3$:MeOH = 1:1), $R_F$ = 0,77 ($CHCl_3$:MeOH = 9:1).

Beispiel 18: Analog Beispiel 1a erhält man aus Dodecanoyl-Cys(2[R],3-didecanoyloxy-propyl)-Ala-D-Glu(OtBu)-$NH_2$ mit Trifluoressigsäure in Methylenchlorid Dodecanoyl-Cys(2[R],3-didecanoyloxy-propyl)-Ala-D-Glu-$NH_2$ in Form farbloser Kristalle; Smp. 152-154°, $[\alpha]_D^{20}$ = -16° (c = 1,185; $CHCl_3$:MeOH = 1:1), $R_F$ = 0,35 ($CHCl_3$:MeOH = 85:15).

Der Ausgangsstoff wird folgendermassen hergestellt:

Stufe 18.1: Analog Stufe 6.3 erhält man aus Dodecanoyl-Cys(2[R],3-dihydroxy-propyl) und Ala-D-Glu(OtBu)-$NH_2$ x HCl Dodecanoyl-Cys(2[R],3-dihydroxy-propyl)-Ala-D-Glu(OtBu)-$NH_2$ in Form farbloser Kristalle; Smp. 177-179°, $[\alpha]_D^{20}$ = -27° (c = 0,864; $CHCl_3$:MeOH = 1:1), $R_F$ = 0,605 ($CHCl_3$:MeOH = 5:1).

Stufe 18.2 Analog Stufe 6.4 erhält man aus Dodecanoyl-Cys(2[R],3-dihydroxy-propyl)-Ala-D-Glu(OtBu)-$NH_2$ mit Decanoylchlorid in Pyridin Dodecanoyl-Cys(2[R],3-didecanoyloxy-propyl)-Ala-D-Glu(OtBu)-$NH_2$ in Form farbloser Kristalle; Smp. 129-132°, $[\alpha]_D^{20}$ = -13° (c = 0,882; $CHCl_3$:MeOH = 1:1), $R_F$ = 0,59 ($CHCl_3$:MeOH = 9:1).

Beispiel 19: Analog Beispiel 1a erhält man aus Dodecanoyl-Cys(2[R],3-didodecanoyloxy-propyl)-Ala-D-Glu(OtBu)-$NH_2$ mit Trifluoressigsäure in Methylenchlorid Dodecanoyl-Cys(2[R],3-didodecanoyloxy-propyl)-Ala-D-Glu-$NH_2$; Smp. 149-150°, $[\alpha]_D^{20}$ = -15°, (c = 1,019; $CHCl_3$:Methanol = 1:1), $R_F$ = 0,44 ($CHCl_3$:MeOH) = 85:15).

Der Ausgangsstoff wird folgendermassen hergestellt:

Stufe 19.1: Analog Stufe 6.4 erhält man aus Dodecanoyl-Cys(2[R],3-dihydroxy-propyl)-Ala-D-Glu(OtBu)-$NH_2$ mit Dodecanoylchlorid in Pyridin Dodecanoyl-Cys(2[R],3-didodecanoyloxy-propyl)-Ala-D-Glu(OtBu)-$NH_2$ in Form farbloser Kristalle; Smp. 125-128°, $[\alpha]_D^{20}$ =-12° (c = 0,951; $CHCl_3$:MeOH = 1:1), $R_F$ = 0,425 ($CHCl_3$:MeOH = 95:5).

Beispiel 20: Analog Beispiel 1a erhält man aus Dodecanoyl-Cys(2[R],3-dioctanoyloxy-propyl)-Ala-D-Glu-(OtBu)-$NH_2$ Dodecanoyl-Cys(2[R],3-dioctanoyloxy-propyl)-Ala-D-Glu-$NH_2$ in Form farbloser Kristalle; Smp. 156-158°, $[\alpha]_D^{20}$ = -17° (c = 0,969; $CHCl_3$:MeOH = 1:1), $R_F$ - 0,355 ($CHCl_3$:MeOH = 85:15).

Der Ausgangsstoff wird folgendermassen hergestellt:

Stufe 20.1: Analog Stufe 6.4 erhält man aus Dodecanoyl-Cys(2[R],3-dihydroxy-propyl)-Ala-D-Glu(OtBu)-$NH_2$ mit Octanoylchlorid in Pyridin Dodecanoyl-Cys(2[R],3-dioctanoyloxy-propyl)-Ala-Glu(OtBu)-$NH_2$ in Form farbloser Kristalle; Smp. 130-132°, $[\alpha]_D^{20}$ = -13° (c = 0,874; $CHCl_3$:MeOH = 1:1), $R_F$ = 0,565 ($CHCl_3$:MeOH = 95:5).

Beispiel 21: Aus Palmitoyl-Cys(2[R],3-dilauroyloxy-propyl) und Ala-D-Glu(Arg-OMe)-$NH_2$ x 2 HCl erhält man analog Beispiel 1b Palmitoyl-Cys(2[R],3-dilauroyloxy-propyl)-Ala-D-Glu(Arg-OMe)-$NH_2$ x HCl; $[\alpha]_D^{20}$ = -8° (c = 0,493; Chloroform:Methanol = 1:1), $R_F$ = 0,39 (Chloroform:Methanol:Wasser = 70:30:5), $R_F$ =

0,74 (Chloroform:Methanol:Wasser:Essigsäure = 55:47:13:5).

Das Ausgangsmaterial erhält man folgendermassen:

Stufe 21.1: 29,79 g (75 mMol) Boc-Ala-D-Glu-$NH_2$, 9,49 g (82,5 mMol) N-Hydroxy-succinimid und 19,59 g (75 mMol) Arg-OMe x 2 HCl werden in 300 ml absolutem Dimethylformamid gelöst, dann in der Kälte erst mit 8,26 ml (75 mMol) N-Methyl-morpholin und schliesslich mit 18,57 g (90 mMol) Dicyclohexylcarbodiimid versetzt. Nach 22stündigem Rühren bei Raumtemperatur wird die gelbe Suspension mit 150 ml Essigsäureäthylester verdünnt, der unlösliche Niederschlag abgesaugt, und das Filtrat zur Trockene eingedampft. Der Rückstand wird in 300 ml destilliertem Wasser in der Kälte suspendiert, der Dicyclohexylharnstoff abgesaugt und das Filtrat zur Trockene eingedampft. Das Rohprodukt wird durch Gegenstromverteilung (Unter- und Oberphase je 25 ml) im System n-Butanol:Wasser = 1:1 gereinigt, K-Wert (Verteilungskoeffizient) = 0,40. Das nach 326 Verteilungsschritten in den Gefässen 84-120 enthaltene Material wird gesammelt, das Lösungsmittel bei 30° im Vakuum eingedampft und der Rückstand durch zweimaliges Kristallisieren aus Isopropanol und Essigsäureäthylester-Diäthyläther (1:2) gereinigt. Man erhält Boc-Ala-D-Glu(Arg-OMe)-$NH_2$ x HCl in Form farbloser Kristalle; Smp. 71°, $[\alpha]_D^{20}$ = -18° (c = 0,549; Methanol), $R_F$ = 0,28 (Essigsäureäthylester:n-Butanol:Pyridin:Essigsäure:Wasser = 42:21:21:6:10), $R_F$ = 0,64 (Chloroform:Methanol:Wasser:Essigsäure = 55:47:13:5).

Stufe 21.2: Aus Boc-Ala-D-Glu(Arg-OMe)-$NH_2$ x 2 HCl erhält man durch Spaltung mit 5N HCl in Essigsäureäthylester Ala-D-Glu(Arg-OMe)-$NH_2$ x 2 HCl; $[\alpha]_D^{20}$ = -4° (c = 0,407; Methanol), $R_F$ = 0,16 (Chloroform:Methanol:Wasser:Essigsäure = 55:47:13:5), $R_F$ = 0,03 (Essigsäureäthylester:n-Butanol:Pyridin:Essigsäure:Wasser = 42:21:21:6:10).

Beispiel 22: Aus Palmitoyl-Cys(2[R],3-dilauroyloxy-propyl)-MeAla-CO-D-Glu(OtBu)-$NH_2$ und Trifluoressigsäure in Methylenchlorid erhält man analog Beispiel 1a Palmitoyl-Cys(2[R],3-dilauroyloxy-propyl)-MeAla-D-Glu-$NH_2$; $[\alpha]_D^{20}$ = -12° (c = 0,592; Chloroform:Methanol 1:1), $R_F$ = 0,67 (Chloroform:Methanol:Wasser = 70:30:5), $R_F$ = 0,36 (Chloroform:Isopropanol:Essigsäure = 70:8:2).

Das Ausgangsmaterial erhält man folgendermassen:

Stufe 22.1: Aus Palmitoyl-Cys(2[R],3-dilauroyloxy-propyl) und Me-Ala-D-Glu(OtBu)-$NH_2$ erhält man analog Beispiel 1b Palmitoyl-Cys(2[R],3-dilauroyloxy-propyl)-MeAla-d-Glu(OtBu)-$NH_2$; $R_F$ = 0,31 (Chloroform:Dimethoxyäthan = 4:1), $R_F$ = 0,90 (Chloroform:Methanol:Wasser = 70:30:5).

Beispiel 23: Aus Palmitoyl-Cys(2[R],3-dilauroyloxy-propyl) und Ser-D-Glu(OMe)-OMe erhält man analog Beispiel 1b Palmitoyl-Cys(2[R],3-dilauroyloxy-propyl)-Ser-D-Glu(OMe)-OMe; $[\alpha]_D^{20}$ = -12° (c = 0,470; Chloroform), $R_F$ = 0,91 (Chloroform:Methanol:Wasser = 70:30:5) , $R_F$ = 0,27 (Chloroform:Dimethoxyäthan = 4:1).

Beispiel 24: Aus Palmitoyl-Cys(2[R,S],3-dilauroyloxy-propyl) und Ala-D-Glu($NH_2$)-OnBu x HCl erhält man analog Beispiel 1b Palmitoyl-Cys(2[R,S],3-dilauroyloxy-propyl)-Ala-D-Glu($NH_2$)-OnBu; Smp. 156-159°, $[\alpha]_D^{20}$ = -8° (c = 0,631; Chloroform), $R_F$ = 0,82 (n-Butanol:Essigsäure:Wasser = 75:7,5:21), $R_F$ = 0,12 (Chloroform:Dimethoxyäthan = 4:1).

Beispiel 25: Palmitoyl-Cys(2[R],3-dilauroyloxy-propyl)-Ala-D-Gla{$(OtBu)_2$}-$NH_2$ wird mit Trifluoressigsäure in Methylenchlorid analog Beispiel 1a umgesetzt. Der erhaltene Rückstand wird in wenig Pyridin gelöst, die Lösung wird im Verhältnis 1:10 mit doppelt destilliertem Wasser verdünnt, durch ein Millipore-Filter (0,45 μ) filtriert und lyophilisiert, worauf man Palmitoyl-Cys(2[R],3-dilauroyloxy-propyl)-Ala-D-Gla-$NH_2$ erhält; Smp. 172-174°, $[\alpha]_D^{20}$ =-7° (c = 0,818; Pyridin), $R_F$ = 0,26 (Chloroform:Methanol:Wasser = 70:30:5), $R_F$ = 0,40 (n-Butanol:Essigsäure:Wasser = 75:7,5:21), $R_F$ = 0,64 (Essigsäureäthylester:n-Butanol:Pyridin:Essigsäure:Wasser = 42:21:21:6:10).

Die erhaltene Säure wird in abs. Pyridin gelöst und unter Ausschluss von Feuchtigkeit mit 2 Aequivalenten 0,1 M methanolischer Natriummethylatlösung titriert. Man verdünnt im Verhältnis 1:10 mit doppelt destilliertem Wasser, filtriert durch ein Millipore-Filter (0,45 μ) und lyophilisiert, worauf man Palmitoyl-Cys(2-[R],3-dilauroyloxypropyl)-Ala-D-Gla-$NH_2$-dinatriumsalz erhält.

Das Ausgangsmaterial erhält man folgendermassen:

Stufe 25.1: Aus Z-Ala und D,L-Gla{$(OtBu)_2$}-$NH_2$ erhält man mittels der EEDQ-Methode Z-Ala-D,L-Gla{-$(OtBu)_2$}-$NH_2$ in Form farbloser Nadeln, Smp. 119-120°, $[\alpha]_D^{20}$ = -13° (c = 0,891; Methanol), $R_F$ = 0,74 (Chloroform:Methanol:Wasser = 70:30:5), $R_F$ = 0,68 (n-Butanol:Pyridin:Essigsäure-Wasser = 38:24:8:30).

Stufe 25.2: 10,2 g (20 mMol) Z-Ala-D,L-Gla{$(OtBu)_2$}-$NH_2$ in 300 ml Methanol werden nach Zugabe von 2 g Pd/C (10%ig) unter Normaldruck hydriert. Der Katalysator wird abfiltriert, das Filtrat eingedampft und das erhaltene Diasteromerengemisch als "freie Base" an Kieselgel (60, Merck, 1:100; 15 ml Fraktionen) im System Chloroform:Methanol = 9:1 chromatographiert. Die Fraktionen 60-105 enthalten Ala-L-Gla{$(OtBu)_2$}-$NH_2$ als farbloses Oel; $[\alpha]_D^{20}$ = +1° (c = 0,944; Methanol), $R_F$ = 0,52 (Chloroform:Methanol:Wasser = 70:30:5). Das in den Fraktionen 106-200 enthaltene Diasteromere kann durch vorsichtige Kristallistion aus Dimethoxyäthan (19 ml) rein erhalten werden. Nach 3stündigem Stehen bei -10° wird die Kristallmasse

abgesaugt und erst mit kaltem Dimethoxyäthan, dann mit Petroläther-Diäthyläther (9:1) gewaschen. Nach dem Trocknen erhält man Ala-D-Gla{$(OtBu)_2$}-$NH_2$ in Form farbloser Nadeln; Smp. 139-140°, $[\alpha]_D^{20}$ = +13° (c = 0,931; Methanol), $R_F$ = 0,60 (n-Butanol:Pyridin:Essigsäure:Wasser = 38:24:8:30), $R_F$ = 0,38 (Chloroform:Methanol:Wasser = 70:30:5).

Stufe 25.3: 1,08 g (2,5 mMol) Palmitoyl-Cys(2[R],3-dihydroxy-propyl), 1,13 g (2,75 mMol) Ala-D-Gla{$(OtBu)_2$}-$NH_2$ und 0,80 g (3,25 mMol) EEDQ werden in einer Mischung aus 4,5 ml Dimethylformamid und 1,5 ml Chloroform gelöst und während 22 Stunden unter Stickstoff bei Raumtemperatur stehen gelassen. Die Reaktionslösung wird zur Trockene eingedampft. Der Rückstand wird an der 60fachen Menge Kieselgel [60, Merck, Korngrösse: 0,063-0,200 mm (70-230 mesh ASTM)] im System Chloroform:Methanol = 95:5 (50 ml Fraktionen) chromatographiert. Die das Produkt enthaltenden Fraktionen werden gesammelt. Nach dem Verdampfen der leichtflüchtigen Anteile verbleibt Palmitoyl-Cys(2[R],3-dihydroxy-propyl)-Ala-D-Gla{$(OtBu)_2$}-$NH_2$in Form eines farblosen Oels, das beim Stehen in der Kälte in Drusen kristallisiert; $[\alpha]_D^{20}$ = -30° (c = 1,004; Chloroform), $R_F$ = 0,42 (Chloroform:Methanol = 9:1), $R_F$ = 0,88 (Chloroform:Methanol:Wasser:Essigsäure = 55:47:13:5).

Stufe 25.4: 1,42 g (1,80 mMol) Palmitoyl-Cys(2[R],3-dihydroxy-propyl)-Ala-D-Gla{$(OtBu)_2$}-$NH_2$ werden in 12 ml abs. Pyridin gelöst und unter Stickstoff und Ausschluss von Feuchtigkeit mit 1,28 ml (5,4 mMol) Laurinsäurechlorid in 3 ml Chloroform versetzt. Das Säurechlorid wird so eingetropft, dass eine Temperatur von 20° nicht überschritten wird. Nach 24stündigem Stehen wird die Reaktionslösung mit 3 ml Methanol versetzt und nach 30 Minuten zur Trockene eingedampft. Der harzige Rückstand, bestehend aus zwei Hauptkomponenten (Mono- und Dilauroyl-Verbindung), wird an 200 g Kieselgel [60, Merck, Korngrösse 0,040-0,063 mm (230-400 mesh ASTM)] erst mit Chloroform, dann mit Chloroform-Methanol-Gemischen (99:1 bis 97:3) einer Flash-Chromatographie (W. Clark Still et al., J. Org. Chem. 43, 2923 [1978]; 17 ml Fraktionen; 0,4 bar) unterworfen. Aus den vereinigten Fraktionen 17 bis 190 erhält man Palmitoyl-Cys(2[R]-,3-dilauroyloxy-propyl)-Ala-D-Gla{$(OtBu)_2$}-$NH_2$ als farbloses Oel; $[\alpha]_D^{20}$ = -16° (c = 0,762; Chloroform), $R_F$ = 0,42 (Chloroform:Methanol = 9:1), $R_F$ = 0,93 (Chloroform:Methanol:Wasser = 70:30:5), $R_F$ 0,84 (n-Butanol:Essigsäure:Wasser = 75:7,5:21).

Aus den vereinigten Fraktionen 215-262 erhält man Palmitoyl-Cys(2[R]-hydroxy-3-lauroyloxy-propyl)-Ala-Gla{$(OtBu)_2$}-$NH_2$;$[\alpha]_D^{20}$ = -23° (c = 0,864, Chloroform), $R_F$ = 0,32 (Chloroform:Methanol = 9:1), $R_F$ = 0,84 (Chloroform:Methanol:Wasser = 70:30:5).

Beispiel 26: Aus Palmitoyl-Cys(2[R]-hydroxy-3-lauroyloxy-propyl)-Ala-D-Gla{$(OtBu)_2$}-$NH_2$ erhält man analog Beispiel 1a mit Trifluoressigsäure in Methylenchlorid Palmitoyl-Cys(2[R]-hydroxy-3-lauroyoxy-propyl)-Ala-D-Gla-$NH_2$; $R_F$ = 0,30 (Chloroform:Methanol:Wasser = 70:30:5), $R_F$ 0,43 (Essigsäureäthylester:n-Butanol:Pyridin:Essigsäure-Wasser = 42:21:21:6:10), $[\alpha]_D^{20}$ = -22° (c = 0,318; Dimethylsulfoxid).

Beispiel 27: Aus Palmitoyl-Cys(2,[R],3-dilauroyloxy-propyl)-Abu-D-Glu(OtBu)-OtBu erhält man analog Beispiel 1a Palmitoyl-Cys(2[R],3-dilauroyloxy-propyl)-Abu-D-Glu; Smp. 93-94° (mit Cyclohexan:Aethylmethylketon = 9:1 verrieben), $[\alpha]_D^{20}$ = -7,1° (c = 0,577; DMF), $[\alpha]_D^{20}$ = -9,4° (c = 0,448; $CHCl_3$:MeOH = 1:1), $R_F$ = 0,54 ($CHCl_3$:MeOH:$H_2O$ = 70:30:5), $R_F$ = 0,86 ($CH_2Cl_2$:MeOH:$H_2O$ = 5:5:1).

Das Ausgangsmaterial erhält man folgendermassen:

Stufe 27.1: Aus Palmitoyl-Cys(2[R],3-dilauroyloxy-propyl) und Abu-D-Glu(OtBu)-OtBu x HCl erhält man analog Beispiel 1b unter Zusatz von 4-Dimethylamino-Pyridin Palmitoyl-Cys(2[R],3-dilauroyloxy-propyl)-Abu-D-Glu(OtBu)-OtBu; Smp. 44-45° (aus $CHCl_3$:MeOH = 7:3), $[\alpha]_D^{20}$ = -2,9° (c = 0,381; DMF), $[\alpha]_D^{20}$ = -7,1° (c = -0,325; $CHCl_3$:MeOH = 1:1), $R_F$ = 0,46 ($CH_2Cl_2$:MeOH = 95:5), $R_F$ = 0,10 ($CHCl_3$), $R_F$ = 0,91 (Essigsäureäthylester).

Beispiel 28: Aus Palmitoyl-Cys(2[R],3-dilauroyloxy-propyl)-D-Ala-Ala-D-Glu(OtBu)-$NH_2$ erhält man analog Beispiel 1a Palmitoyl-Cys(2[R],3-dilauroyloxy-propyl)-D-Ala-Ala-D-Glu-$NH_2$; Smp. 173-174° (aus Acetonitril), $[\alpha]_D^{20}$ = -6,4° (c = 1,018; DMF), $[\alpha]_D^{20}$ = -6,8° (c = 0,989; $CHCl_3$:MeOH = 1:1), $R_F$ = 0,67 ($CHCl_3$:MeOH:$H_2O$ = 70:30:5), $R_F$ = 0,40 ($CHCl_3$:MeOH = 4:1).

Das Ausgangsmaterial erhält man folgendermassen:

Stufe 28.1: Aus Palmitoyl-Cys(2[R],3-dihydroxy-propyl) und D-Ala-Ala-D-Glu(OtBu)-$NH_2$ x HCl erhält man analog Stufe 6.3 Palmitoyl-Cys(2[R],3-dihydroxy-propyl)-D-Ala-Ala-D-Glu(OtBu)-$NH_2$; Smp. 209-210° (aus Essigsäureäthylester), $[\alpha]_D^{20}$ = +0,2° (c = 0,655; DMF), $[\alpha]_D^{20}$ = -7,8° (c = 0,934; $CHCl_3$:MeOH = 1:1), $R_F$ = 0,77 ($CHCl_3$:MeOH:$H_2O$ = 70:30:5), $R_F$ = 0,82 ($CHCl_3$:MeOH = 7:3).

Stufe 28.2: Aus Palmitoyl-Cys(2[R],3-dihydroxy-propyl)-D-Ala-Ala-D-Glu(OtBu)-$NH_2$ erhält man analog Stufe 6.4 Palmitoyl-Cys(2[R],3-dilauroyloxy-propyl)-D-Ala-Ala-D-Glu(OtBu)-$NH_2$; Smp. 147-148° (aus Aceton), $[\alpha]_D^{20}$ = -5,3° (c = 1,079; DMF), $[\alpha]_D^{20}$ = -7,8° (c = 1,128; $CHCl_3$:MeOH = 1:1), $R_F$ = 0,42 ($CHCl_3$:MeOH = 9:1), $R_F$ = 0,51 (Essigsäureäthylester).

Beispiel 29: Aus Palmitoyl-Cys(2[R,S],3-dilauroyloxy-propyl) und Abu-D-Glu(OMe)-OMe x HCl erhält man analog Beispiel 1b unter Zusatz von N-Methyl-morpholin Palmitoyl-Cys(2[R,S],3-dilauroyloxy-propyl)-Abu-D-Glu(OMe)-OMe; Smp. 89-91° (aus Acetonitril), $[\alpha]_D^{20}$ = -1,8° (c = 0,569; DMF), $[\alpha]_D^{20}$ = -9,5° (c = 0,474; $CHCl_3$:MeOH = 1:1), $R_F$ = 0,34 ($CHCl_3$:Essigsäureäthylester = 7:3), $R_F$ = 0,95 ($CHCl_3$:MeOH = 9:1).

Beispiel 30: Zu 1,01 g (0,001 Mol) Palmitoyl-Cys(2[R],3-dilauroyloxy-propyl)-Abu-D-Glu in 30 ml absolutem Methanol gibt man unter Rühren und Eiskühlung eine ätherische Diazomethanlösung bis zur bleibenden Gelbfärbung. Die Mischung wird noch 30 Minuten im Eisbad belassen und das überschüssige Diazomethan dann durch Zugabe einiger Tropfen Eisessig zerstört. Nach Eindampfen im Vakuum wird der so erhaltene rohe Dimethylester zweimal aus Acetonitril umkristallisiert. Man erhält Palmitoyl-Cys(2[R],3-dilauroyloxy-propyl)-Abu-D-Glu(OMe)-OMe in Form farbloser Kristalle; $R_F$ = 0,34 ($CHCl_3$:Essigsäureäthylester = 7:3), $R_F$ = 0,95 ($CHCl_3$:MeOH = 9:1).

Beispiel 31: Aus Palmitoyl-Cys(2[R,S],3-dilauroyloxy-propyl)-Ala-D-Glu[Lys(Boc)-D-Ala-OtBu]-OtBu und Trifluoressigsäure in Methylenchlorid erhält man analog Beispiel 1a Palmitoyl-Cys(2[R,S],3-dilauroyloxy-propyl)-Ala-D-Glu(Lys-D-Ala)-OH; $[\alpha]_D^{20}$ = - 14° (c = 0,163; Dichlormethan:Aethanol = 1:1), $R_F$= 0,15 (Chloroform:Methanol:Wasser = 70:30:5), $R_F$ = 0,16 (Essigsäureäthylester:n-Butanol: Pyridin:Essigsäure:Wasser = 42:21:21:6:10), $R_F$ = 0,58 (Chloroform: Methanol:Wasser:Essigsäure = 55:47:13:5).

Das Ausgangsmaterial erhält man folgendermassen:

Stufe 31.1: Aus Palmitoyl-Cys(2[R,S],3-dilauroyloxy-propyl) und Ala-D-Glu[Lys(Boc)-D-Ala-OtBu]-OtBu erhält man analog Beispiel 1b Palmitoyl-Cys(2[R,S],3-dilauroyloxy-propyl)-Ala-D-Glu[Lys(Boc)-D-Ala-OtBu]-OtBu; $[\alpha]_D^{20}$ = -14° (c = 1,9 ; Dichlormethan), $R_F$ = 0,94 (Chloroform: Methanol:Wasser = 70:30:5), $R_F$ = 0,28 (Chloroform: Dimethoxyäthan = 4:1).

Beispiel 32: 615 mg (1 mMol) Palmitoyl-Cys-Ala-D-Glu(OtBu)-$NH_2$, 645 mg (1,1 mMol) 1-Tosyl-2,3-didodecanoyl-D-glycerin und 1,4 g trockenes Kaliumcarbonat erhitzt man in 30 ml absolutem Acetonitril unter Stickstoff 15 Stunden auf 75°. Man erhält nach Eindampfen des Ansatzes, Aufnehmen in Methylenchlorid und mehrmaligem Ausschütteln mit Wasser ein Reaktionsgemisch in der organischen Phase, das nach Trocknen mit $Na_2SO_4$, Filtrieren und Eindampfen über Kieselgel mit $CHCl_3$-EtOH (9:1) chromatographisch gereinigt wird, worauf man das in Beispiel 1b beschriebene Palmitoyl-Cys(2[R], 3-dilauroyloxy-propyl)-Ala-D-Glu(OtBu)-$NH_2$ erhält. Das Ausgangsmaterial, Palmitoyl-Cys-Ala-D-Glu(OtBu)-$NH_2$ erhält man in bekannter Weise aus Palmitoyl-Cys(trityl)-Ala-D-Glu(OtBu)-$NH_2$ durch Behandlung mit Quecksilber (II)-chlorid in Essigsäure [J.Am.Chem.Soc. 87, 4922 (1965); J.Org.Chem. 35, 4148 (1970)] und nachfolgender Spaltung des Quecksilbermercaptids mit $H_2S$. Die Verbindung wird roh zur weiteren Reaktion mit dem Glycerintosylat eingesetzt.

1-Tosyl-2,3-didodecanoyl-D-glycerin erhält man in bekannter Weise aus 1,2-Isopropyliden-glycerin und Tosylchlorid in Pyridin, gefolgt von Verseifung mit 80proz.Essigsäure zum 1-Tosyl-D-glycerin, das in bekannter Weise mit Dodecansäurechlorid acyliert wird. Die Verbindung stellt einen Sirup dar, der im Eisbad kristallisiert, jedoch oberhalb 0° C rasch wieder schmilzt.

**Patentansprüche**

**Patentansprüche für folgende Vertragsstaaten: BE, CH, DE, FR, GB, IT, LU, NL, SE**

**1.** Lipopeptide der Formel

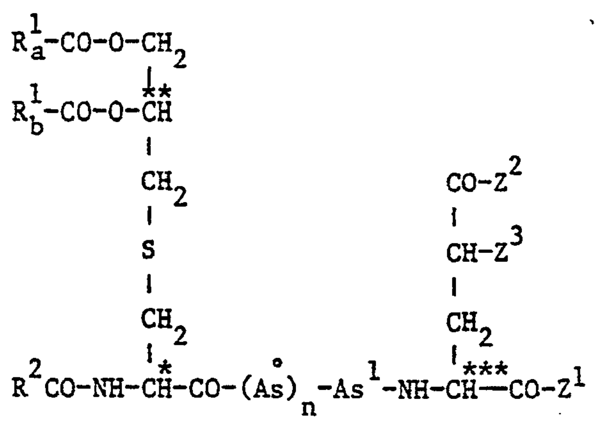

(I)

* = R

** = R oder S

*** = R

worin $R_a^1$ und $R_b^1$ unabhängig voneinander je einen aliphatischen oder cycloaliphatisch-aliphatischen ,

gegebenenfalls durch Sauerstoffunktionen substituierten Kohlenwasserstoffrest mit 7 - 21 C-Atomen, oder der eine der Reste $R_a^1$ -CO und $R_b^1$ -CO Wasserstoff und der andere der Reste $R_a^1$ -CO und $R_b^1$ -CO einen Acylrest, worin $R_a^1$ beziehungsweise $R_b^1$ die obengenannte Bedeutung haben, $R^2$ einen aliphatischen oder cycloaliphatisch-aliphatischen, gegebenenfalls durch Sauerstoffunktionen substituierten Kohlenwasserstoffrest mit 1 - 21 C-Atomen, n = 0 oder 1, $As^{\circ}$ einen Rest der Formel -O-Kw-CO- oder -NH-Kw-CO-, worin Kw für einen aliphatischen Kohlenwasserstoffrest mit höchstens 12 C-Atomen steht, $As^1$ eine D- oder L-α-Aminosäure, $Z^1$ und $Z^2$ unabhängig voneinander Hydroxy oder den N-terminalen Rest einer D- oder L-α-Amino-carbonsäure, einer Amino-niederalkansulfonsäure oder eines Peptids mit maximal 6 Aminosäuren aus der Gruppe der D- oder L-α-Amino-carbonsäuren und der Amino-niederalkansulfonsäuren, und $Z^3$ Wasserstoff oder -CO-$Z^4$, worin $Z^4$ für Hydroxy oder den N-terminalen Rest einer D- oder L-α-Aminosäure, einer Amino-niederalkansulfonsäure oder eines Peptids mit maximal 6 Aminosäuren aus der Gruppe der D- oder L-α-Amino-carbonsäuren und der Amino-niederalkansulfonsäuren steht, bedeuten, und die Amide und Ester von solchen Verbindungen, die Carboxylgruppen aufweisen, wobei die mit * bzw. ** bzw. *** bezeichneten Asymmetrie-Zentren die angegebenen absoluten Konfigurationen besitzen, und die Konfiguration an einem die Gruppe $Z^3$ tragenden asymmetrischen C-Atom R oder S sein kann, und entsprechende Diastereomerengemische, sowie Salze solcher Verbindungen mit mindestens einer salzbildenden Gruppe und gegebenenfalls Komplexsalze dieser Verbindungen.

**2.** Verbindungen nach Anspruch 1, worin $R_a^1$ und $R_b^1$ die gleiche Bedeutung haben und je einen aliphatischen oder cycloaliphatisch-aliphatischen, gegebenenfalls durch Sauerstoffunktionen substituierten Kohlenwasserstoffrest mit 7 - 21 C-Atomen, $Z^1$ und $Z^2$ unabhängig voneinander Hydroxy oder den N-terminalen Rest einer D- oder L-α-Aminosäure oder eines Peptids mit maximal 6 D- oder L-α-Aminosäuren und $Z^3$ Wasserstoff oder -CO-$Z^4$, worin $Z^4$ für Hydroxy oder den N-terminalen Rest einer D- oder L-α-Aminosäure oder eines Peptids mit maximal 6 D- oder L-α-Aminosäuren steht, bedeuten, und Salze von solchen Verbindungen mit mindestens einer salzbildenden Gruppe.

**3.** Verbindungen gemäss Anspruch 2, worin α-Aminosäuren die in der Natur vorkommenden L-α-Aminosäuren oder ihre Antipoden der D-Reihe sind, und Salze von solchen Verbindungen mit mindestens einer salzbildenden Gruppe.

**4.** Verbindungen gemäss einem der Ansprüche 1 - 3 , worin eine Aminosäure $As^1$ ausgewählt ist aus der Gruppe, bestehend aus Gly, Ala, Ser, Abu(α-Amino-buttersäure), Val, αMeAla(α-Methyl-alanin) und Leu, eine Aminosäure im Rest $Z^1$ aus der Gruppe Lys, Orn (Ornithin), Dpm(α,α'-Diamino-pimelinsäure), Gly, Ala, D-Asn und D-Ala und eine Aminosäure $Z^2$ und/oder $Z^4$ aus der Gruppe Lys, Orn, Dpm, Lan (Lanthionin), Gly oder Ala, wobei ein Peptidrest $Z^1$, $Z^2$ oder $Z^4$ aus zwei in solcher Weise ausgewählten Aminosäuren aufgebaut ist, und Salze von solchen Verbindungen mit mindestens einer salzbildenden Gruppe.

**5.** Verbindungen gemäss einem der Ansprüche 1 - 4, in denen $Z^1$ und $Z^2$ in Formel (I) unabhängig voneinander Hydroxy oder den Rest einer Aminosäure und $Z^3$ Wasserstoff oder -CO$Z^4$, worin $Z^4$ für Hydroxy oder den Rest einer Aminosäure steht, bedeuten, und Salze von solchen Verbindungen mit mindestens einer salzbildenden Gruppe.

**6.** Verbindungen gemäss Anspruch 5, in denen $Z^1$ in Formel (I) einen Aminosäurerest bedeutet, welcher ausgewählt ist aus der Gruppe bestehend aus -Lys, -Orn(Ornithinrest), -Dpm(α,α'-Diamino-pimelinsäurerest), -Gly, -Ala, -D-Ala und -D-Asn, und $Z^2$ und/oder $Z^4$ einen Aminosäurerest bedeuten, der ausgewählt ist aus der Gruppe bestehend aus -Lys, -Orn, -Dpm, -Lan(Lanthioninrest), -Gly und -Ala, und Salze von solchen Verbindungen mit mindestens einer salzbildenden Gruppe.

**7.** Verbindungen gemäss einem der Ansprüche 1 - 6, worin die Acylreste $R_a^1$ -CO-,$R_b^1$ -CO- und $R^2$-CO- sich von gesättigten oder ungesättigten, gegebenenfalls oxygenierten Fettsäuren mit 8 - 16 C-Atomen bzw.(im Falle von $R^2$-CO-)2 - 16 C-Atomen ableiten, und Salze von solchen Verbindungen mit mindestens einer salzbildenden Gruppe.

**8.** Verbindungen gemäss einem der Ansprüche 1- 6, worin sich die Acylreste $R_a^1$ -CO-, $R_b^1$ -CO- und $R^2$-CO- von der Capryl-, der Pelargon-, der Caprin-, der Undecyl-, der Laurin-, der Myristin-, der Palmitin-, der Margarin-, der Stearin-, der Arachin-, der Behen-, der Oel-, der Elaidin-, der Linol-, der α- oder β-Eläostearin-, der Stearol- oder der $\alpha$-Linolensäure, oder im Falle von $R^2CO$- auch von der Essig-, Propion-, Butter-, Oenanth- oder Capronsäure ableiten, und Salze von solchen Verbindungen mit mindestens einer salzbildenden Gruppe.

**9.** Verbindungen gemäss einem der Ansprüche 1-6, worin sich die Acylreste $R_a^1$ -CO-, $R_b^1$ -CO und $R^2$-CO- von gesättigten oder ungesättigten cycloaliphatisch-aliphatischen Carbonsäuren mit 8-16 C-Atomen bzw., im Falle von $R^2$-CO-, 2-16 C-Atomen im aliphatischen Teil ableiten und die an beliebiger Stelle in der aliphatischen Kohlenstoffkette durch einen Cycloalkyl- oder Cycloalkenyl-Ring mit 3-8 C-Atomen substituiert oder durch einen Cycloalkylen- oder Cycloalkenylen-Rest von 3-8 C-Atomen unterbrochen sind, und Salze von solchen Verbindungen mit mindestens einer salzbildenden Gruppe.

**10.** Verbindungen gemäss Anspruch 9, worin sich die Acylreste von der Dihydrosterkul-, der Malval-, der Hydnocarpus- oder Chaulmoograsäure ableiten, und Salze von solchen Verbindungen mit mindestens einer salzbildenden Gruppe.

**11.** Verbindungen gemäss einem der Ansprüche 1 - 6, in denen $R_a^1$ -CO-und $R_b^1$ -CO- von $R^2$-CO- verschieden sind und $R_a^1$ -CO- und $R_b^1$ -CO- Lauroyl, Myristoyl,Palmitoyl oder Stearoyl und $R^2$-CO- Stearoyl,Myristoyl,Lauroyl, Caprinoyl oder Capryloyl bedeuten, und Salze von solchen Verbindungen mit mindestens einer salzbildenden Gruppe.

**12.** Die Ester und Amide einer Verbindung gemäss einem der Ansprüche 6 - 11.

**13.** Ester gemäss Anspruch 2 oder 12, worin sich die Estergruppen von gegebenenfalls substituierten, aliphatischen, araliphatischen, aromatischen oder heterocyclischen Alkoholen ableiten, und Salze von solchen Verbindungen mit mindestens einer salzbildenden Gruppe.

**14.** Ester gemäss Anspruch 13, worin sich aliphatische Estergruppen von niederaliphatischen Alkoholen mit 1 - 7 C-Atomen, araliphatische Esterkomponenten von monocyclisch-niederaliphatischen Alkoholen mit 1 - 7 C-Atomen im aliphatischen Teil, aromatische Esterkomponenten von $C_{1-7}$-Alkoxy-, $C_{1-7}$-Alkylamino- oder Di-($C_{1-7}$)-alkylamino- oder Halogenphenolen und heterocyclische Estergruppen von Tetrahydrofuranol oder Tetrahydropyranol ableiten, und Salze von solchen Verbindungen mit mindestens einer salzbildenden Gruppe.

**15.** Ester gemäss Anspruch 13 oder 14, worin Substituenten in der Esterkomponente freie, veresterte oder verätherte Hydroxygruppen darstellen, wobei sich veresterte Gruppen von aliphatischen Carbonsäuren mit 1 - 7 C-Atomen, und verätherte Gruppen von aliphatischen Alkoholen mit 1 - 7 C-Atomen ableiten, und Salze von solchen Verbindungen mit mindestens einer salzbildenden Gruppe.

**16.** Ester gemäss einem der Ansprüche 12 - 15, worin die $\alpha$-Carboxylgruppe der betreffenden Aminosäure verestertist, und Salze von solchen Verbindungen mit mindestens einer salzbildenden Gruppe.

**17.** Mono-, Di-, Tri-, Tetra-, Penta-, Hexa- und Nonaester gemäss einem der Ansprüche 12 - 16, und Salze von solchen Verbindungen mit mindestens einer salzbildenden Gruppe.

**18.** Amide gemäss Anspruch 2 oder 12, worin die Amidgruppe unsubstituiert ist oder sich von einem cyclischen oder acyclischen, primären oder sekundären Amin ableitet, worin der oder die das Amin-Stickstoffatom substituierenden Kohlenwasserstoffrest(e) im Falle der nichtcyclischen Amine Alkylgruppen mit 1 - 7 C-Atomen, und im Falle der cyclischen Amine Alkylengruppen mit 2 - 6 C-Atomen darstellen, und Salze von solchen Verbindungen mit mindestens einer salzbildenden Gruppe.

**19.** Mono-, Di-, Tri-, Tetra-, Penta-, Hexa- und Nonaamide gemäss einem der Ansprüche 11 - 15, und Salze von solchen Verbindungen mit mindestens einer salzbildenden Gruppe.

**20.** Verbindungen gemäss einem der Ansprüche 1 - 19, in denen in Formel (I) des Anspruchs 1 der Rest Kw im Rest $As^{\circ}$ einen unsubstituierten Alkylen- oder Alkylidenrest mit 2 - 6 C-Atomen bedeutet, und Salze von solchen Verbindungen mit mindestens einer salzbildenden Gruppe.

**21.** Verbindungen gemäss Anspruch 20, worin Kw Methylen, Di-, Tri- oder Tetramethylen, Aethyliden, 2-Propyliden, 2,2-Dimethyläthyliden, 2-Butyliden, 3,3-Dimethylpropyliden, 2-Methyläthyliden, 2-Aethyläthyliden oder Pentyliden bedeutet, und Salze von solchen Verbindungen mit mindestens einer salzbildenden Gruppe.

**22.** Verbindungen gemäss Anspruch 20, worin As° den Rest der D- oder L-Milchsäure, von Glycin, Alanin, α-Aminobuttersäure, Valin, Norvalin, Leucin, Isoleucin, Norleucin oder von entsprechenden Aminosäuren der D-Reihe darstellt, und Salze von solchen Verbindungen mit mindestens einer salzbildenden Gruppe.

**23.** Verbindungen nach einem der Ansprüche 1 - 19 , worin in Formel (I) des Anspruchs 1 in der Peptidsequenz n = 0 ist, und Salze von solchen Verbindungen mit mindestens einer salzbildenden Gruppe.

**24.** Verbindungen nach Anspruch 23, worin die Sequenz der Formel

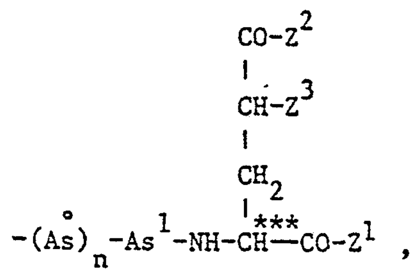

worin n für 0 steht, in Formel (I)
des Anspruchs 1 ausgewählt ist aus der Gruppe, bestehend aus -Ala-D-Glu, -Ala-D-Glu-$NH_2$, -Ala-D-Glu($NH_2$), -Ala-D-Glu-D-Ala-$NH_2$, -Ala-D-Glu($NH_2$)-$NH_2$, -Ala-D-Glu(Ala), -Ala-D-Glu($NH_2$)-D-Ala-$NH_2$, -Ala-D-Glu(Ala)-$NH_2$ und Ala-D-Glu(Gly-taurin)-$NH_2$ sowie entsprechenden Sequenzen, worin -Gly-, -Ser-, -Abu-, -Leu- -αMeAla-(α-Methylalaninrest) oder -Val- anstelle des ersten Alanin-Restes stehen, und Salze von solchen Verbindungen mit mindestens einer salzbildenden Gruppe.

**25.** Verbindungen nach einem der Ansprüche 1 - 21, worin die Sequenz der Formel

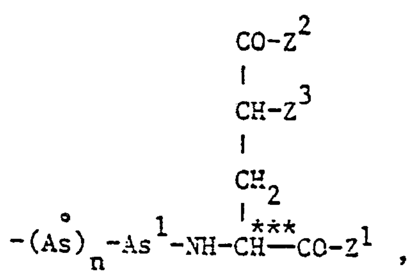

in Formel (I) des Anspruchs 1 ausgewählt ist aus der Gruppe bestehend aus -As°-Ala-D-Glu, -As°-Ala-D-Glu-$NH_2$, -As°-Ala-D-Glu($NH_2$), As°-Ala-D-Glu-D-Ala-$NH_2$, As°-Ala-D-Glu($NH_2$)-$NH_2$, As°-Ala-D-Glu-(Ala), As°-Ala-D-Glu($NH_2$)-D-Ala-$NH_2$ und As°-Ala-D-Glu(Ala)-$NH_2$,worin As° den Rest des D- oder L-Alanins, der D- oder L-Milchsäure, der Glykolsäure oder des Glycins bedeutet, und Salze von solchen Verbindungen mit mindestens einer salzbildenden Gruppe.

**26.** Verbindungen der Formel (I) gemäss Anspruch 1, 2, 7 und 20-22, welche die R-Konfiguration am **Asymetriezentrum in Formel (I) des Anspruchs 1 haben, in denen die Reste $R_b^1$ -CO-,$R_b^1$ -CO- und $R^2$-CO- 8 - 16 C-Atome aufweisen und worin die Sequenz der Formel

$$\begin{array}{rl} & CO-Z^2 \\ & | \\ & CH-Z^3 \\ & | \\ & CH_2 \\ & |\ ^{***} \\ -(\overset{\circ}{As})_n-As^1-NH- & CH-CO-Z^1 \end{array} ,$$

worin n für 0 oder 1 steht, in Formel (I) des Anspruchs 1 ausgewählt ist aus der Gruppe, bestehend aus -Ala-D-Glu, -Ala-D-Glu-$NH_2$, -Ala-D-Glu($NH_2$), -Ala-D-Glu-D-Ala-$NH_2$, -Ala-D-Glu($NH_2$)-$NH_2$, -Ala-D-Glu-(Ala) -Ala-D-Glu($NH_2$)-D-Ala-$NH_2$, -Ala-D-Glu(Ala)-$NH_2$,-As°-Ala-D-Glu, -As°-Ala-D-Glu-$NH_2$, -As°-Ala-D-Glu($NH_2$),As°-Ala-D-Glu-D-Ala-$NH_2$, As°-Ala-D-Glu($NH_2$)-$NH_2$, As°-Ala-D-Glu(Ala), As°-Ala-D-Glu-($NH_2$)-D-Ala-$NH_2$ und As°-Ala-D-Glu(Ala)-$NH_2$, und Salze von solchen Verbindungen mit mindestens einer salzbildenden Gruppe.

**27.** Verbindungen gemäss Anspruch 26, worin Acylreste $R_b^1$ -CO- und $R_b^1$ -CO-von $R^2$-CO- verschieden sind und den Rest der Capryl-, Caprin-, der Laurin-, der Myristin-, der Palmitin-, der Stearin- oder der Oelsäure bedeuten, und Salze von solchen Verbindungen mit mindestens einer salzbildenden Gruppe.

**28.** Ein Lipopeptid gemäss Anspruch 2, ausgewählt aus der Gruppe bestehend aus
Palmitoyl-Cys(2[R],3-dilauroyloxy-propyl)-Ala-D-Glu-$NH_2$,
Palmitoyl-Cys(2[R],3-dipalmitoyloxy-propyl)-Ala-D-Glu(Ala)-$NH_2$,
Palmitoyl-Cys(2[R],3-dilauroyloxy-propyl)-Ala-D-Glu($NH_2$),
Palmitoyl-Cys(2[R],3-dilauroyloxy-propyl)-Abu-D-Glu-$NH_2$ (Abu = $\alpha$-Amino-buttersäure),
Palmitoyl-Cys(2[R],3-dilauroyloxy-propyl)-Ala-D-Glu($NH_2$)-OnBu(nBu = n-Butyl),
Palmitoyl-Cys(2[R],3-dilauroyloxy-propyl)-Ala-D-Glu(OnBu)-$NH_2$,
Palmitoyl-Cys(2[R],3-dilauroyloxy-propyl)-D-Ala-Ala-D-Glu-$NH_2$,

$$\text{Palmitoyl-Cys(2[R],3-dilauroyloxy-propyl)-O-}\underset{CH_3}{\underset{|}{\overset{(D)}{CH}}}\text{-CO-Ala-D-Glu-}NH_2,$$

Palmitoyl-Cys(2[R],3-dilauroyloxy-propyl)-O-$CH_2$CO-Ala-D-Glu-$NH_2$,
Palmitoyl-Cys(2[R],3-dilauroyloxy-propyl)-Ala-D-Glu($NH_2$)-O-$CH_2$-O-CO-C($CH_3$)$_3$,
Palmitoyl-Cys(2[R],3-dilauroyloxy-propyl)-Ser-D-Glu($OCH_3$)-$OCH_3$,
Palmitoyl-Cys(2[R],3-dilauroyloxy-propyl)-Ala-D-Gla-$NH_2$ (Gla = $\gamma$-Carboxy-glutaminsäure),
Palmitoyl-Cys(2[R],3-dilauroyloxy-propyl)-Abu-D-Glu,
Palmitoyl-Cys(2[R],3-dilauroyloxy-propyl)-Val-D-Glu-$NH_2$,
Palmitoyl-Cys(2[R],3-dilauroyloxy-propyl)-$\alpha$MeAla-D-Glu-$NH_2$($\alpha$MeAla = $\alpha$-Methyl-alanin),
Palmitoyl-Cys(2[R],3-dilauroyloxy-propyl)-Ala-D-Glu-(Lys-$OCH_3$)-$NH_2$,
Palmitoyl-Cys(2[R],3-dilauroyloxy-propyl)-Ala-D-Glu(Lys-Lys-$OCH_3$)-$NH_2$,
Palmitoyl-Cys(2[R],3-dilauroyloxy-propyl)-Ala-D-Glu(Arg),
dessen Mono- und Dimethylester und dessen Mono- und Diamid,

$$\text{Palmitoyl-Cys(2[R],3-dilauroyloxy-propyl)-Ala-D-Glu(Lys-O}\underset{CH_3}{\underset{|}{\overset{(D)}{CH}}}\text{-COOH)},$$

dessen Mono- und Dimethylester und dessen Mono- und Diamid,
Palmitoyl-Cys(2[R],3-dilauroyloxy-propyl)-Ala-D-Glu(Tly),
(Tly = 4-Thia-Lysin), dessen Mono- und Dimethylester und dessen Mono- und Diamid, und entsprechende Lipopeptide, in denen anstelle von Palmitoyl am Stickstoff des Cysteinrestes Lauroyl, Caprinoyl,

Capryloyl oder Myristoyl vorhanden sind, und diesen und den oben angeführten Lipopeptiden entsprechende Verbindungen, in denen im Diacyloxypropyl-Rest anstelle von Lauroyl-Resten die Reste der Palmitin-, der Capryl-, der Caprin- und der Myristinsäure vorhanden sind, sowie die all diesen Lipopeptiden entsprechenden Verbindungen, in denen die Konfiguration am chiralen Atom des Diacyloxypropylrestes S statt R ist, und entsprechende Diastereomeren-Gemische von R und S-Verbindungen, sowie gegebenenfalls deren unsubstituierte oder substituierte Amide, und Salze von solchen Verbindungen mit mindestens einer salzbildenden Gruppe.

**29.** Verbindungen nach Anspruch 2, nämlich die Ester von aliphatischen Alkoholen mit 1 - 7 C-Atomen oder die Ester von $C_{1-7}$-Alkanoyloxymethylalkoholen, $C_{1-7}$-Alkanoyloxy-äthylalkoholen ($C_{3-8}$-Cycloalkyl)-carbonyloxymethylalkoholen, ($C_{3-8}$-Cycloalkyl)-carbonyloxy-äthylalkoholen, Propylenglykol, Glyzerin oder eines $C_{1-7}$-Alkoxy-, $C_{1-7}$-Alkylamino-, Di-($C_{1-7}$-Alkyl)-amino- oder Halogenphenols eines der in Anspruch 28 genannten Lipopeptide.

**30.** Verbindungen nach Anspruch 2, nämlich unsubstituierte Amide oder Amide von $C_{1-7}$-Alkylaminen, Pyrrolidin, Piperidin oder Piperazin eines der in Anspruch 28 genannten Lipopeptide.

**31.** Ein Lipopeptid nach Anspruch 2, ausgewählt aus der Gruppe, bestehend aus
Lauroyl-Cys(2[R,S],3-didecanoyloxy-propyl)-Ala-D-Glu-$NH_2$,
Decanoyl-Cys(2(R,S],3-dilauroyloxy-propyl)-Ala-D-Glu-$NH_2$,
Myristoyl-Cys(2[R,S],3-dilauroyloxy-propyl)-Ala-D-Glu-$NH_2$,
Palmitoyl-Cys(2[R,S],3-didecanoyloxy-propyl)-Ala-D-Glu-$NH_2$,
Palmitoyl-Cys(2[R,S],3-didecanoyloxy-propyl)-Abu-D-Glu($OCH_3$)-$OCH_3$,
und aus deren Estern und Amiden gemäss einem der Ansprüche 29 und 30.

**32.** Palmitoyl-Cys(2[R],3-dilauroyloxy-propyl)-Ala-D-Glu(Gly-taurin)-$NH_2$ oder ein Salz davon nach Anspruch 1.

**33.** Palmitoyl-Cys(2[R],3-dilauroyloxy-propyl)-Ala-D-Glu-$NH_2$ oder ein Salz davon nach Anspruch 1.

**34.** Verbindungen nach Anspruch 1 oder 2, nämlich Ammoniumsalze, Alkali- oder Erdalkalimetallsalze von einem der in den Ansprüchen 1-33 beanspruchten sauren Lipopeptide, und pharmazeutisch anwendbare, nichttoxische Säureadditionssalze der in den genannten Ansprüchen beanspruchten basischen Lipopeptide.

**35.** Verbindungen der Formel (I) oder ein pharmazeutisch verwendbares Salz davon nach einem der Ansprüche 1-34 zur Anwendung in einem Verfahren zur Steigerung der körperlichen Abwehrkräfte des menschlichen oder tierischen Körpers.

**36.** Pharmazeutische Präparate, enthaltend eine Verbindung der Formel (I) oder ein pharmazeutisch verwendbares Salz davon nach einem der Ansprüche 1-34 zusammen mit einem-pharmazeutischen Trägermaterial.

**37.** Verwendung der Lipopeptide der Formel (I) oder eines pharmazeutisch verwendbaren Salzes davon gemäss einem der Ansprüche 1-34 zur Herstellung von pharmazeutischen Präparaten, die für die Anwendung zur Erzielung eines immunstimulierenden Effektes oder als Prophylaktika oder Therapeutika gegen Infektionskrankheiten bei Mensch und Tier bestimmt sind.

**38.** Kombinations-Präparate, enthaltend eines oder mehrere der Lipopeptide der Formel (I) oder ein pharmazeutisch verwendbares Salz davon nach einem der Ansprüche 1-34 zusammen mit einem oder mehreren Antibiotika.

**39.** Verfahren zur Herstellung einer Verbindung der Formel I,

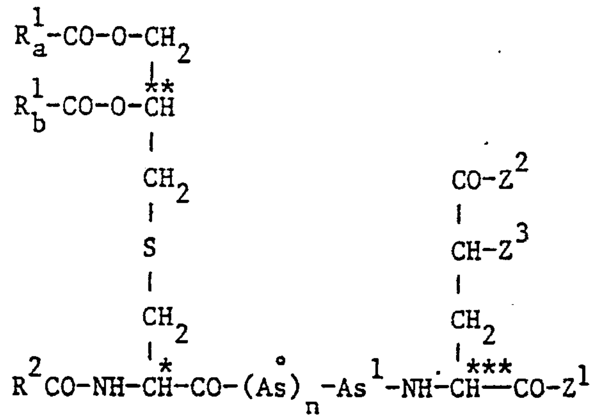

(I)

* = R

** = R oder S

*** = R

worin $R_a^1$ und $R_b^1$ unabhängig voneinander je einen aliphatischen oder cycloaliphatisch-aliphatischen , gegebenenfalls durch Sauerstoffunktionen substituierten Kohlenwasserstoffrest mit 7 - 21 C-Atomen, oder der eine der Reste $R_a^1$ -CO und $R_b^1$ -CO Wasserstoff und der andere der Reste $R_a^1$ -CO und $R_b^1$ -CO einen Acylrest, worin $R_a^1$ beziehungsweise $R_b^1$ die obengenannte Bedeutung haben, $R^2$ einen aliphatischen oder cycloaliphatisch-aliphatischen, gegebenenfalls durch Sauerstoffunktionen substituierten Kohlenwasserstoffrest mit 1 - 21 C-Atomen, n = 0 oder 1, As° einen Rest der Formel -O-Kw-CO- oder -NH-Kw-CO-, worin Kw für einen aliphatischen Kohlenwasserstoffrest mit höchstens 12 C-Atomen steht, $As^1$ eine D- oder L-α-Aminosäure, $Z^1$ und $Z^2$ unabhängig voneinander Hydroxy oder den N-terminalen Rest einer D- oder L-α-Amino-carbonsäure, einer Amino-niederalkansulfonsäure oder eines Peptids mit maximal 6 Aminosäuren aus der Gruppe der D- oder L-α-Amino-carbonsäuren und der Amino-niederalkansulfonsäuren, und $Z^3$ Wasserstoff oder -CO-$Z^4$ worin $Z^4$ für Hydroxy oder den N-terminalen Rest einer D- oder L-α-Aminosäure, einer Amino-niederalkansulfonsäure oder eines Peptids mit maximal 6 Aminosäuren aus der Gruppe der D- oder L-α-Amino-carbonsäuren und der Amino-niederalkansulfonsäuren steht, bedeuten, oder eines Amids oder Esters einer solchen Verbindung, die mindestens eine Carboxylgruppe aufweist, wobei die mit * bzw. ** bzw. *** bezeichneten Asymmetrie-Zentren die angegebenen absoluten Konfigurationen besitzen, und die Konfiguration an einem die Gruppe $Z^3$ tragenden asymmetrischen C-Atom R oder S sein kann, oder eines entsprechenden Diastereomerengemisches, oder eines Salzes einer solchen Verbindung mit mindestens einer salzbildenden Gruppe, oder gegebenenfalls eines Komplexsalzes einer solchen Verbindung, dadurch gekennzeichnet, dass man

a) in einer der Formel (I) entsprechenden Verbindung oder einem Salz derselben, worin die Substituenten die obengenannten Bedeutungen haben mit der Massgabe, dass die Peptidkette

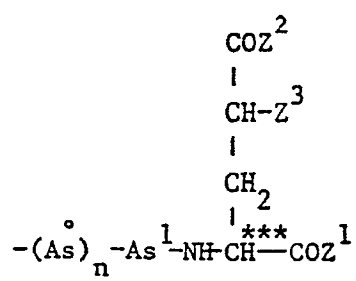

(II)

mindestens eine geschützte funktionelle Gruppe enthält, die Schutzgruppe(n) abspaltet, oder

b) eine Verbindung der Formel I, worin mindestens einer der Reste $R_a^1$ -CO-, $R_b^1$ -CO- und $R^2$-CO-für Wasserstoff steht und die übrigen Substituenten die obengenannten Bedeutungen haben mit der Massgabe, dass in diesem Ausgangsmaterial vorhandene freie funktionelle Gruppen mit Ausnahme der an der Reaktion teilnehmenden Hydroxy- und/oder Aminogruppe(n), wenn nötig, in geschützter Form vorliegen, oder ein Salz derselben, mit einer Säure $R_a^1$ -COOH, $R_b^1$ -COOH beziehungsweise $R^2$-COOH oder einem reaktionsfähigen Carbonsäurederivat davon acyliert und vorhandene Schutzgruppen abspaltet, oder

c) eine Amidbindung einer Verbindung der Formel I durch Umsetzung eines entsprechenden Bruchstücks einer Verbindung der Formel I mit einer freien Carboxylgruppe oder eines reaktionsfähigen Säurederivats davon mit einem komplementierenden Bruchstück mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen, wenn nötig, in geschützter Form vorliegen, herstellt und vorhandene Schutzgruppen abspaltet, oder

d) dass man in einer Verbindung der Formel (I), worin zumindestens eine freie Carboxylgruppe vorhanden ist, die freie(n) Carboxylgruppe(n) verestert oder amidiert und/oder in einer Verbindung der Formel (I), worin zumindestens eine Estergruppe vorhanden ist, die Estergruppe(n) verseift, oder

e) eine Verbindung der Formel III,

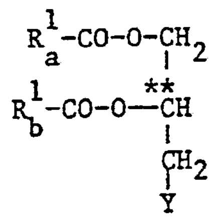

** = R oder S

(III)

worin $R_a^1$ und $R_b^1$ die obengenannten Bedeutungen haben und Y für eine nucleofuge Gruppe steht, mit einer Verbindung der Formel IV,

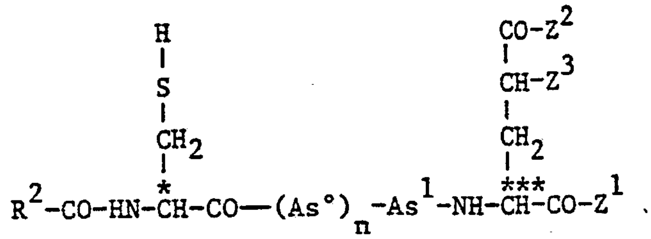

* = R

*** = R

(IV)

worin die Substituenten die obengenannten Bedeutungen haben, wobei freie funktionelle Gruppen mit Ausnahme der an der Reaktion teilnehmenden Mercaptogruppe, wenn nötig, durch leicht abspaltbare Schutzgruppen geschützt sind, oder mit einem reaktionsfähigen Derivat einer Verbindung der Formel IV umsetzt und vorhandene Schutzgruppen abspaltet, oder

(f) eine Verbindung der Formel V,

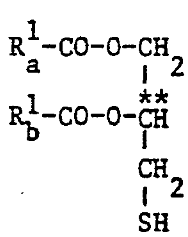

** = R oder S

(V)

worin $R_a^1$ und $R_b^1$ die obengenannten Bedeutungen haben, oder ein reaktionsfähiges Derivat dieser Verbindung mit einer Verbindung der Formel VI,

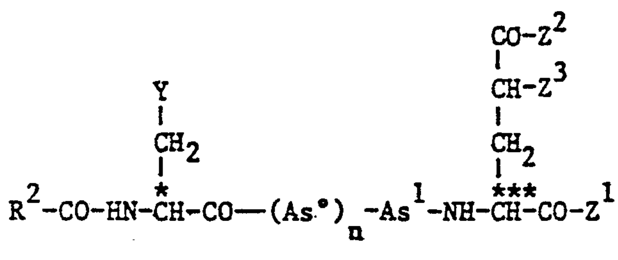

* = R

*** = R

(VI)

worin Y für eine nucleofuge Gruppe steht und die übrigen Substituenten die obengenannten Bedeutungen haben, wobei freie funktionelle Gruppen, wenn nötig, in geschützter Form vorliegen, umsetzt und vorhandene Schutzgruppen abspaltet, und, wenn erwünscht, nach Durchführung einer der Verfahrensvarianten a - f) eine erhaltene Verbindung der Formel I mit mindestens einer salzbildenden Gruppe in ein Salz oder Komplexsalz überführt oder ein erhaltenes Salz oder Komplexsalz in die freie Verbindung überführt und, wenn erwünscht, erhaltene Isomerengemische auftrennt.

**Patentansprüche für folgenden Vertragsstaat: AT**

1. Verfahren zur Herstellung einer Verbindung der Formel I,

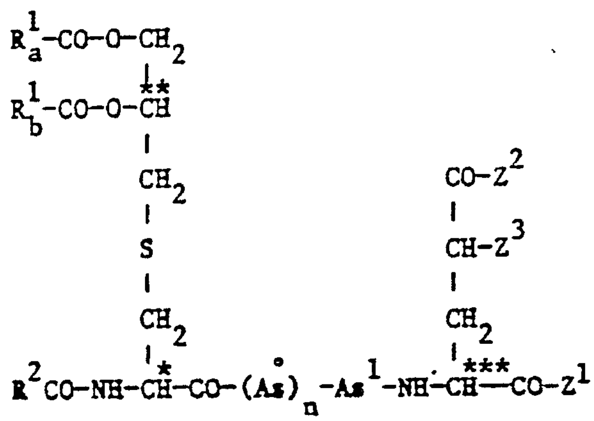

(I)

* = R

** = R oder S

*** = R

worin $R_a^1$ und $R_b^1$ unabhängig voneinander je einen aliphatischen oder cycloaliphatisch-aliphatischen , gegebenenfalls durch Sauerstoffunktionen substituierten Kohlenwasserstoffrest mit 7 - 21 C-Atomen, oder der eine der Reste $R_a^1$ -CO und $R_b^1$ -CO Wasserstoff und der andere der Reste $R_a^1$ -CO und $R_b^1$ -CO einen Acylrest, worin $R_a^1$ beziehungsweise $R_b^1$ die obengenannte Bedeutung haben; $R^2$ einen aliphatischen oder cycloaliphatisch-aliphatischen, gegebenenfalls durch Sauerstoffunktionen substituierten Kohlenwasserstoffrest mit 1 - 21 C-Atomen, n = 0 oder 1, $As^0$ einen Rest der Formel -O-Kw-CO- oder -NH-Kw-CO-, worin Kw für einen aliphatischen Kohlenwasserstoffrest mit höchstens 12 C-Atomen steht, $As^1$ eine D- oder L-$\alpha$-Aminosäure, $Z^1$ und $Z^2$ unabhängig voneinander Hydroxy oder den N-terminalen Rest einer D- oder L-$\alpha$-Amino-carbonsäure, einer Amino-niederalkansulfonsäure oder eines Peptids mit maximal 6 Aminosäuren aus der Gruppe der D- oder L-$\alpha$-Amino-carbonsäuren und der Amino-niederalkansulfonsäuren, und $Z^3$ Wasserstoff oder -CO-$Z^4$ worin $Z^4$ für Hydroxy oder den N-terminalen Rest einer D- oder L-$\alpha$-Aminosäure, einer Amino-niederalkansulfonsäure oder eines Peptids mit maximal 6 Aminosäuren aus der Gruppe der D- oder L-$\alpha$-Amino-carbonsäuren und der Amino-niederalkansulfonsäuren steht, bedeuten, oder eines Amids oder Esters einer solchen Verbindung, die mindestens eine Carboxylgruppe aufweist, wobei die mit * bzw. ** bzw. *** bezeichneten Asymmetrie-Zentren die angegebenen absoluten Konfigurationen besitzen, und die Konfiguration an einem die Gruppe $Z^3$ tragenden asymmetrischen C-Atom R oder S sein kann, oder eines entsprechenden Diastereomerengemisches, oder eines Salzes einer solchen Verbindung mit mindestens einer salzbildenden Gruppe, oder gegebenenfalls eines Komplexsalzes einer solchen Verbindung, dadurch gekennzeichnet, dass man

a) in einer der Formel (I) entsprechenden Verbindung oder einem Salz derselben, worin die Substituenten die obengenannten Bedeutungen haben mit der Massgabe, dass die Peptidkette

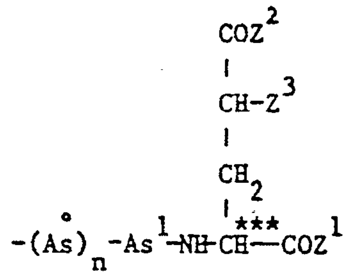

(II)

mindestens eine geschützte funktionelle Gruppe enthält, die Schutzgruppe(n) abspaltet, oder
b) eine Verbindung der Formel I, worin mindestens einer der Reste $R_a^1$-CO-, $R_b^1$-CO- und $R^2$-CO- für Wasserstoff steht und die übrigen Substituenten die obengenannten Bedeutungen haben mit der Massgabe, dass in diesem Ausgangsmaterial vorhandene freie funktionelle Gruppen mit Ausnahme der an der Reaktion teilnehmenden Hydroxy- und/oder Aminogruppe(n), wenn nötig, in geschützter Form vorliegen, oder ein Salz derselben, mit einer Säure $R_a^1$-COOH, $R_b^1$-COOH beziehungsweise $R^2$-COOH oder einem reaktionsfähigen Carbonsäurederivat davon acyliert und vorhandene Schutzgruppen abspaltet, oder
c) eine Amidbindung einer Verbindung der Formel I durch Umsetzung eines entsprechenden Bruchstücks einer Verbindung der Formel I mit einer freien Carboxylgruppe oder eines reaktionsfähigen Säurederivats davon mit einem komplementierenden Bruchstück mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen, wenn nötig, in geschützter Form vorliegen, herstellt und vorhandene Schutzgruppen abspaltet, oder
d) dass man in einer Verbindung der Formel (I), worin zumindestens eine freie Carboxylgruppe vorhanden ist, die freie(n) Carboxylgruppe(n) verestert oder amidiert und/oder in einer Verbindung der Formel (I), worin zumindestens eine Estergruppe vorhanden ist, die Estergruppe(n) verseift, oder
e) eine Verbindung der Formel III,

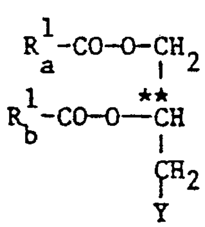

** = R oder S

(III)

worin $R_a^1$ und $R_b^1$ die obengenannten Bedeutungen haben und Y für eine nucleofuge Gruppe steht, mit einer Verbindung der Formel IV,

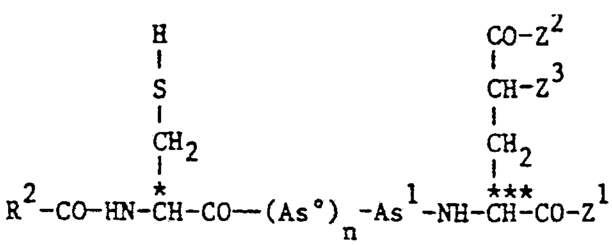

* = R

*** = R

(IV)

worin die Substituenten die obengenannten Bedeutungen haben, wobei freie funktionelle Gruppen mit Ausnahme der an der Reaktion teilnehmenden Mercaptogruppe, wenn nötig, durch leicht abspaltbare Schutzgruppen geschützt sind, oder mit einem reaktionsfähigen Derivat einer Verbindung der Formel IV umsetzt und vorhandene Schutzgruppen abspaltet, oder
(f) eine Verbindung der Formel V,

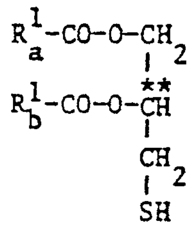

** = R oder S

(V)

worin $R_a^1$ und $R_b^1$ die obengenannten Bedeutungen haben, oder ein reaktionsfähiges Derivat dieser Verbindung mit einer Verbindung der Formel VI,

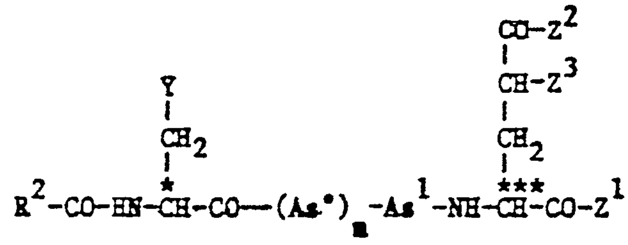

* = R

*** = R (VI)

worin Y für eine nucleofuge Gruppe steht und die übrigen Substituenten die obengenannten Bedeutungen haben, wobei freie funktionelle Gruppen, wenn nötig, in geschützter Form vorliegen, umsetzt und vorhandene Schutzgruppen abspaltet, und, wenn erwünscht, nach Durchführung einer der Verfahrensvarianten a - f) eine erhaltene Verbindung der Formel I mit mindestens einer salzbildenden Gruppe in ein Salz oder Komplexsalz überführt oder ein erhaltenes Salz oder Komplexsalz in die freie Verbindung überführt und, wenn erwünscht, erhaltene Isomerengemische auftrennt.

**2.** Verfahren nach Anspruch 1, dadurch gekennzeichnet, dass man die Ausgangsstoffe so wählt, dass man eine Verbindung der Formel I,worin $R_a^1$ und $R_b^1$ die gleiche Bedeutung haben und je einen aliphatischen oder cycloaliphatisch-aliphatischen, gegebenenfalls durch Sauerstofffunktionen substituierten Kohlenwasserstoffrest mit 7 - 21 C-Atomen, $Z^1$ und $Z^2$ unabhängig voneinander Hydroxy oder den N-terminalen Rest einer D- oder L-α-Aminosäure oder eines Peptids mit maximal 6 D- oder L-α-Aminosäuren und $Z^3$ Wasserstoff oder -CO-$Z^4$, worin $Z^4$ für Hydroxy oder den N-terminalen Rest einer D- oder L-α-Aminosäure oder eines Peptids mit maximal 6 D- oder L-α--Aminosäuren steht, bedeuten, oder ein Salz einer solchen Verbindung mit mindestens einer salzbildenden Gruppe herstellt.

**3.** Verfahren gemäss Anspruch 2, dadurch gekennzeichnet, dass man die Ausgangsstoffe so wählt, dass man eine Verbindung der Formel I, worin α-Aminosäuren die in der Natur vorkommenden L-α-Aminosäuren oder ihre Antipoden der D-Reihe sind, oder ein Salz einer solchen Verbindung mit mindestens einer salzbildenden Gruppe herstellt.

**4.** Verfahren gemäss einem der Ansprüche 1 - 3, dadurch gekennzeichnet, dass man die Ausgangsstoffe so wählt, dass man eine Verbindung der Formel (I), worin eine Aminosäure $As^1$ ausgewählt ist aus der Gruppe, bestehend aus Gly, Ala, Ser, Abu(α-Amino-buttersäure), Val, α-MeAla(α-Methyl-alanin) und Leu, eine Aminosäure im Rest $Z^1$ aus der Gruppe Lys, Orn (Ornithin), Dpm(α,α'-Diamino-pimelinsäure), Gly, Ala, D-Asn und D-Ala und eine Aminosäure $Z^2$ und/oder $Z^4$ aus der Gruppe Lys, Orn, Dpm, Lan-(Lanthionin), Gly oder Ala, wobei ein Peptidrest $Z^1$, $Z^2$ oder $Z^4$ aus 2 in solcher Weise ausgewählten Aminosäuren aufgebaut ist, oder ein Salz einer solchen Verbindung mit mindestens einer salzbildenden Gruppe herstellt.

**5.** Verfahren gemäss einem der Ansprüche 1 - 4, dadurch gekennzeichnet, dass man die Ausgangsstoffe so wählt, dass man eine Verbindung der Formel I, in der $Z^1$ und $Z^2$ in Formel (I) unabhängig voneinander Hydroxy oder den Rest einer Aminosäure und $Z^3$ Wasserstoff oder -$COZ^4$, worin $Z^4$ für Hydroxy oder den Rest einer Aminosäure steht, bedeuten, oder ein Salz einer solchen Verbindung mit mindestens einer salzbildenden Gruppe herstellt.

6. Verfahren gemäss Anspruch 5, dadurch gekennzeichnet, dass man die Ausgangsstoffe so wählt, dass man eine Verbindung der Formel I, in der $Z^1$ in Formel (I) einen Aminosäurerest bedeutet, welcher ausgewählt ist aus der Gruppe bestehend aus -Lys, -Orn(Ornithinrest), -Dpm($\alpha$,$\alpha$'-Diamino-pimelinsäurerest), -Gly, -Ala, -D-Ala und -D-Asn, und $Z^2$ und/oder $Z^4$ einen Aminosäurerest bedeuten, der ausgewählt ist aus der Gruppe bestehend aus -Lys, -Orn, -Dpm, -Lan(Lanthioninrest), -Gly und -Ala, oder ein Salz einer solchen Verbindung mit mindestens einer salzbildenden Gruppe herstellt.

7. Verfahren gemäss einem der Ansprüche 1 - 6, dadurch gekennzeichnet, dass man die Ausgangsstoffe so wählt, dass man eine Verbindung der Formel I, worin die Acylreste $R_a^1$ -CO-, $R_b^1$ -CO- und $R^2$-CO- sich von gesättigten oder ungesättigten, gegebenenfalls oxygenierten Fettsäuren mit 8 - 16 C-Atomen bzw. (im Falle von $R^2$-CO-)2 - 16 C-Atomen ableiten, oder ein Salz einer solchen Verbindung mit mindestens einer salzbildenden Gruppe herstellt.

8. Verfahren gemäss einem der Ansprüche 1 - 6, dadurch gekennzeichnet, dass man die Ausgangsstoffe so wählt, dass man eine Verbindung der Formel I worin sich die Acylreste $R_a^1$ -CO-, $R_b^1$ -CO- und $R^2$-CO- von der Capryl-, der Pelargon-, der Caprin-, der Undecyl-, der Laurin-, der Myristin-, der Palmitin-, der Margarin-, der Stearin-, der Arachin-, der Behen-, der Oel-, der Elaidin-, der Linol-, der $\alpha$- oder $\beta$-Eläostearin-, der Stearol- oder der $\alpha$-Linolensäure, oder im Falle von $R^2$CO- auch von der Essig-, Propion-, Butter-, Oenanth- oder Capronsäure ableiten, oder ein Salz einer solchen Verbindung mit mindestens einer salzbildenden Gruppe herstellt.

9. Verfahren gemäss einem der Ansprüche 1 - 6, dadurch gekennzeichnet, dass man die Ausgangsstoffe so wählt, dass man eine Verbindung der Formel I,worin sich die Acylreste $R_a^1$ -CO-, $R_b^1$ -CO und $R^2$-CO- von gesättigten oder ungesättigten cycloaliphatisch-aliphatischen Carbonsäuren mit 8-16 C-Atomen bzw., im Falle von $R^2$-CO-, 2-16 C-Atomen im aliphatischen Teil ableiten und die an beliebiger Stelle in der aliphatischen Kohlenstoffkette durch einen Cycloalkyl- oder Cycloalkenyl-Ring mit 3-8 C-Atomen substituiert oder durch einen Cycloalkylen- oder Cycloalkenylen-Rest von 3-8 C-Atomen unterbrochen sind, oder ein Salz einer solchen Verbindung mit mindestens einer salzbildenden Gruppe herstellt.

10. Verfahren gemäss einem der Ansprüche 1 - 6, dadurch gekennzeichnet dass man die Ausgangsstoffe so wählt, dass man eine Verbindung der Formel I,in der $R_a^1$ -CO- und $R_b^1$ -CO- von $R^2$-CO- verschieden sind und $R_a^1$ -CO- und $R_b^1$ -CO- Lauroyl, Myristoyl, Palmitoyl oder Stearoyl und $R^2$-CO-Stearoyl, Myristoyl, Lauroyl, Caprinoyl oder Capryloyl bedeuten, oder ein Salz einer solchen Verbindung mit mindestens einer salzbildenden Gruppe herstellt.

11. Verfahren gemäss einem der Ansprüche 2 und 6-10, dadurch gekennzeichnet, dass man die Ausgangsstoffe so wählt, dass man einen Ester der Formel I, worin sich die Estergruppen von niederaliphatischen Alkoholen mit 1-7 C-Atomen, von monocyclisch-niederaliphatischen Alkoholen mit 1-7 C-Atomen im aliphatischen Teil, von $C_{1-7}$ -Alkoxy-, $C_{1-7}$-Alkylamino- oder Di-($C_{1-7}$)-alkyl-amino- oder Halogenphenolen oder von Tetrahydrofuranol oder Tetrahydropyranol ableiten, oder ein Salz einer solchen Verbindung mit mindestens einer salzbildenden Gruppe herstellt.

12. Verfahren gemäss einem der Ansprüche 2 und 6-10, dadurch gekennzeichnet, dass man die Ausgangsstoffe so wählt, dass man Amide der Formel I, worin die Amidgruppe unsubstituiert ist oder sich von einem cyclischen oder acyclischen, primären oder sekundären Amin ableitet, worin der oder die das Amin-Stickstoffatom substituierenden Kohlenwasserstoffrest(e) im Falle der nicht-cyclischen Amine Alkylgruppen mit 1-7 C-Atomen, und im Falle der cyclischen Amine Alkylengruppen mit 2-6 C-Atomen darstellen, oder ein Salz einer solchen Verbindung mit mindestens einer salzbildenden Gruppe herstellt.

13. Verfahren gemäss einem der Ansprüche 1 - 12, dadurch gekennzeichnet, dass man die Ausgangsstoffe so wählt, dass man eine Verbindung der Formel I, in der in Formel (I) des Anspruchs 1 der Rest Kw im Rest $As^{\circ}$ einen unsubstituierten Alkylen- oder Alkylidenrest mit 2 - 6 C-Atomen bedeutet, oder ein Salz einer solchen Verbindung mit mindestens einer salzbildenden Gruppe herstellt.

14. Verfahren gemäss Anspruch 13, dadurch gekennzeichnet, dass man die Ausgangsstoffe so wählt, dass man eine Verbindung der Formel I,worin $As^{\circ}$ den Rest der D- oder L-Milchsäure, von Glycin, Alanin, $\alpha$-Aminobuttersäure, Valin, Norvalin, Leucin, Isoleucin, Norleucin oder von entsprechenden Aminosäuren der D-Reihe darstellt, oder ein Salz einer solchen Verbindung mit mindestens einer salzbildenden

Gruppe herstellt.

**15.** Verfahren nach einem der Ansprüche 1 - 12, dadurch gekennzeichnet, dass man die Ausgangsstoffe so wählt, dass man eine Verbindung der Formel I,worin in Formel (I) des Anspruchs 1 in der Peptidsequenz n = 0 ist, oder ein Salz einer solchen Verbindung mit mindestens einer salzbildenden Gruppe herstellt.

**16.** Verfahren nach Anspruch 15, dadurch gekennzeichnet, dass man die Ausgangsstoffe so wählt, dass man eine Verbindung der Formel I,worin die Sequenz der Formel

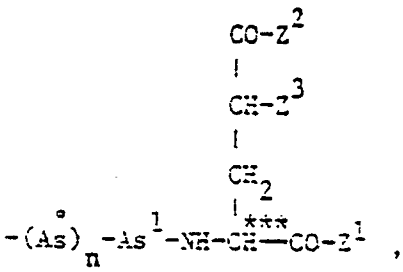

worin n für 0 steht, in Formel (I) des Anspruchs 1 ausgewählt ist aus der Gruppe, bestehend aus -Ala-D-Glu, -Ala-D-Glu-$NH_2$, -Ala-D-Glu($NH_2$), -Ala-D-Glu-D-Ala-$NH_2$, -Ala-D-Glu($NH_2$)-$NH_2$, -Ala-D-Glu-(Ala), -Ala-D-Glu($NH_2$)-D-Ala-$NH_2$, -Ala-D-Glu(Ala)-$NH_2$ und -Ala-D-Glu(Gly-taurin)-$NH_2$ sowie entsprechenden Sequenzen, worin -Gly-, -Ser-, -Abu-, -Leu-, -αMeAla-(α-Methylalaninrest) oder -Val- anstelle des ersten Alanin-Restes stehen, oder ein Salz einer solchen Verbindung mit mindestens einer salzbildenden Gruppe herstellt.

**17.** Verfahren nach einem der Ansprüche 1 - 12, dadurch gekennzeichnet, dass man die Ausgangsstoffe so wählt, dass man eine Verbindung der Formel (I), worin die Sequenz der Formel

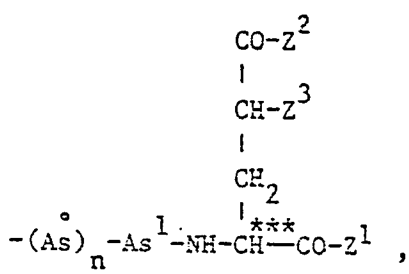

in Formel (I) des Anspruchs 1 ausgewählt ist aus der Gruppe bestehend aus -As°-Ala-D-Glu, -As°-Ala-D-Glu-$NH_2$, -As°-Ala-D-Glu($NH_2$), As°-Ala-D-Glu-D-Ala-$NH_2$, As°-Ala-D-Glu($NH_2$)-$NH_2$, As°-Ala-D-Glu-(Ala), As°-Ala-D-Glu($NH_2$)-D-Ala-$NH_2$ und As°-Ala-D-Glu(Ala)-$NH_2$,worin As° den Rest des D- oder L-Alanins, der D- oder L-Milchsäure, der Glykolsäure oder des Glycins bedeutet, oder ein Salz einer solchen Verbindung mit mindestens einer salzbildenden Gruppe herstellt.

**18.** Verfahren gemäss Anspruch 1, 2, 7, 13 oder 14, dadurch gekennzeichnet, dass man die Ausgangsstoffe so wählt, dass man eine Verbindung der Formel I, welche die R-Konfiguration am **Asymmetriezentrum in Formel (I) des Anspruchs 1 hat, in denen die Reste $R_a^1$-CO-, $R_b^1$-CO- und $R^2$-CO- 8-16 C-Atome aufweisen und worin die Sequenz der Formel

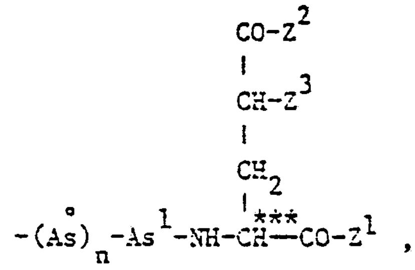

worin n für 0 oder 1 steht, in Formel I des Anspruchs 1 ausgewählt ist aus der Gruppe, bestehend aus -Ala-D-Glu, -Ala-D-Glu-$NH_2$, -Ala-D-Glu($NH_2$), -Ala-D-Glu-D-Ala-$NH_2$, -Ala-D-Glu($NH_2$)-$NH_2$, -Ala-D-Glu-(Ala), -Ala-D-Glu($NH_2$)-D-Ala-$NH_2$, -Ala-D-Glu(Ala)-$NH_2$,-As°-Ala-D-Glu, -As°-Ala-D-Glu-$NH_2$, -As°-Als-D-Glu($NH_2$), As°-Ala-D-Glu-D-Ala-$NH_2$, As°-Ala-D-Glu($NH_2$)-$NH_2$, As°-Ala-D-Glu(Ala), As°-Ala-D-Glu-($NH_2$)-D-Ala-$NH_2$ und As°-Ala-D-Glu(Ala)-$NH_2$, oder ein Salz einer solchen Verbindung mit mindestens einer salzbildenden Gruppe herstellt.

**19.** Verfahren gemäss Anspruch 18, dadurch gekennzeichnet, dass man die Ausgangsstoffe so wählt, dass man eine Verbindung der Formel I, worin Acylreste $R_a^1$ -CO- und $R_b^1$ -CO- von $R^2$-CO- verschieden sind und den Rest der Capryl-, Caprin-, der Laurin, der Myristin-, der Palmitin-, der Stearin- oder der Oelsäure bedeuten, oder ein Salz einer solchen Verbindung mit mindestens einer salzbildenden Gruppe herstellt.

**20.** Verfahren gemäss Anspruch 2, dadurch gekennzeichnet, dass man die Ausgangsstoffe so wählt, dass man eine Verbindung der Formel I, ausgewählt aus der Gruppe bestehend aus
Palmitoyl-Cys(2[R],3-dilauroyloxy-propyl)-Ala-D-Glu-$NH_2$,
Palmitoyl-Cys(2[R],3-dipalmitoyloxy-propyl)-Ala-D-Glu(Ala)$NH_2$,
Palmitoyl-Cys(2[R],3-dilauroyloxy-propyl)-Ala-D-Glu($NH_2$),
Palmitoyl-Cys(2[R],3-dilauroyloxy-propyl)-Abu-D-Glu-$NH_2$ (Abu = α-Amino-buttersäure),
Palmitoyl-Cys(2[R],3-dilauroyloxy-propyl)-Ala-D-Glu($NH_2$)-OnBu (nBu = n-Butyl),
Palmitoyl-Cys(2[R],3-dilauroyloxy-propyl)-Ala-D-Glu(OnBu)-$NH_2$,
Palmitoyl-Cys(2[R],3-dilauroyloxy-propyl)-D-Ala-Ala-D-Glu-$NH_2$,

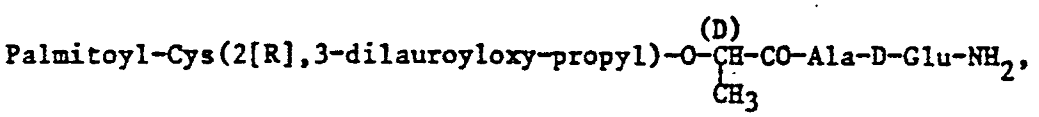

Palmitoyl-Cys(2[R],3-dilauroyloxy-propyl)-O-$CH_2$CO-Ala-D-Glu-$NH_2$,
Palmitoyl-Cys(2[R],3-dilauroyloxy-propyl)-Ala-D-Glu($NH_2$)-O-$CH_2$-O-CO-C($CH_3$)$_3$,
Palmitoyl-Cys(2[R],3-dilauroyloxy-propyl)-Ser-D-Glu($OCH_3$)-$OCH_3$,
Palmitoyl-Cys(2[R],3-dilauroyloxy-propyl)-Ala-D-Gla-$NH_2$ Gla = γ-Carboxy-glutaminsäure),
Palmitoyl-Cys(2[R],3-dilauroyloxy-propyl)-Abu-D-Glu,
Palmitoyl-Cys(2[R],3-dilauroyloxy-propyl)-Val-D-Glu-$NH_2$,
Palmitoyl-Cys(2[R],3-dilauroyloxy-propyl)-αMeAla-D-Glu-$NH_2$ (αMeAla = α-Methyl-alanin),
Palmitoyl-Cys(2[R],3-dilauroyloxy-propyl)-Ala-D-Glu-(Lys-$OCH_3$)-$NH_2$,
Palmitoyl-Cys(2[R],3-dilauroyloxy-propyl)-Ala-D-Glu(Lys-Lys-$OCH_3$)-$NH_2$,
Palmitoyl-Cys(2[R],3-dilauroyloxy-propyl)-Ala-D-Glu(Arg),
dessen Mono- und Dimethylester und dessen Mono- und Diamid,

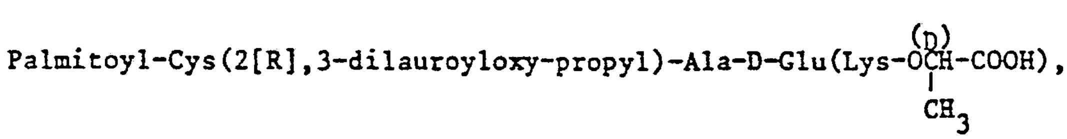

dessen Mono- und Dimethylester und dessen Mono- und Diamid,
Palmitoyl-Cys(2[R],3-dilauroyloxy-propyl)-Ala-D-Glu(Tly),

(Tly = 4-Thia-Lysin), dessen Mono- und Dimethylester und dessen Mono- und Diamid, und entsprechenden Lipopeptiden, in denen anstelle von Palmitoyl am Stickstoff des Cysteinrestes Lauroyl, Caprinoyl, Capryloyl oder Myristoyl vorhanden sind, und diesen und den oben angeführten Lipopeptiden entsprechenden Verbindungen, in denen im Diacyloxypropyl-Rest anstelle von Lauroyl-Resten die Reste der Palmitin-, der Capryl-, der Caprin- und der Myristinsäure vorhanden sind, sowie den all diesen Lipopeptiden entsprechenden Verbindungen, in denen die Konfiguration am chiralen Atom des Diacyloxypropylrestes S statt R ist, und entsprechenden Diastereomeren-Gemischen von R und S-Verbindungen, sowie gegebenenfalls deren unsubstituierten oder substituierten Amiden, oder ein Salz einer solchen Verbindung mit mindestens einer salzbildendenden Gruppe herstellt.

**21.** Verfahren nach Anspruch 2, dadurch gekennzeichnet, dass man die Ausgangsstoffe so wählt, dass man einen Ester von aliphatischen Alkoholen mit 1-7 C-Atomen oder einen Ester von $C_{1-7}$-Alkanoyloxymethylalkoholen, $C_{1-7}$-Alkanoyloxy-äthylalkoholen, ($C_{3-8}$-Cycloalkyl)-carbonyloxymethylalkoholen, ($C_{3-8}$-Cycloalkyl)-carbonyloxy-äthylalkoholen, Propylenglykol, Glyzerin oder eines $C_{1-7}$-Alkoxy-, $C_{1-7}$-Alkylamino-, Di-($C_{1-7}$-Alkyl)-amino- oder Halogenphenols eines der in Anspruch 20 genannten Lipopeptide herstellt.

**22.** Verfahren nach Anspruch 2, dadurch gekennzeichnet, dass man die Ausgangsstoffe so wählt, dass man ein unsubstituiertes Amid oder ein Amid von $C_{1-7}$-Alkylaminen, Pyrrolidin, Piperidin oder Piperazin eines der in Anspruch 20 genannten Lipopeptide herstellt.

**23.** Verfahren nach Anspruch 2, dadurch gekennzeichnet, dass man die Ausgangsstoffe so wählt, dass man eine Verbindung der Formel I, ausgewählt aus der Gruppe, bestehend aus
Lauroyl-Cys(2[R,S],3-didecanoyloxy-propyl)-Ala-D-Glu-$NH_2$,
Decanoyl-Cys(2(R,S],3-dilauroyloxy-propyl_-Ala-D-Glu-$NH_2$,
Myristoyl-Cys(2[R,S],3-dilauroyloxy-propyl)-Ala-D-Glu-$NH_2$,
Palmitoyl-Cys(2[R,S],3-didecanoyloxy-propyl)-Ala-D-Glu-$NH_2$,
Palmitoyl-Cys(2[R,S],3-didecanoyloxy-propyl)-Abu-D-Glu($OCH_3$)-$OCH_3$,
und aus deren Estern und Amiden gemäss einem der Ansprüche 21 und 23 , herstellt.

**24.** Verfahren nach Anspruch 1, dadurch gekennzeichnet, dass man die Ausgangsstoffe so wählt, dass man Palmitoyl-Cys(2[R],3-dilauroyloxy-propyl)-Ala-D-Glu(Gly-taurin)-$NH_2$ oder ein Salz davon herstellt.

**25.** Verfahren nach Anspruch 1, dadurch gekennzeichnet, dass man die Ausgansstoffe so wählt, dass man Palmitoyl-Cys(2[R],3-dilauroyloxy-propyl)-Ala-D-Glu-$NH_2$ oder ein Salz davon herstellt.

**26.** Verfahren nach Anspruch 2, dadurch gekennzeichnet, dass man die Ausgangsstoffe so wählt, dass man ein Ammoniumsalz, Alkali- oder Erdalkalisalz von einem der in den Ansprüchen 1-25 beanspruchten sauren Lipopeptide oder ein pharmazeutisch anwendbares, nichttoxisches Säureadditionssalz der in den genannten Ansprüchen beanspruchten basischen Lipopeptide herstellt.

**Claims**

**Claims for the following Contracting States: BE, CH, DE, FR, GB, IT, LU, NL, SE**

**1.** A lipopeptide of the formula I

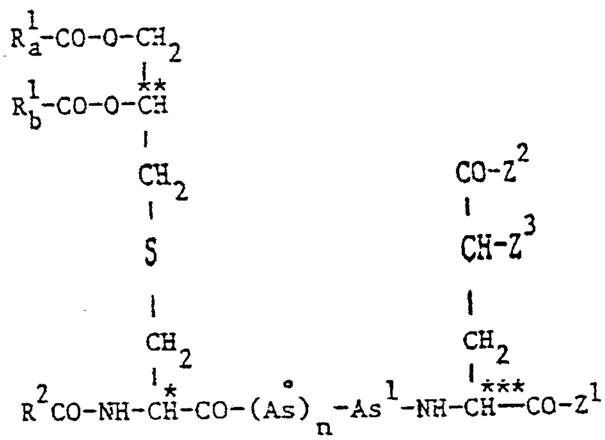

(I)

* = R

** = R or S

*** = R

in which each of $R_a^1$ and $R_b^1$ , independently of the other, represents an aliphatic or cycloaliphatic-aliphatic hydrocarbon radical having from 7 to 21 carbon atoms that is optionally substituted by oxygen functions, or one of the radicals $R_a^1$ -CO and $R_b^1$ -CO represents hydrogen and the other of the radicals $R_a^1$ -CO and $R_b^1$ -CO represents an acyl radical, wherein $R_a^1$ and $R_b^1$ have the meanings given above, $R^2$ represents an aliphatic or cycloaliphatic-aliphatic hydrocarbon radical having from 1 to 21 carbon atoms that is optionally substituted by oxygen functions, n = 0 or 1, $As^{\circ}$ represents a radical of the formula -O-Kw-CO- or -NH-Kw-CO- wherein Kw represents an aliphatic hydrocarbon radical having a maximum of 12 carbon atoms, $As^1$ represents a D- or L-α-amino acid, each of $Z^1$ and $Z^2$, independently of the other, represents hydroxy or the N-terminal radical of a D- or L-α-aminocarboxylic acid, of an amino-lower alkanesulphonic acid or of a peptide having a maximum of 6 amino acids from the group consisting of D- or L-α-aminocarboxylic acids and amino-lower alkanesulphonic acids, and $Z^3$ represents hydrogen or -CO-$Z^4$ wherein $Z^4$ represents hydroxy or the N-terminal radical of a D- or L-α-amino acid, of an amino-lower alkanesulphonic acid or of a peptide having a maximum of 6 amino acids from the group consisting of D-or L-α-aminocarboxylic acids and amino-lower alkanesulphonic acids, or an amide or ester of such a compound that contains carboxy group(s), wherein the centres of asymmetry designated by *, ** and *** have the absolute configurations indicated, and the configuration at an asymmetric carbon atom carrying the group $Z^3$ may be R or S, or a corresponding diastereoisomeric mixture, or a salt of such a compound having at least one salt-forming group, or optionally a complex salt of such a compound.

2. A compound according to claim 1, in which $R_a^1$ and $R_b^1$ have the same meaning and each represents an aliphatic or cycloaliphatic-aliphatic hydrocarbon radical having from 7 to 21 carbon atoms that is optionally substituted by oxygen functions, each of $Z^1$ and $Z^2$, independently of the other, represents hydroxy or the N-terminal radical of a D- or L-α-amino acid or of a peptide having a maximum of 6 D- or L-α-amino acids, and $Z^3$ represents hydrogen or -CO-$Z^4$ wherein $Z^4$ represents hydroxy or the N-terminal radical of a D- or L-α-amino acid or of a peptide having a maximum of 6 D- or L-α-amino acids, or a salt of such a compound having at least one salt-forming group.

3. A compound according to claim 2, in which α-amino acids are the naturally occurring L-α-amino acids or the antipodes thereof of the D-series, or a salt of such a compound having at least one salt-forming group.

4. A compound according to any one of claims 1 to 3, in which an amino acid $As^1$ is selected from the group consisting of Gly, Ala, Ser, Abu (α-aminobutyric acid), Val, αMeAla (α-methyl-alanine) and Leu, an amino acid in the radical $Z^1$ is selected from the group consisting of Lys, Orn (ornithine), Dpm (α,α'-diamino-pimelic acid), Gly, Ala, D-Asn and D-Ala, and an amino acid $Z^2$ and/or $Z^4$ is selected from the group consisting of Lys, Orn, Dpm, Lan (lanthionine), Gly or Ala, a peptide radical $Z^1$, $Z^2$ or $Z^4$ consisting of 2 amino acids selected in such a manner, or a salt of such a compound having at least one salt-forming group.

5. A compound according to any one of claims 1 to 4, in which each of $Z^1$ and $Z^2$ in formula (I), independently of the other, represents hydroxy or the radical of an amino acid, and $Z^3$ represents hydrogen or -CO-$Z^4$ wherein $Z^4$ represents hydroxy or the radical of an amino acid, or a salt of such a

compound having at least one salt-forming group.

6. A compound according to claim 5, in which $Z^1$ in formula (I) represents an amino acid radical that is selected from the group consisting of -Lys, -Orn (ornithine radical), -Dpm (α,α'-diamino-pimelic acid radical), -Gly, -Ala, -D-Ala and -D-Asn, and $Z^2$ and/or $Z^4$ represent an amino acid radical that is selected from the group consisting of -Lys, -Orn, -Dpm, -Lan (lanthionine radical), -Gly and -Ala, or a salt of such a compound having at least one salt-forming group.

7. A compound according to any one of claims 1 to 6, in which the acyl radicals $R_a^1$ -CO-, $R_b^1$ -CO- and $R^2$-CO- are derived from saturated or unsaturated, optionally oxygenated fatty acids having from 8 to 16 carbon atoms or (in the case of $R^2$-CO-) from 2 to 16 carbon atoms, or a salt of such a compound having at least one salt-forming group.

8. A compound according to any one of claims 1 to 6, in which the acyl radicals $R_a^1$ -CO-, $R_b^1$ -CO- and $R^2$-CO- are derived from caprylic, pelargonic, capric, undecylic, lauric, myristic, palmitic, margaric, stearic, arachidic, behenic, oleic, elaidic, linoleic, α- or β-eleostearic, stearolic or α-linolenic acid or, in the case of $R^2$-CO-, also from acetic, propionic, butyric, oenanthic or caproic acid, or a salt of such a compound having at least one salt-forming group.

9. A compound according to any one of claims 1 to 6, in which the acyl radicals $R_a^1$ -CO-, $R_b^1$ -CO- and $R^2$-CO- are derived from saturated or unsaturated cycloaliphatic-aliphatic carboxylic acids having from 8 to 16 carbon atoms or, in the case of $R^2$-CO-, from 2 to 16 carbon atoms in the aliphatic moiety and, at any position in the aliphatic carbon chain, are substituted by a cycloalkyl or cycloalkenyl ring having from 3 to 8 carbon atoms or interrupted by a cycloalkylene or cycloalkenylene radical having from 3 to 8 carbon atoms, or a salt of such a compound having at least one salt-forming group.

10. A compound according to claim 9, in which the acyl radicals are derived from dihydrosterculic, malvalic, hydnocarpic or chaulmoogric acid, or a salt of such a compound having at least one salt-forming group.

11. A compound according to any one of claims 1 to 6, in which $R_a^1$ -CO- and $R_b^1$ -CO- are different from $R^2$-CO-, and $R_a^1$ -CO- and $R_b^1$ -CO- represent lauroyl, myristoyl, palmitoyl or stearoyl and $R^2$-CO- represents stearoyl, myristoyl, lauroyl, caprinoyl or capryloyl, or a salt of such a compound having at least one salt-forming group.

12. An ester or amide of a compound according to any one of claims 6 to 11.

13. An ester according to claim 2 or 12, in which the ester group(s) are derived from optionally substituted, aliphatic, araliphatic, aromatic or heterocyclic alcohols, or a salt of such a compound having at least one salt-forming group.

14. An ester according to claim 13, in which aliphatic ester group(s) are derived from lower aliphatic alcohols having from 1 to 7 carbon atoms, araliphatic ester component(s) are derived from monocyclic-lower aliphatic alcohols having from 1 to 7 carbon atoms in the aliphatic moiety, aromatic ester component(s) are derived from $C_{1-7}$-alkoxy-, $C_{1-7}$-alkylamino- or di-($C_{1-7}$)-alkylamino-or halo-phenols, and heterocyclic ester group(s) are derived from tetrahydrofuranol or tetrahydropyranol, or a salt of such a compound having at least one salt-forming group.

15. An ester according to claim 13 or 14, in which substituent(s) in the ester component are free, esterified or etherified hydroxy groups, esterified groups being derived from aliphatic carboxylic acids having from 1 to 7 carbon atoms, and etherified groups being derived from aliphatic alcohols having from 1 to 7 carbon atoms, or a salt of such a compound having at least one salt-forming group.

16. An ester according to any one of claims 12 to 15, in which the α-carboxy group of the amino acid concerned is esterified, or a salt of such a compound having at least one salt-forming group.

17. A mono-, di-, tri-, tetra-, penta-, hexa- or nona-ester according to any one of claims 12 to 16, or a salt of such a compound having at least one salt-forming group.

**18.** An amide according to claim 2 or 12, in which the amide group is unsubstituted or is derived from a cyclic or acyclic, primary or secondary amine, the hydrocarbon radical or radicals substituting the amine nitrogen atom being, in the case of the acyclic amines, alkyl groups having from 1 to 7 carbon atoms and, in the case of the cyclic amines, alkylene groups having from 2 to 6 carbon atoms, or a salt of such a compound having at least one salt-forming group.

**19.** A mono-, di-, tri-, tetra-, penta-, hexa- or nona-amide according to any one of claims 11 to 15, or a salt of such a compound having at least one salt-forming group.

**20.** A compound according to any one of claims 1 to 19, in which in formula (I) of claim 1 the radical Kw in the radical As° is an unsubstituted alkylene or alkylidene radical having from 2 to 6 carbon atoms, or a salt of such a compound having at least one salt-forming group.

**21.** A compound according to claim 20, in which Kw is methylene, di-, tri- or tetra-methylene, ethylidene, 2-propylidene, 2,2-dimethylethylidene, 2-butylidene, 3,3-dimethylpropylidene, 2-methylethylidene, 2-ethylethylidene or pentylidene, or a salt of such a compound having at least one salt-forming group.

**22.** A compound according to claim 20, in which As° represents the radical of D- or L-lactic acid, of glycine, alanine, α-aminobutyric acid, valine, norvaline, leucine, isoleucine, norleucine or of corresponding amino acids of the D-series, or a salt of such a compound having at least one salt-forming group.

**23.** A compound according to any one of claims 1 to 19, in which in formula (I) of claim 1, in the peptide sequence n = 0, or a salt of such a compound having at least one salt-forming group.

**24.** A compound according to claim 23, in which the sequence of the formula

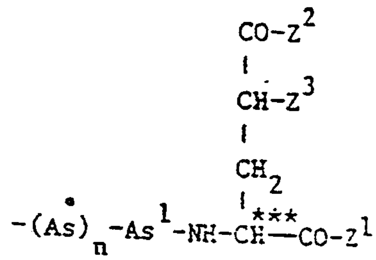

in which n represents 0, in formula (I) of claim 1 is selected from the group consisting of -Ala-D-Glu, -Ala-D-Glu-$NH_2$,-Ala-D-Glu($NH_2$), -Ala-D-Glu-D-Ala-$NH_2$, -Ala-D-Glu($NH_2$)-$NH_2$, -Ala-D-Glu(Ala), -Ala-D-Glu($NH_2$)-D-Ala-$NH_2$,-Ala-D-Glu(Ala)-$NH_2$ and -Ala-D-Glu (Gly-taurine)-$NH_2$, and corresponding sequences in which -Gly-, -Ser-, -Abu-, -Leu-, -αMeAla- (α-methyl-alanine radical) or -Val- stand in place of the first alanine radical, or a salt of such a compound having at least one salt-forming group.

**25.** A compound according to any one of claims 1 to 21, in which the sequence of the formula

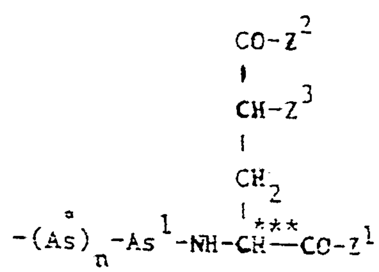

in formula (I) of claim 1 is selected from the group consisting of -As°-Ala-D-Glu, -As°-Ala-D-Glu-$NH_2$, -As°-Ala-D-Glu($NH_2$), As°-Ala-D-Glu-D-Ala-$NH_2$, As°-Ala-D-Glu($NH_2$)-$NH_2$, As°-Ala-D-Glu(Ala), As°-Ala-D-Glu($NH_2$)-D-Ala-$NH_2$ and As°-Ala-D-Glu(Ala)-$NH_2$, wherein As° represents the radical of D- or L-alanine, D- or L-lactic acid, glycolic acid or glycine, or a salt of such a compound having at least one

salt-forming group.

**26.** A compound of formula (I) according to claims 1, 2, 7 and 20 to 22, that has the R configuration at the ** centre of asymmetry in formula (I) of claim 1, in which the radicals $R_a^1$ -CO-, $R_b^1$ -CO- and $R^2$-CO- have from 8 to 16 carbon atoms and in which the sequence of the formula

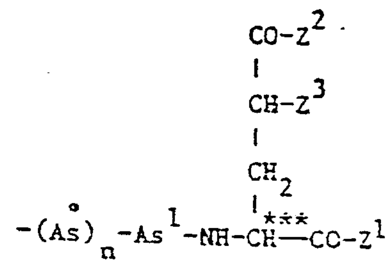

in which n represents 0 or 1, in formula (I) of claim 1 is selected from the group consisting of -Ala-D-Glu, -Ala-D-Glu-$NH_2$, -Ala-D-Glu($NH_2$), -Ala-D-Glu-D-Ala-$NH_2$, -Ala-D-Glu($NH_2$)-$NH_2$, -Ala-D-Glu(Ala), -Ala-D-Glu($NH_2$)-D-Ala-$NH_2$, -Ala-D-Glu(Ala)-$NH_2$, -As° -Ala-D-Glu, -As° -Ala-D-Glu-$NH_2$, -As° -Ala-D-Glu-($NH_2$), As° -Ala-D-Glu-D-Ala-$NH_2$, As° -Ala-D-Glu($NH_2$)-$NH_2$, As° -Ala-D-Glu(Ala), As° -Ala-D-Glu($NH_2$)-D-Ala-$NH_2$ and As° -Ala-D-Glu(Ala)-$NH_2$, or a salt of such a compound having at least one salt-forming group.

**27.** A compound according to claim 26, in which the acyl radicals $R_a^1$ -CO- and $R_b^1$ -CO- are different from $R^2$-CO- and represent the radical of caprylic, capric, lauric, myristic, palmitic, stearic or oleic acid, or a salt of such a compound having at least one salt-forming group.

**28.** A lipopeptide according to claim 2, selected from the group consisting of
palmitoyl-Cys(2[R],3-dilauroyloxy-propyl)-Ala-D-Glu-$NH_2$,
palmitoyl-Cys(2[R],3-dipalmitoyloxy-propyl)-Ala-D-Glu(Ala)-$NH_2$,
palmitoyl-Cys(2[R],3-dilauroyloxy-propyl)-Ala-D-Glu($NH_2$),
palmitoyl-Cys(2[R],3-dilauroyloxy-propyl)-Abu-D-Glu-$NH_2$ (Abu = $\alpha$-aminobutyric acid),
palmitoyl-Cys(2[R],3-dilauroyloxy-propyl)-Ala-D-Glu($NH_2$)-OnBu (nBu = n-butyl),
palmitoyl-Cys(2[R],3-dilauroyloxy-propyl)-Ala-D-Glu(OnBu)-$NH_2$,
palmitoyl-Cys(2[R],3-dilauroyloxy-propyl)-D-Ala-Ala-D-Glu-$NH_2$,

(D)
palmitoyl-Cys(2[R],3-dilauroyloxy-propyl)-O-CH-CO-Ala-
$CH_3$
D-Glu-$NH_2$,

palmitoyl-Cys(2[R],3-dilauroyloxy-propyl)-O-$CH_2$CO-Ala-D-Glu-$NH_2$
palmitoyl-Cys(2[R],3-dilauroyloxy-propyl)-Ala-D-Glu($NH_2$)-O-$CH_2$-O-CO-C($CH_3$)$_3$,
palmitoyl-Cys(2[R],3-dilauroyloxy-propyl)-Ser-D-Glu($OCH_3$)-$OCH_3$,
palmitoyl-Cys(2[R],3-dilauroyloxy-propyl)-Ala-D-Gla-$NH_2$, (Gla = $\gamma$-carboxyglutamic acid),
palmitoyl-Cys(2[R],3-dilauroyloxy-propyl)-Abu-D-Glu,
palmitoyl-Cys(2[R],3-dilauroyloxy-propyl)-Val-D-Glu-$NH_2$,
palmitoyl-Cys(2[R],3-dilauroyloxy-propyl)-$\alpha$MeAla-D-Glu-$NH_2$ ($\alpha$MeAla = $\alpha$-methyl-alanine),
palmitoyl-Cys(2[R],3-dilauroyloxy-propyl)-Ala-D-Glu(Lys-$OCH_3$)-$NH_2$,
palmitoyl-Cys(2[R],3-dilauroyloxy-propyl)-Ala-D-Glu(Lys-Lys-$OCH_3$)-$NH_2$,
palmitoyl-Cys(2[R],3-dilauroyloxy-propyl)-Ala-D-Glu-(Arg), the mono- and di-methyl ester and mono- and di-amide thereof,

palmitoyl-Cys(2[R],3-dilauroyloxy-propyl)-Ala-D-Glu(Lys-

(D)
OCH-COOH),
 |
 $CH_3$

the mono- and di-methyl ester and mono- and di-amide thereof,
palmitoyl-Cys(2[R],3-dilauroyloxy-propyl)-Ala-D-Glu(Tly), (Tly = 4-thia-lysine), the mono- and di-methyl ester and mono- and di-amide thereof,
and corresponding lipopeptides in which, instead of palmitoyl there is present at the nitrogen of the cysteine radical lauroyl, caprinoyl, capryloyl or myristoyl, and compounds corresponding to these and to the above-listed lipopeptides in which there are present in the diacyloxypropyl radical instead of lauroyl radicals the radicals of palmitic, caprylic, capric and myristic acid, and the compounds corresponding to all of these lipopeptides in which the configuration at the chiral atom of the diacyloxypropyl radical is S instead of R, and corresponding diastereoisomeric mixtures of R and S compounds, as well as, optionally, the unsubstituted or substituted amides thereof, or a salt of such a compound having at least one salt-forming group.

**29.** A compound according to claim 2, namely an ester of an aliphatic alcohol having from 1 to 7 carbon atoms or an ester of a $C_{1-7}$-alkanoyloxymethyl alcohol, $C_{1-7}$-alkanoyloxyethyl alcohol, ($C_{3-8}$-cycloalkyl)-carbonyloxymethyl alcohol, ($C_{3-8}$-cycloalkyl)-carbonyloxyethyl alcohol, propylene glycol, glycerin, or of a $C_{1-7}$-alkoxy-, $C_{1-7}$-alkylamino-, di-($C_{1-7}$-alkyl)-amino- or halo-phenol, of one of the lipopeptides mentioned in claim 28.

**30.** A compound according to claim 2, namely an unsubstituted amide or an amide of a $C_{1-7}$-alkylamine, pyrrolidine, piperidine or piperazine of one of the lipopeptides mentioned in claim 28.

**31.** A lipopeptide according to claim 2, selected from the group consisting of
lauroyl-Cys(2[R,S],3-didecanoyloxy-propyl)-Ala-D-Glu-$NH_2$,
decanoyl-Cys(2[R,S],3-dilauroyloxy-propyl)-Ala-D-Glu-$NH_2$,
myristoyl-Cys(2[R,S],3-dilauroyloxy-propyl)-Ala-D-Glu-$NH_2$,
palmitoyl-Cys(2[R,S],3-didecanoyloxy-propyl)-Ala-D-Glu-$NH_2$,
palmitoyl-Cys(2[R,S],3-didecanoyloxy-propyl)-Abu-D-Glu($OCH_3$)-$OCH_3$,
and of the esters and amides thereof according to either claim 29 or claim 30.

**32.** Palmitoyl-Cys(2[R],3-dilauroyloxy-propyl)-Ala-D-Glu(Gly-taurine)-$NH_2$ or a salt thereof according to claim 1.

**33.** Palmitoyl-Cys(2[R],3-dilauroyloxy-propyl)-Ala-D-Glu-$NH_2$ or a salt thereof according to claim 1.

**34.** A compound according to claim 1 or 2, namely an ammonium, alkali metal or alkaline earth metal salt of one of the acidic lipopeptides claimed in claims 1 to 33, or a pharmaceutically acceptable nontoxic acid addition salt of the basic lipopeptides claimed in the said claims.

**35.** A compound of the formula (I) or a pharmaceutically acceptable salt thereof according to any one of claims 1 to 34 for use in a method for increasing the bodily powers of defence of the human or animal body.

**36.** A pharmaceutical preparation comprising a compound of the formula (I) or a pharmaceutically acceptable salt thereof according to any one of claims 1 to 34 together with a pharmaceutical carrier.

**37.** The use of a lipopeptide of the formula (I) or of a pharmaceutically acceptable salt thereof according to any one of claims 1 to 34 for the manufacture of pharmaceutical preparations intended for use in obtaining an immunity-stimulating effect or as prophylactic or therapeutic agents against infectious diseases in humans and animals.

**38.** A combination preparation comprising one or more of the lipopeptides of the formula (I) or a

pharmaceutically acceptable salt thereof according to any one of claims 1 to 34 together with one or more antibiotics.

**39.** A process for the manufacture of a compound of the formula I

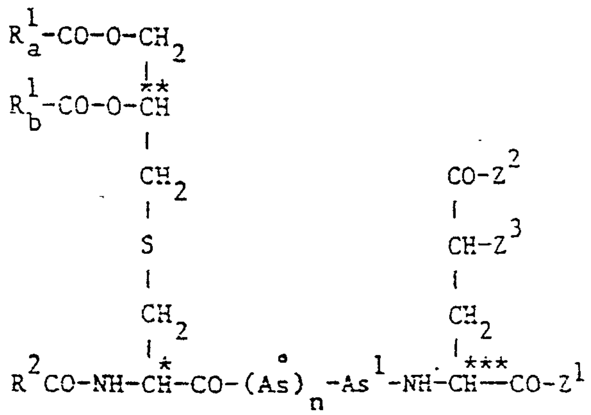

(I)

* = R

** = R or S

*** = R

in which each of $R_a^1$ and $R_b^1$ , independently of the other, represents an aliphatic or cycloaliphatic-aliphatic hydrocarbon radical having from 7 to 21 carbon atoms that is optionally substituted by oxygen functions, or one of the radicals $R_a^1$ -CO and $R_b^1$ -CO represents hydrogen and the other of the radicals $R_a^1$ -CO and $R_b^1$ -CO represents an acyl radical, wherein $R_a^1$ and $R_b^1$ have the meanings given above, $R^2$ represents an aliphatic or cycloaliphatic-aliphatic hydrocarbon radical having from 1 to 21 carbon atoms that is optionally substituted by oxygen functions, n = 0 or 1, As° represents a radical of the formula -O-Kw-CO- or -NH-Kw-CO- wherein Kw represents an aliphatic hydrocarbon radical having a maximum of 12 carbon atoms, $As^1$ represents a D- or L-$\alpha$-amino acid, each of $Z^1$ and $Z^2$, independently of the other, represents hydroxy or the N-terminal radical of a D- or L-$\alpha$-aminocarboxylic acid, of an amino-lower alkanesulphonic acid or of a peptide having a maximum of 6 amino acids from the group consisting of D- or L-$\alpha$-aminocarboxylic acids and amino-lower alkanesulphonic acids, and $Z^3$ represents hydrogen or -CO-$Z^4$ wherein $Z^4$ represents hydroxy or the N-terminal radical of a D- or L-$\alpha$-amino acid, of an amino-lower alkanesulphonic acid or of a peptide having a maximum of 6 amino acids from the group consisting of D-or L-$\alpha$-aminocarboxylic acids and amino-lower alkanesulphonic acids, or of an amide or ester of such a compound that contains at least one carboxy group, wherein the centres of asymmetry designated by *, ** and *** have the absolute configurations indicated, and the configuration at an asymmetric carbon atom carrying the group $Z^3$ may be R or S, or of a corresponding diastereoisomeric mixture, or of a salt of such a compound having at least one salt-forming group, or optionally of a complex salt of such a compound, characterised in that

a) in a compound corresponding to the formula (I) or in a salt thereof, in which the substituents have the meanings given above with the proviso that the peptide chain

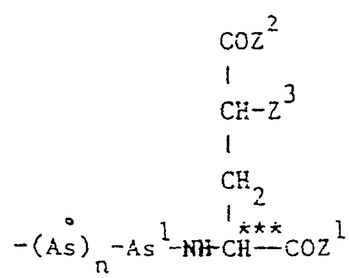

(II)

contains at least one protected functional group, the protecting group(s) is (are) removed, or

b) a compound of the formula I in which at least one of the radicals $R_a^1$ -CO-, $R_b^1$ -CO- and $R^2$-CO- represents hydrogen and the remaining substituents have the meanings given above with the proviso that any free functional groups present in this starting material, with the exception of the hydroxy and/or amino group(s) participating in the reaction, are, if necessary, in protected form, or a salt

thereof, is acylated with an acid $R_a^1$ -COOH, $R_b^1$ -COOH or $R^2$-COOH respectively or a reactive carboxylic acid derivative thereof, and any protecting groups present are removed, or

c) an amide bond of a compound of the formula I is produced by reacting a corresponding fragment of a compound of the formula I having a free carboxy group, or of a reactive acid derivative thereof, with a complementary fragment having a free amino group or with a reactive derivative thereof having an activated amino group, any free functional groups present in the reactants, with the exception of the groups participating in the reaction, if necessary being in protected form, and any protecting groups present are removed, or

d) in a compound of the formula (I) in which at least one free carboxy group is present, the free carboxy group(s) is (are) esterified or amidated and/or, in a compound of the formula (I) in which at least one ester group is present, the ester group(s) is (are) hydrolysed, or

e) a compound of the formula III

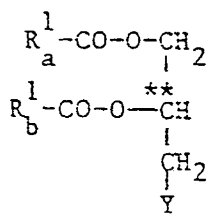

** = R or S

(III)

in which $R_a^1$ and $R_b^1$ have the meanings given above and Y represents a nucleofugal group is reacted with a compound of the formula IV

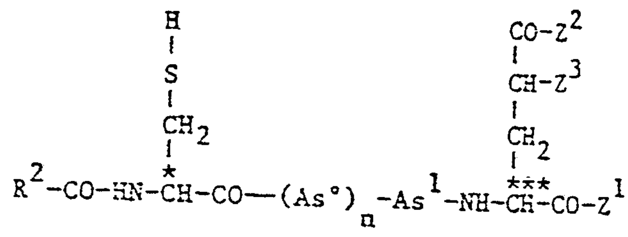

* = R

*** = R

(IV)

in which the substituents have the meanings given above, wherein free functional groups, with the exception of the mercapto group participating in the reaction, are if necessary protected by readily removable protecting groups, or with a reactive derivative of a compound of the formula IV, and any protecting groups present are removed, or

f) a compound of the formula V

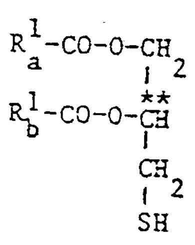

** = R or S

(V)

in which $R_a^1$ and $R_b^1$ have the meanings given above, or a reactive derivative of this compound, is reacted with a compound of the formula VI

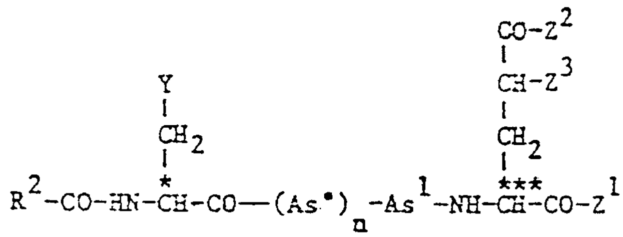

* = R
*** = R
(VI)

in which Y represents a nucleofugal group and the remaining substituents have the meanings given above, wherein free functional groups are, if necessary, in protected form, and any protecting groups present are removed, and, if desired, after carrying out one of the process variants a - f), a resulting compound of the formula I having at least one salt-forming group is converted into a salt or complex salt, or a resulting salt or complex salt is converted into the free compound and, if desired, resulting mixtures of isomers are resolved.

**Claims for the following Contracting State: AT**

**1.** A process for the manufacture of a compound of the formula I

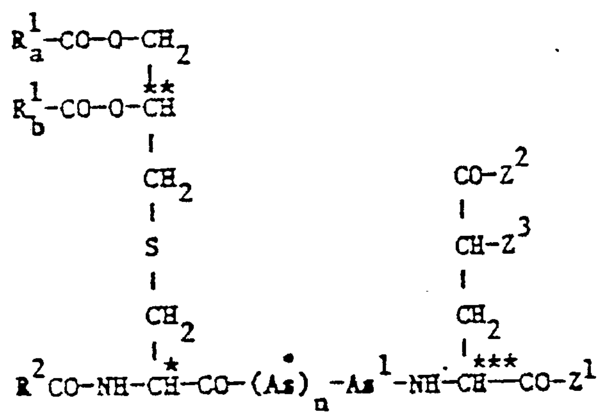

(I)
* = R
** = R or S
*** = R

in which each of $R_a^1$ and $R_b^1$ , independently of the other, represents an aliphatic or cycloaliphatic-aliphatic hydrocarbon radical having from 7 to 21 carbon atoms that is optionally substituted by oxygen functions, or one of the radicals $R_a^1$ -CO and $R_b^1$ -CO represents hydrogen and the other of the radicals $R_a^1$ -CO and $R_b^1$ -CO represents an acyl radical, wherein $R_a^1$ and $R_b^1$ have the meanings given above, $R^2$ represents an aliphatic or cycloaliphatic-aliphatic hydrocarbon radical having from 1 to 21 carbon atoms that is optionally substituted by oxygen functions, n = 0 or 1, $As^{\circ}$ represents a radical of the formula -O-Kw-CO- or -NH-Kw-CO- wherein Kw represents an aliphatic hydrocarbon radical having a maximum of 12 carbon atoms, $As^1$ represents a D- or L-α-amino acid, each of $Z^1$ and $Z^2$, independently of the other, represents hydroxy or the N-terminal radical of a D- or L-α-aminocarboxylic acid, of an amino-lower alkanesulphonic acid or of a peptide having a maximum of 6 amino acids from the group consisting of D- or L-α-aminocarboxylic acids and amino-lower alkanesulphonic acids, and $Z^3$ represents hydrogen or -CO-$Z^4$ wherein $Z^4$ represents hydroxy or the N-terminal radical of a D- or L-α-amino acid, of an amino-lower alkanesulphonic acid or of a peptide having a maximum of 6 amino acids from the group consisting of D-or L-α-aminocarboxylic acids and amino-lower alkanesulphonic acids, or of an amide or ester of such a compound that contains at least one carboxy group, wherein the centres of asymmetry designated by *, ** and *** have the absolute configurations indicated, and the configuration at an asymmetric carbon atom carrying the group $Z^3$ may be R or S, or of a corresponding diastereoisomeric mixture, or of a salt of such a compound having at least one salt-forming group, or optionally of a complex salt of such a compound, characterised in that

a) in a compound corresponding to the formula (I) or in a salt thereof, in which the substituents have the meanings given above with the proviso that the peptide chain

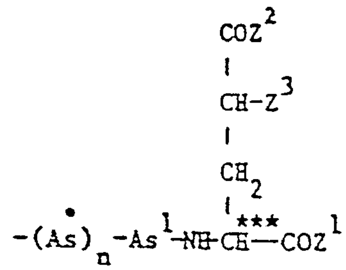

(II)

contains at least one protected functional group, the protecting group(s) is (are) removed, or

b) a compound of the formula I in which at least one of the radicals $R_a^1$ -CO-, $R_b^1$ -CO- and $R^2$-CO- represents hydrogen and the remaining substituents have the meanings given above with the proviso that any free functional groups present in this starting material, with the exception of the hydroxy and/or amino group(s) participating in the reaction, are, if necessary, in protected form, or a salt thereof, is acylated with an acid $R_a^1$ -COOH, $R_b^1$ -COOH or $R^2$-COOH respectively or a reactive carboxylic acid derivative thereof, and any protecting groups present are removed, or

c) an amide bond of a compound of the formula I is produced by reacting a corresponding fragment of a compound of the formula I having a free carboxy group, or of a reactive acid derivative thereof, with a complementary fragment having a free amino group or with a reactive derivative thereof having an activated amino group, any free functional groups present in the reactants, with the exception of the groups participating in the reaction, if necessary being in protected form, and any protecting groups present are removed, or

d) in a compound of the formula (I) in which at least one free carboxy group is present, the free carboxy group(s) is (are) esterified or amidated and/or, in a compound of the formula (I) in which at least one ester group is present, the ester group(s) is (are) hydrolysed, or

e) a compound of the formula III

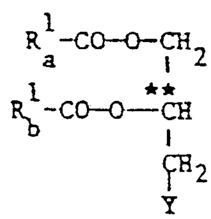

** = R or S

(III)

in which $R_a^1$ and $R_b^1$ have the meanings given above and Y represents a nucleofugal group is reacted with a compound of the formula IV

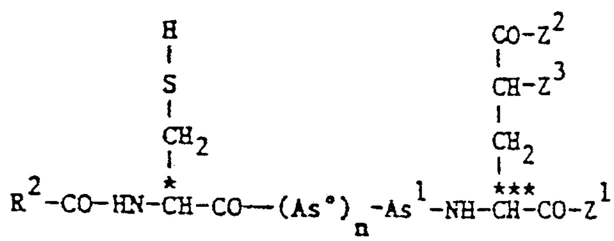

* = R

*** = R

(IV)

in which the substituents have the meanings given above, wherein free functional groups, with the exception of the mercapto group participating in the reaction, are if necessary protected by readily removable protecting groups, or with a reactive derivative of a compound of the formula IV, and any protecting groups present are removed, or

f) a compound of the formula V

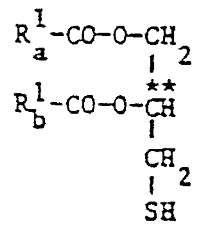

** = R or S

(V)

in which $R_a^1$ and $R_b^1$ have the meanings given above, or a reactive derivative of this compound, is reacted with a compound of the formula VI

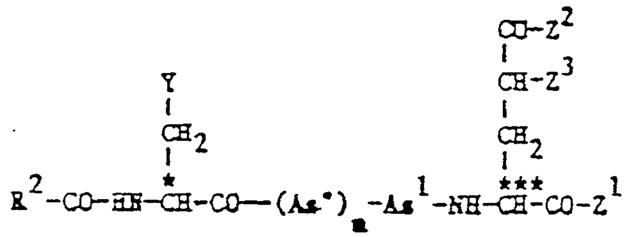

* = R

*** = R

(VI)

in which Y represents a nucleofugal group and the remaining substituents have the meanings given above, wherein free functional groups are, if necessary, in protected form, and any protecting groups present are removed, and, if desired, after carrying out one of the process variants a - f), a resulting compound of the formula I having at least one salt-forming group is converted into a salt or complex salt, or a resulting salt or complex salt is converted into the free compound and, if desired, resulting mixtures of isomers are resolved.

2. A process according to claim 1, characterised in that the starting materials are chosen so as to manufacture a compound of the formula I in which $R_a^1$ and $R_b^1$ have the same meaning and each represents an aliphatic or cycloaliphatic-aliphatic hydrocarbon radical having from 7 to 21 carbon atoms that is optionally substituted by oxygen functions, each of $Z^1$ and $Z^2$, independently of the other, represents hydroxy or the N-terminal radical of a D- or L-α-amino acid or of a peptide having a maximum of 6 D-or L-α-amino acids, and $Z^3$ represents hydrogen or -CO-$Z^4$ wherein $Z^4$ represents hydroxy or the N-terminal radical of a D- or L-α-amino acid or of a peptide having a maximum of 6 D- or L-α-amino acids, or a salt of such a compound having at least one salt-forming group.

3. A process according to claim 2, characterised in that the starting materials are chosen so as to manufacture a compound of the formula I in which α-amino acids are the naturally occurring L-α-amino acids or the antipodes thereof of the D-series, or a salt of such a compound having at least one salt-forming group.

4. A process according to any one of claims 1 to 3, characterised in that the starting materials are chosen so as to manufacture a compound of the formula (I) in which an amino acid $As^1$ is selected from the group consisting of Gly, Ala, Ser, Abu (α-aminobutyric acid), Val, αMeAla (α-methyl-alanine) and Leu, an amino acid in the radical $Z^1$ is selected from the group consisting of Lys, Orn (ornithine), Dpm (α,α'-diamino-pimelic acid), Gly, Ala, D-Asn and D-Ala, and an amino acid $Z^2$ and/or $Z^4$ is selected from the group consisting of Lys, Orn, Dpm, Lan (lanthionine), Gly or Ala, a peptide radical $Z^1$, $Z^2$ or $Z^4$ consisting of 2 amino acids selected in such a manner, or a salt of such a compound having at least one salt-forming group.

5. A process according to any one of claims 1 to 4, characterised in that the starting materials are chosen so as to manufacture a compound of the formula I in which each of $Z^1$ and $Z^2$ in formula (I), independently of the other, represents hydroxy or the radical of an amino acid, and $Z^3$ represents hydrogen or -CO-$Z^4$ wherein $Z^4$ represents hydroxy or the radical of an amino acid, or a salt of such a compound having at least one salt-forming group.

**6.** A process according to claim 5, characterised in that the starting materials are chosen so as to manufacture a compound of the formula I in which $Z^1$ in formula (I) represents an amino acid radical that is selected from the group consisting of -Lys, -Orn (ornithine radical), -Dpm ($\alpha,\alpha'$-diamino-pimelic acid radical), -Gly, -Ala, -D-Ala and -D-Asn, and $Z^2$ and/or $Z^4$ represent an amino acid radical that is selected from the group consisting of -Lys, -Orn, -Dpm, -Lan (lanthionine radical), -Gly and -Ala, or a salt of such a compound having at least one salt-forming group.

**7.** A process according to any one of claims 1 to 6, characterised in that the starting materials are chosen so as to manufacture a compound of the formula I in which the acyl radicals $R^1_a$ -CO-, $R^1_b$ -CO- and $R^2$-CO- are derived from saturated or unsaturated, optionally oxygenated fatty acids having from 8 to 16 carbon atoms or (in the case of $R^2$-CO-) from 2 to 16 carbon atoms, or a salt of such a compound having at least one salt-forming group.

**8.** A process according to any one of claims 1 to 6, characterised in that the starting materials are chosen so as to manufacture a compound of the formula I in which the acyl radicals $R^1_a$ -CO-, $R^1_b$ -CO- and $R^2$-CO- are derived from caprylic, pelargonic, capric, undecylic, lauric, myristic, palmitic, margaric, stearic, arachidic, behenic, oleic, elaidic, linoleic, $\alpha$- or $\beta$-eleostearic, stearolic or $\alpha$-linolenic acid or, in the case of $R^2$-CO-, also from acetic, propionic, butyric, oenanthic or caproic acid, or a salt of such a compound having at least one salt-forming group.

**9.** A process according to any one of claims 1 to 6, characterised in that the starting materials are chosen so as to manufacture a compound of the formula I in which the acyl radicals $R^1_a$ -CO, $R^1_b$ -CO- and $R^2$-CO- are derived from saturated or unsaturated cycloaliphatic-aliphatic carboxylic acids having from 8 to 16 carbon atoms or, in the case of $R^2$-CO-, from 2 to 16 carbon atoms in the aliphatic moiety and, at any position in the aliphatic carbon chain, are substituted by a cycloalkyl or cycloalkenyl ring having from 3 to 8 carbon atoms or interrupted by a cycloalkylene or cycloalkenylene radical having from 3 to 8 carbon atoms, or a salt of such a compound having at least one salt-forming group.

**10.** A process according to any one of claims 1 to 6, characterised in that the starting materials are chosen so as to manufacture a compound of the formula I in which $R^1_a$ -CO- and $R^1_b$ -CO- are different from $R^2$-CO-, and $R^1_a$ -CO-and $R^1_a$ -CO- represent lauroyl, myristoyl, palmitoyl or stearoyl and $R^2$-CO- represents stearoyl, myristoyl, lauroyl, caprinoyl or capryloyl, or a salt of such a compound having at least one salt-forming group.

**11.** A process according to any one of claims 2 and 6 to 10, characterised in that the starting materials are chosen so as to manufacture an ester of the formula I in which the ester groups are derived from lower aliphatic alcohols having from 1 to 7 carbon atoms, from monocyclic-lower aliphatic alcohols having from 1 to 7 carbon atoms in the aliphatic moiety, from $C_{1-7}$-alkoxy-, $C_{1-7}$-alkylamino- or di-$(C_{1-7})$-alkylamino- or halo-phenols, or from tetrahydrofuranol or tetrahydropyranol, or a salt of such a compound having at least one salt-forming group.

**12.** A process according to any one of claims 2 and 6 to 10, characterised in that the starting materials are chosen so as to manufacture an amide of the formula I in which the amide group is unsubstituted or is derived from a cyclic or acyclic, primary or secondary amine, the hydrocarbon radical or radicals substituting the amine nitrogen atom being, in the case of the acyclic amines, alkyl groups having from 1 to 7 carbon atoms and, in the case of the cyclic amines, alkylene groups having from 2 to 6 carbon atoms, or a salt of such a compound having at least one salt-forming group.

**13.** A process according to any one of claims 1 to 12, characterised in that the starting materials are chosen so as to manufacture a compound of the formula I in which in formula (I) of claim 1 the radical Kw in the radical $As^{\circ}$ is an unsubstituted alkylene or alkylidene radical having from 2 to 6 carbon atoms, or a salt of such a compound having at least one salt-forming group.

**14.** A process according to claim 13, characterised in that the starting materials are chosen so as to manufacture a compound of the formula I in which $As^{\circ}$ represents the radical of D- or L-lactic acid, of glycine, alanine, $\alpha$-aminobutyric acid, valine, norvaline, leucine, isoleucine, norleucine or of corresponding amino acids of the D-series, or a salt of such a compound having at least one salt-forming group.

**15.** A process according to any one of claims 1 to 12, characterised in that the starting materials are chosen so as to manufacture a compound of the formula I in which in formula (I) of claim 1, in the peptide sequence n = 0, or a salt of such a compound having at least one salt-forming group.

**16.** A process according to claim 15, characterised in that the starting materials are chosen so as to manufacture a compound of the formula I in which the sequence of the formula

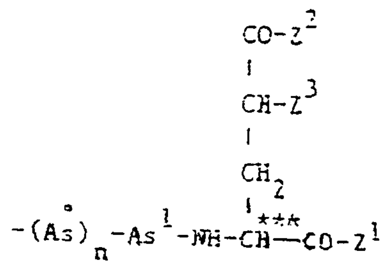

in which n represents 0, in formula (I) of claim 1 is selected from the group consisting of -Ala-D-Glu, -Ala-D-Glu-$NH_2$,-Ala-D-Glu($NH_2$), -Ala-D-Glu-D-Ala-$NH_2$, -Ala-D-Glu($NH_2$)-$NH_2$, -Ala-D-Glu(Ala), -Ala-D-Glu($NH_2$)-D-Ala-$NH_2$,-Ala-D-Glu(Ala)-$NH_2$ and -Ala-D-Glu(Gly-taurine)-$NH_2$, and corresponding sequences in which -Gly-, -Ser-, -Abu-, -Leu-, -αMeAla- (α-methyl-alanine radical) or -Val- stand in place of the first alanine radical, or a salt of such a compound having at least one salt-forming group.

**17.** A process according to any one of claims 1 to 12, characterised in that the starting materials are chosen so as to manufacture a compound of the formula (I) in which the sequence of the formula

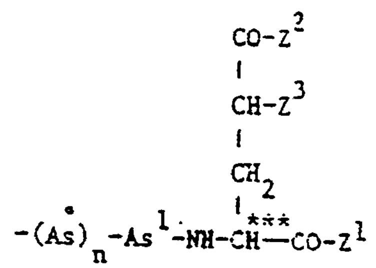

in formula (I) of claim 1 is selected from the group consisting of -As°-Ala-D-Glu, -As°-Ala-D-Glu-$NH_2$, -As°-Ala-D-Glu($NH_2$), As°-Ala-D-Glu-D-Ala-$NH_2$, As°-Ala-D-Glu($NH_2$)-$NH_2$, As°-Ala-D-Glu(Ala), As°-Ala-D-Glu($NH_2$)-D-Ala-$NH_2$ and As°-Ala-D-Glu(Ala)-$NH_2$, wherein As° represents the radical of D- or L-alanine, D- or L-lactic acid, glycolic acid or glycine, or a salt of such a compound having at least one salt-forming group.

**18.** A process according to claim 1, 2, 7, 13 or 14, characterised in that the starting materials are chosen so as to manufacture a compound of the formula I that has the R configuration at the ** centre of asymmetry in formula (I) of claim 1, in which the radicals $R_a^1$ -CO-, $\bar{R}_b^1$ -CO- and $R^2$-CO- have from 8 to 16 carbon atoms and in which the sequence of the formula

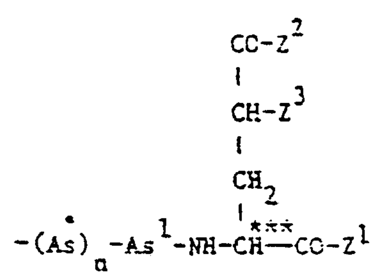

in which n represents 0 or 1, in formula I of claim 1 is selected from the group consisting of -Ala-D-Glu,

-Ala-D-Glu-$NH_2$,-Ala-D-Glu($NH_2$), -Ala-D-Glu-D-Ala-$NH_2$, -Ala-D-Glu($NH_2$)-$NH_2$, -Ala-D-Glu(Ala), -Ala-D-Glu($NH_2$)-D-Ala-$NH_2$,-Ala-D-Glu(Ala)-$NH_2$, -As°-Ala-D-Glu, -As°-Ala-D-Glu-$NH_2$, -As°-Ala-D-Glu($NH_2$), As°-Ala-D-Glu-D-Ala-$NH_2$, As°-Ala-D-Glu($NH_2$)-$NH_2$, As°-Ala-D-Glu(Ala), As°-Ala-D-Glu($NH_2$)-D-Ala-$NH_2$ and As°-Ala-D-Glu(Ala)-$NH_2$, or a salt of such a compound having at least one salt-forming group.

**19.** A process according to claim 18, characterised in that the starting materials are chosen so as to manufacture a compound of the formula I in which the acyl radicals $R_a^1$ -CO- and $R_b^1$ -CO- are different from $R^2$-CO- and represent the radical of caprylic, capric, lauric, myristic, palmitic, stearic or oleic acid, or a salt of such a compound having at least one salt-forming group.

**20.** A process according to claim 2, characterised in that the starting materials are chosen so as to manufacture a compound of the formula I selected from the group consisting of
palmitoyl-Cys(2[R],3-dilauroyloxy-propyl)-Ala-D-Glu-$NH_2$,
palmitoyl-Cys(2[R],3-dipalmitoyloxy-propyl)-Ala-D-Glu(Ala)-$NH_2$,
palmitoyl-Cys(2[R],3-dilauroyloxy-propyl)-Ala-D-Glu($NH_2$),
palmitoyl-Cys(2[R],3-dilauroyloxy-propyl)-Abu-D-Glu-$NH_2$ (Abu = $\alpha$-aminobutyric acid),
palmitoyl-Cys(2[R],3-dilauroyloxy-propyl)-Ala-D-Glu($NH_2$)-OnBu (nBu = n-butyl),
palmitoyl-Cys(2[R],3-dilauroyloxy-propyl)-Ala-D-Glu(OnBu)-$NH_2$,
palmitoyl-Cys(2[R],3-dilauroyloxy-propyl)-D-Ala-Ala-D-Glu-$NH_2$,

(D)
palmitoyl-Cys(2[R],3-dilauroyloxy-propyl)-O-CH-CO-Ala-
CH3
D-Glu-$NH_2$,

palmitoyl-Cys(2[R],3-dilauroyloxy-propyl)-O-$CH_2$CO-Ala-D-Glu-$NH_2$,
palmitoyl-Cys(2[R],3-dilauroyloxy-propyl)-Ala-D-Glu($NH_2$)-O-$CH_2$-O-CO-C($CH_3$)$_3$,
palmitoyl-Cys(2[R],3-dilauroyloxy-propyl)-Ser-D-Glu($OCH_3$)-$OCH_3$,
palmitoyl-Cys(2[R],3-dilauroyloxy-propyl)-Ala-D-Gla-$NH_2$, (Gla = $\gamma$-carboxyglutamic acid),
palmitoyl-Cys(2[R],3-dilauroyloxy-propyl)-Abu-D-Glu,
palmitoyl-Cys(2[R],3-dilauroyloxy-propyl)-Val-D-Glu-$NH_2$,
palmitoyl-Cys(2[R],3-dilauroyloxy-propyl)-$\alpha$MeAla-D-Glu-$NH_2$ ($\alpha$MeAla = $\alpha$-methyl-alanine),
palmitoyl-Cys(2[R],3-dilauroyloxy-propyl)-Ala-D-Glu(Lys-$OCH_3$)-$NH_2$,
palmitoyl-Cys(2[R],3-dilauroyloxy-propyl)-Ala-D-Glu(Lys-Lys-$OCH_3$)-$NH_2$,
palmitoyl-Cys(2[R],3-dilauroyloxy-propyl)-Ala-D-Glu-(Arg), the mono- and di-methyl ester and mono- and di-amide thereof,

palmitoyl-Cys(2[R],3-dilauroyloxy-propyl)-Ala-D-Glu(Lys-
(D)
OCH-COOH),
CH3

the mono- and di-methyl ester and mono- and di-amide thereof,
palmitoyl-Cys(2[R],3-dilauroyloxy-propyl)-Ala-D-Glu(Tly), (Tly = 4-thia-lysine), the mono- and di-methyl ester and mono- and di-amide thereof,
and corresponding lipopeptides in which, instead of palmitoyl there is present at the nitrogen of the cysteine radical lauroyl, caprinoyl, capryloyl or myristoyl, and compounds corresponding to these and to the above-listed lipopeptides in which there are present in the diacyloxypropyl radical instead of lauroyl radicals the radicals of palmitic, caprylic, capric and myristic acid, and the compounds corresponding to all of these lipopeptides in which the configuration at the chiral atom of the diacyloxypropyl radical is S instead of R, and corresponding diastereoisomeric mixtures of R and S compounds, as well as, optionally, the unsubstituted or substituted amides thereof, or a salt of such a compound having at least one salt-forming group.

**21.** A process according to claim 2, characterised in that the starting materials are chosen so as to

manufacture an ester of an aliphatic alcohol having from 1 to 7 carbon atoms or an ester of a $C_{1-7}$-alkanoyloxymethyl alcohol, $C_{1-7}$-alkanoyloxyethyl alcohol, ($C_{3-8}$-cycloalkyl)-carbonyloxymethyl alcohol, ($C_{3-8}$-cycloalkyl)-carbonyloxyethyl alcohol, propylene glycol, glycerin, or of a $C_{1-7}$-alkoxy-, $C_{1-7}$-alkylamino-, di-($C_{1-7}$-alkyl)-amino- or halo-phenol, of one of the lipopeptides mentioned in claim 20.

**22.** A process according to claim 2, characterised in that the starting materials are chosen so as to manufacture an unsubstituted amide or an amide of a $C_{1-7}$-alkylamine, pyrrolidine, piperidine or piperazine of one of the lipopeptides mentioned in claim 20.

**23.** A process according to claim 2, characterised in that the starting materials are chosen so as to manufacture a compound of the formula I selected from the group consisting of
lauroyl-Cys(2[R,S],3-didecanoyloxy-propyl)-Ala-D-Glu-$NH_2$,
decanoyl-Cys(2[R,S],3-dilauroyloxy-propyl)-Ala-D-Glu-$NH_2$,
myristoyl-Cys(2[R,S],3-dilauroyloxy-propyl)-Ala-D-Glu-$NH_2$,
palmitoyl-Cys(2[R,S],3-didecanoyloxy-propyl)-Ala-D-Glu-$NH_2$,
palmitoyl-Cys(2[R,S],3-didecanoyloxy-propyl)-Abu-D-Glu($OCH_3$)-$OCH_3$,
and of the esters and amides thereof according to either claim 21 or claim 23.

**24.** A process according to claim 1, characterised in that the starting materials are chosen so as to manufacture palmitoyl-Cys(2[R],3-dilauroyloxy-propyl)-Ala-D-Glu(Gly-taurine)-$NH_2$ or a salt thereof.

**25.** A process according to claim 1, characterised in that the starting materials are chosen so as to manufacture palmitoyl-Cys(2[R],3-dilauroyloxy-propyl)-Ala-D-Glu-$NH_2$ or a salt thereof.

**26.** A process according to claim 2, characterised in that the starting materials are chosen so as to manufacture an ammonium, alkali metal or alkaline earth metal salt of one of the acidic lipopeptides claimed in claims 1 to 25, or a pharmaceutically acceptable non-toxic acid addition salt of the basic lipopeptides claimed in the said claims.

## Revendications

**Revendications pour les Etats contractants suivants: BE, CH, DE, FR, GB, IT, LU, NL, SE**

**1.** Lipopeptides de formule

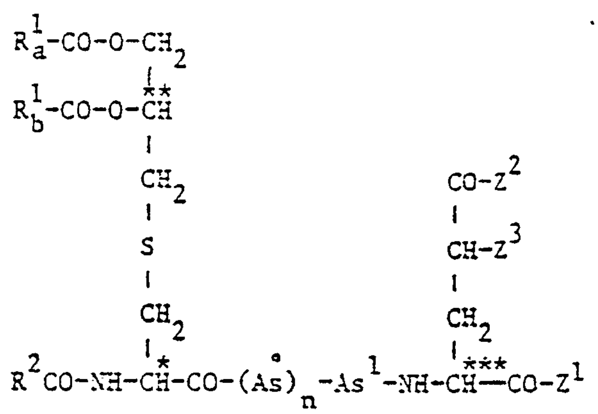

(I)

* = R
** = R ou S
*** = R

où $R^1_a$ et $R^1_b$ représentent indépendamment l'un de l'autre un reste hydrocarboné ayant 7 à 21 atomes de C, aliphatique ou cycloaliphatique-aliphatique, éventuellement substitué par des fonctions oxygène ou l'un des restes $R^1_a$ -CO et $R^1_b$ -CO représente l'hydrogène et l'autre des restes $R^1_a$ -CO et $R^1_b$ -CO représente un acyle, $R^1_a$ ou $R^1_b$ ayant la signification précitée, $R^2$ représente un reste hydrocarboné ayant 1 à 21 atomes de C, aliphatique ou cycloaliphatique-aliphatique, éventuellement substitué par des fonctions oxygène, n = 0 ou 1, $As^°$ représente un reste de formule -O-Hc-CO- ou -NH-Hc-CO-, Hc représentant un reste hydrocarboné aliphatique ayant au plus 12 atomes de C, $As^1$ représente un D- ou L-$\alpha$-amino-acide, $Z^1$ et $Z^2$ représentent indépendamment l'un de l'autre un hydroxy ou le reste N terminal d'un D- ou L-$\alpha$-amino-acide, d'un acide amino alcane inférieur sulfonique ou d'un peptide

ayant au plus 6 amino-acides du groupe des D- ou L-$\alpha$-amino-acideset des acides amino alcane inférieur sulfoniques et $Z^3$ représente l'hydrogène ou -CO-$Z^4$, $Z^4$ représentantun hydroxy ou le reste N terminal d'un D- ou L-$\alpha$-amino-acide, d'un acide amino alcane inférieur sulfonique ou d'un peptide ayant au plus 6 amino-acides du groupe des D- ou L-$\alpha$-amino-acides et des acides amino alcane inférieur sulfoniques et les amides et esters de tels composés présentant des groupes carboxyliques, les centres d'asymétrie désignés par * ou ** ou *** présentent les configurations absolues indiquées, la configuration sur un atome de C asymétrique portant le groupe $Z^3$ pouvant être R ou S et les mélanges des diastéréoisomères correspondants ainsi que les sels de tels composés comportant au moins un groupe salifiable et éventuellement les sels complexes de ces composés.

2. Composés selon la revendication 1 où $R_a^1$ et $R_b^1$ ont la même signification et représentent un reste hydrocarboné ayant 7 à 21 atomes de C, aliphatique ou cycloaliphatique-aliphatique, éventuellement substitués par des fonctions oxygène, $Z^1$ et $Z^2$ représentent indépendamment l'un de l'autre un hydroxy ou le reste N terminal d'un D- ou t-$\alpha$-amino-acide ou d'un peptide ayant au plus 6 D- ou L-$\alpha$-amino-acides et $Z^3$ représente l'hydrogène ou -Co-$Z^4$, $Z^4$ représentant un hydroxy ou le reste N terminal d'un D- ou L-$\alpha$-amino-acide ou d'un peptide contenant au plus 6 D- ou L-$\alpha$-amino-acides et les sels de tels composés ayant au moins un groupe salifiable.

3. Composés selon la revendication 2 où les $\alpha$-amino-acides sont les L-$\alpha$-amino-acides naturels ou leurs antipodes de la série D et les sels de tels composés ayant au moins un groupe salifiable.

4. Composés selon l'une des revendications 1 à 3 où un amino-acide $As^1$ est choisi dans le groupe constitué par Gly, Ala, Ser, Abu (acide $\alpha$-amino-butyrique), Val, $\alpha$MeAla ($\alpha$-méthyl-alanine) et Leu, un amino-acide dans le groupe $Z^1$, étant choisi dans le groupe Lys, Orn (ornithine), Dpm (acide $\alpha,\alpha'$-diamino-pimélique), Gly, Ala, D-Asn et D-Ala et un amino-acide $Z^2$ et/ou $Z^4$ étant choisi dans le groupe Lys, Orn, Dpm, Lan (lanthionine), Gly ou Ala, un reste peptidique $Z^1$, $Z^2$ ou $Z^4$ étant construit à partir de deux amino-acides choisis de cette façon et les sels de tels composés ayant au moins un groupe salifiable.

5. Composés selon l'une des revendications 1 à 4, dans lesquels $Z^1$ et $Z^2$ représentent dans la formule (I) indépendamment l'un de l'autre l'hydroxy ou le reste d'un amino-acide et $Z^3$ représente l'hydrogène ou -CO$Z^4$, $Z^4$ représentant l'hydroxy ou le reste d'un amino-acide et les sels de tels composés ayant au moins un groupe salifiable.

6. Composés selon la revendication 5, dans lesquels $Z^1$ représente dans la formule (I) un reste aminoacide choisi dans le groupe constitué par -Lys, -Orn (reste ornithine), -Dpm (reste $\alpha,\alpha'$-diamino-pimélique), -Gly, -Ala, -D-Ala et -D-Asn, et $Z^2$ et/ou $Z^4$ représentent un reste amino-acide choisi dans le groupe constitué par -Lys, -Orn-, -Dpm, -Lan (reste lanthionine), -Gly et -Ala et les sels de tels composés ayant au moins un groupe salifiable.

7. Composés selon l'une des revendications 1 à 6 où les restes acyles $R_a^1$ -CO-, $R_b^1$ -CO- et $R^2$-CO- dérivent d'acides gras ayant 8 à 16 atomes de C ou (dans le cas de $R^2$-CO-) 2 à 16 atomes de C, saturés ou insaturés, éventuellement oxygénés et les sels de tels composés ayant au moins un groupe salifiable.

8. Composés selon l'une des revendications 1 à 6 où les restes acyles $R_a^1$ -CO-, $R_b^1$ -CO- et $R^2$-CO- dérivent des acides caprylique, pélargonique, caprique, undécylique, laurique, myristique, palmitique, margarique, stéarique, arachique, behénique, oléique, élaïdique, linolique, $\alpha$-ou $\beta$-élaéostéarique, stéarolique ou $\alpha$ -linolénique, ou, dans le cas de $R^2$-CO-, également des acides acétique, propionique, butyrique, oenanthique ou capronique et les sels de tels composés ayant au moins un groupe salifiable.

9. Composés selon l'une des revendications 1 à 6 où les restes acyles $R_a^1$ -CO-, $R_b^1$ -CO et $R^2$-CO- dérivent d'acides carboxyliques cycloaliphatiques-aliphatiques saturés ou insaturés ayant 8 à 16 atomes de C ou, dans le cas de $R^2$-CO-, 2 à 16 atomes de C dans la partie aliphatique, et étant substitués à un endroit quelconque dans la chaîne carbonée aliphatique par un noyau cycloalkyle ou cycloalkényle ayant 3 à 8 atomes de C ou interrompus par un reste cycloalkylène ou cycloalkénylène ayant 3 à 8 atomes de C et les sels de tels composés ayant au moins un groupe salifiable.

**10.** Composés selon la revendication 9 où les restes acyles dérivent des acides dihydrosterculique, malvalique, hydrocarpusique ou chaulmoogrique et les sels de tels composés ayant au moins un groupe salifiable.

**11.** Composés selon l'une des revendications 1 à 6, dans lesquels $R_a^1$ -CO- et $R_b^1$ -CO- sont différents de $R^2$-CO- et dans lesquels $R_a^1$ -CO- et $R_b^1$ -CO- représentent un lauroyle, un myristoyle, un palmitoyle ou un stéaroyle et dans lesquels $R^2$-CO- représente le stéaroyle, le myristoyle, le lauroyle, le caprinoyle ou le capryloyle et les sels de tels composés ayant au moins un groupe salifiable.

**12.** Les esters et les amides d'un composé selon l'une des revendications 6 à 11.

**13.** Les esters selon la revendication 2 ou 12 où les groupes esters dérivent d'alcools aliphatiques, araliphatiques, aromatiques ou hétérocycliques, éventuellement substitués et les sels de tels composés ayant au moins un groupe salifiable.

**14.** Les esters selon la revendication 13 où les groupes esters aliphatiques dérivent des alcools aliphatiques inférieurs ayant 1 à 7 atomes de C, les composants esters araliphatiques dérivent des alcools monocycliques-aliphatiques inférieurs ayant 1 à 7 atomes de C dans la partie aliphatique, les composants esters aromatiques dérivent d'alcoxy en $C_{1-7}$ phénols, d'alkyle en $C_{1-7}$ aminophénols ou de dialkyle en $C_{1-7}$ aminophénols ou d'halogénophénols et les groupes esters hétérocycliques dérivent du tétrahydrofuranol ou du tétrahydropyranol et les sels de tels composés ayant au moins un groupe salifiable.

**15.** Les esters selon la revendication 13 ou 14 où les substituants dans le composant ester sont des groupes hydroxy libres, estérifiés ou éthérés, les groupes estérifiés dérivant des acides carboxyliques aliphatiques ayant 1 à 7 atomes de C et les groupes éthérés dérivant des alcools aliphatiques ayant 1 à 7 atomes de C et les sels de tels composés ayant au moins un groupe salifiable.

**16.** Les esters selon l'une des revendications 12 à 15 où le groupe $\alpha'$-carboxylique de l'amino-acide concerné est estérifié et les sels de tels composés ayant au moins un groupe salifiable.

**17.** Les mono-, di-, tri-, tétra-, penta-, hexa-et nonaesters selon l'une des revendications 12 à 16 et les sels de tels composés ayant au moins un groupe salifiable.

**18.** Les amides selon la revendication 2 ou 12 où le groupe amide est non substitué ou dérive d'une amine cyclique ou acyclique, primaire ou secondaire, le ou les reste(s) hydrocarboné(s) substituant l'atome d'azote de l'amine étant, dans le cas des amines non cycliques, des groupes alkyles ayant 1 à 7 atomes de C, et, dans le cas des amines cycliques, des groupes alkylènes ayant 2 à 6 atomes de C et les sels de tels composés ayant au moins un groupe salifiable.

**19.** Mono-, di-, tri-, tétra-, penta-, hexa- et nonaamides selon l'une des revendications 11 à 15 et les sels de tels composés ayant au moins un groupe salifiable.

**20.** Composés selon l'une des revendications 1 à 19 dans lesquels, dans la formule (I) de la revendication 1, le reste Hc dans le reste $As^{\circ}$ représente un reste alkylène ou alkylidène ayant 2 à 6 atomes de C et les sels de tels composés ayant au moins un groupe salifiable.

**21.** Composés selon la revendication 20 où Hc représente le méthylène, le di-, tri- ou tétraméthylène, l'éthylidène, le 2-propylidène, le 2,2-diméthyléthylidène, le 2-butylidène, le 3,3-diméthylpropylidène, le 2-méthyléthylidène, le 2-éthyléthylidène ou le pentylidène et les sels de tels composés ayant au moins un groupe salifiable.

**22.** Composés selon la revendication 20 où $As^{\circ}$ représente le reste de l'acide D- ou L-lactique, de la glycine, de l'alanine₁ de l'acide $\alpha$-amino-butyrique, de la valine, de la norvaline, de la leucine, de l'isoleucine, de la norleucine ou des amino-acides correspondants de la série D et les sels de tels composés ayant au moins un groupe salifiable.

**23.** Composés selon l'une des revendications 1 à 19 où dans la formule (I) de la revendication 1 dans la

séquence peptidique n = 0 et les sels de tels composés ayant au moins un groupe salifiable.

**24.** Composés selon la revendication 23 où la séquence de formule

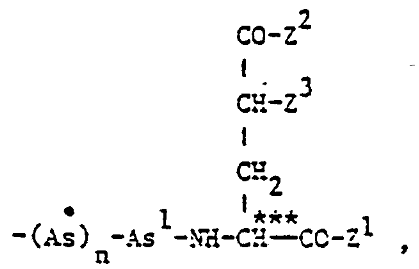

n représentant 0, dans la formule (I) de la revendication 1, est choisie dans le groupe constitué par -Ala-D-Glu, -Ala-D-Glu-$NH_2$, -Ala-D-Glu($NH_2$), -Ala-D-Glu-D-Ala-$NH_2$, -Ala-D-Glu($NH_2$)-$NH_2$, -Ala-D-Glu-(Ala), -Ala-D-Glu($NH_2$)-D-Ala-$NH_2$, -Ala-D-Glu(Ala)-$NH_2$ et Ala-D-Glu(Gly-taurine)-$NH_2$ ainsi que par les séquences correspondantes où figurent -Gly-, -Ser-, -Abu-, -Leu-, -αMeAla- (reste α -méthylalanine) ou -Val- à la place du premier reste alanine et les sels de tels composés ayant au moins un groupe salifiable.

**25.** Composés selon l'une des revendications 1 à 21 où la séquence de formule

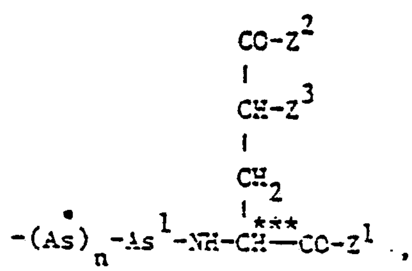

dans la formule (I) de la revendication 1 est choisie dans le groupe constitué par -As° -Ala-D-Glu, -As° -Ala-D-Glu-$NH_2$,-As° -Ala-D-Glu($NH_2$), As° -Ala-D-Glu-D-Ala-$NH_2$, As° -Ala-D-Glu($NH_2$)-$NH_2$, As° -Ala-D-Glu(Ala), As° -Ala-D-Glu($NH_2$)-D-Ala-$NH_2$ et As° -Ala-D-Glu(Ala)-$NH_2$, As° représentant le reste de la D- ou L-alanine, de l'acide D- ou L-lactique, de l'acide glycolique ou de la glycérine et les sels de tels composés ayant au moins un groupe salifiable.

**26.** Composés de formule (I) selon la revendication 1, 2, 7 et 20 à 22, lesdits composés ayant la configuration R sur le centre d'asymétrie ** dans la formule (I) de la revendication 1, dans lesquels les restes $R_a^1$ -CO-, $R_b^1$ -CO- et $R^2$-CO- présentent 8 à 16 atomes de C et où la séquence de formule

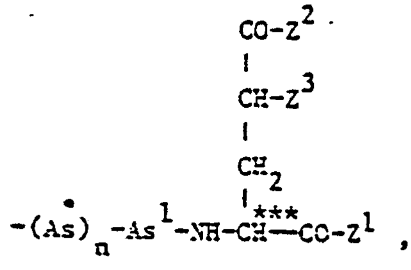

n représentant 0 ou 1, dans la formule (I) de la revendication 1, est choisie dans le groupe constitué par -Ala-D-Glu, -Ala-D-Glu-$NH_2$, -Ala-D-Glu($NH_2$), -Ala-D-Glu-D-Ala-$NH_2$, -Ala-D-Glu($NH_2$)-$NH_2$, -Ala-D-Glu-(Ala), -Ala-D-Glu($NH_2$)-D-Ala-$NH_2$, -Ala-D-Glu(Ala)-$NH_2$, -As° -Ala-D-Glu, -As° -Ala-D-Glu-$NH_2$, -As° -Ala-D-Glu($NH_2$), As° -Ala-D-Glu-D-Ala-$NH_2$, As° -Ala-D-Glu($NH_2$)-$NH_2$, As° -Ala-D-Glu(Ala), As° -Ala-D-Glu-($NH_2$)-D-Ala-$NH_2$ et As° -Ala-D-Glu(Ala)-$NH_2$ et les sels de tels composés ayant au moins un groupe

salifiable.

**27.** Composés selon la revendication 26 où les restes acyles $R^1_a$ -CO et $R^1_b$ -CO- sont différents de $R^2$-CO- et représentent le reste des acides caprylique, caprique, laurique, myristique, palmitique, stéarique ou oléique et les sels de tels composés ayant au moins un groupe salifiable.

**28.** Un lipopeptide selon la revendication 2, choisi dans le groupe constitué par
le palmitoyl-Cys(2[R],3-dilauroyloxy-propyl)-Ala-D-Glu-$NH_2$,
le palmitoyl-Cys(2[R],3-dipalmitoyloxy-propyl)-Ala-D-Glu(Ala)-$NH_2$,
le palmitoyl-Cys(2[R],3-dilauroyloxy-propyl)-Ala-D-Glu($NH_2$),
le palmitoyl-Cys(2[R],3-dilauroyloxy-propyl)-Abu-D-Glu-$NH_2$ (Abu = l'acide α-amino-butyrique),
le palmitoyl-Cys(2[R],3-dilauroyloxy-propyl)-Ala-D-Glu ($NH_2$)-OnBu(nBu = n-butyle),
le palmitoyl-Cys(2[R],3-dilauroyloxy-propyl)-Ala-D-Glu(OnBu)-$NH_2$,
le palmitoyl-Cys(2[R],3-dilauroyloxy-propyl)-D-Ala-D-Glu-$NH_2$,

(D)
le palmitoyl-Cys(2[R],3-dilauroyloxy-propyl)-O-CH-CO-
$CH_3$
Ala-D-Glu-$NH_2$,

le palmitoyl-Cys(2[R],3-dilauroyloxy-propyl)-O-$CH_2$CO-Ala-D-Glu-$NH_2$,
le palmitoyl-Cys(2[R],3-dilauroyloxy-propyl)-Ala-D-Glu($NH_2$)-O-$CH_2$-O-CO-C($CH_3$)$_3$,
le palmitoyl-Cys(2[R],3-dilauroyloxy-propyl)-Ser-D-Glu($OCH_3$)-$OCH_3$,
le palmitoyl-Cys(2[R],3-dilauroyloxy-propyl)-Ala-D-Gla-$NH_2$ (Gla = l'acide γ-carboxy-glutamique),
le palmitoyl-Cys(2[R],3-dilauroyloxy)-propyl)-Abu-D-Glu,
le palmitoyl-Cys(2[R],3-dilauroyloxy-propyl)-Val-D-Glu-$NH_2$,
le palmitoyl-Cys(2[R],3-dilauroyloxy-propyl)-αMeAla-D-Glu-$NH_2$ (αMeAla = α-méthyl-alanine),
la palmitoyl-Cys(2[R],3-dilauroyloxy-propyl)-Ala-D-Glu-(Lys-$OCH_3$)-$NH_2$,
le palmitoyl-Cys(2[R],3-dilauroyloxy-propyl)-Ala-D-Glu(Lys-Lys-$OCH_3$)-$NH_2$,
le palmitoyl-Cys(2[R],3-dilauroyloxy-propyl)-Ala-D-Glu(Arg),
son ester mono- et diméthylique et son mono- et diamide,

le palmitoyl-Cys(2[R],3-dilauroyloxy-propyl)-Ala-D-
(D)
Glu(Lys-OCH-COOH)
$CH_3$

son ester mono- et diméthylique et son mono- et diamide, le palmitoyl-Cys(2[R],3-dilauroyloxy-propyl)-Ala-D-Glu(Tly) (Tly = 4-thia-lysine), son ester mono- et diméthylique et son mono- et diamide,
et les lipopeptides correspondants dans lesquels se trouvent, à la place du palmitoyle sur l'azote du reste cystéine, le lauroyle, le caprinoyle, le capryloyle ou le myristoyle et les composés correspondant aux lipoprotéines indiquées ci-dessus, dans lesquels se trouvent, dans le reste diacyloxypropyle à la place des restes lauroyles, les restes des acides palmitique, caprylique, caprique et myristique ainsi que les composés correspondant à tous ces lipopeptides, dans lesquels la configuration sur l'atome chiral du reste diacyloxypropyle est S au lieu de R et les mélanges de diastéréoisomères correspondants des composés R et S, ainsi qu'éventuellement leurs amides non substitués ou substitués et les sels de tels composés ayant au moins un groupe salifiable.

**29.** Composés selon la revendication 2, à savoir les esters des alcools aliphatiques ayant 1 à 7 atomes de C ou les esters des alcools alcanoyle en $C_{1-7}$ oxyméthyliques, des alcools alcanoyle en $C_{1-7}$ oxyéthyliques, des alcools (cycloalkyles en $C_{3-8}$)-carbonyloxyméthyliques, des alcools (cycloalkyles en $C_{3-8}$)-carbonyloxyéthyliques, du propylèneglycol, de la glycérine ou d'un alcoxy en $C_{1-7}$ phénol, d'un

alkyle en $C_{1-7}$ aminophénol, d'un dialkyle en $C_{1-7}$ aminophénol ou d'un halogénophénol d'un lipopeptide cité dans la revendication 28.

**30.** Composés selon la revendication 2, à savoir les amides non substitués ou les amides des alkyles en $C_{1-7}$ amines, la pyrrolidine, la pipéridine ou la pipérazine d'un des lipopeptides cités dans la revendication 28.

**31.** Un lipopeptide selon la revendication 2, choisi dans le groupe constitué par
le lauroyl-Cys(2[R,S],3-didécanoyloxy-propyl)-Ala-D-Glu-$NH_2$,
le décanoyl-Cys(2[R,S],3-dilauroyloxy-propyl)-Ala-D-Glu-$NH_2$,
le myristoyl-Cys(2[R,S],3-dilauroyloxy-propyl)-Ala-D-Glu-$NH_2$,
le palmitoyl-Cys(2[R,S],3-didécanoyloxy-propyl)-Ala-D-Glu-$NH_2$,
le palmitoyl-Cys(2[R,S],3-didécanoyloxy-propyl)-Abu-D-Glu($OCH_3$)-$OCH_3$
et de leurs esters et amides selon l'une des revendications 29 et 30.

**32.** Le palmitoyl-Cys(2[R],3-dilauroyloxy-propyl)-Ala-D-Glu(Gly-taurine)-$NH_2$ ou l'un de ses sels selon la revendication 1.

**33.** Le palmitoyl-Cys(2[R],3-dilauroyloxy-propyl)Ala-D-Glu-$NH_2$ ou l'un de ses sels selon la revendication 1.

**34.** Composés selon la revendication 1 ou 2, à savoir les sels d'ammonium, les sels des métaux alcalins ou alcalino-terreux d'un des lipopeptides acides revendiqués dans les revendications 1 à 33 et les sels d'addition d'acides non toxiques, pharmaceutiquement utilisables, des lipopeptides basiques revendiqués dans les revendications précitées.

**35.** Composés de formule (I) ou l'un de leurs sels pharmaceutiquement utilisables selon l'une des revendications 1 à 34 destinées à être utilisés dans un procédé pour augmenter les défenses corporelles du corps humain ou animal.

**36.** Préparations pharmaceutiques contenant un composé de formule (I) ou l'un de ses sels pharmaceutiquement utilisables selon l'une des revendications 1 à 34, en association avec un support pharmaceutique.

**37.** Utilisation des lipopeptides de formule (I) ou de l'un de leurs sels pharmaceutiquement utilisables selon l'une des revendications 1 à 34 pour la préparation de compositions pharmaceutiques destinées à être utilisées pour l'obtention d'un effet immunostimulant ou comme prophylactique ou thérapeutique contre les maladies infectieuses chez l'homme et l'animal.

**38.** Préparation de combinaisons contenant un ou plusieurs des lipopeptides de formule (I) ou un de leurs sels pharmaceutiquement utilisables selon l'une des revendications 1 à 34 en association avec un ou plusieurs antibiotiques.

**39.** Procédé pour la préparation d'un composé de formule I

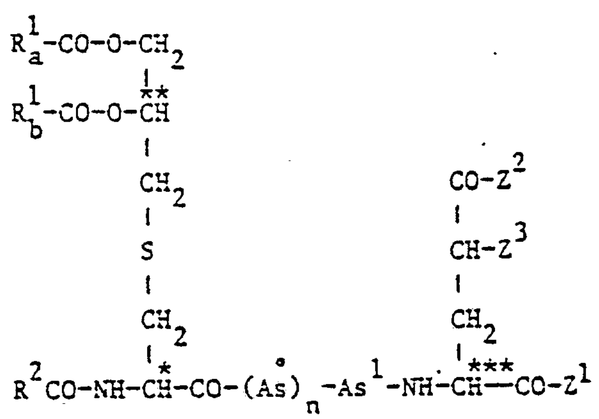

(I)

* = R

** = R ou S

*** = R

où $R_a^1$ et $R_b^1$ représentent indépendamment l'un de l'autre un reste hydrocarboné ayant 7 à 21 atomes de C, aliphatique ou cycloaliphatique-aliphatique, éventuellement substitué par des fonctions oxygène ou l'un des restes $R_a^1$ -CO et $R_b^1$ -CO représente l'hydrogène et l'autre des restes $R_a^1$ -CO et $R_b^1$ -CO représente un acyle, $R_a^1$ ou $R_b^1$ ayant la signification précitée, $R^2$ représente un reste hydrocarboné ayant 1 à 21 atomes de C, aliphatique ou cycloaliphatique-aliphatique, éventuellement substitué par des fonctions oxygène, n = 0 ou 1, $As^\circ$ représente un reste de formule -O-Hc-CO- ou -NH-Hc-CO-, Hc représentant un reste hydrocarboné aliphatique ayant au plus 12 atomes de C, $As^1$ représente un D- ou L-$\alpha$-amino-acide, $Z^1$ et $Z^2$ représentent indépendamment l'un de l'autre un hydroxy ou le reste N terminal d'un D- ou L- $\alpha$ -amino-acide, d'un acide amino alcane inférieur sulfonique ou d'un peptide ayant au plus 6 amino-acides du groupe des D- ou L- $\alpha$ -amino-acides et des acides amino alcane inférieur sulfoniques et $Z^3$ représente l'hydrogène ou -CO-$Z^4$, $Z^4$ représentantun hydroxy ou le reste N terminal d'un D- ou L- $\alpha$ -amino-acide, d'un acide amino alcane inférieur sulfonique ou d'un peptide ayant au plus 6 amino-acides du groupe des D- ou L- $\alpha$ -amino-acides et des acides amino alcane inférieur sulfoniques ou d'un amide ou d'un ester d'un tel composé présentant au moins un groupe carboxylique, les centres d'asymétrie désignés par * ou ** ou *** présentant les configurations absolues indiquées, la configuration sur un atome de C asymétrique portant le groupe $Z^3$ pouvant être R ou S ou d'un mélange de diastéréoisomères correspondant à un sel d'un tel composé comportant au moins un groupe salifiable ou éventuellement d'un sel complexe d'un tel composé,
caractérisé

a) en ce que l'on clive, dans un composé correspondant à la formule (I) ou dans un sel de celui-ci, les substituants ayant les significations données ci-dessus, sous réserve que la chaîne peptidique

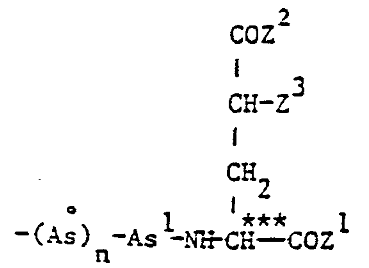

(II)

contienne au moins un groupe fonctionnel protégé, le ou les groupe(s) protecteur(s) ou

b) en ce que l'on acyle un composé de formule I dans laquelle au moins l'un des restes $R_a^1$ -CO-, $R_b^1$ -CO- et $R^2$-CO-représente l'hydrogène et les autres substituants ont les significations données ci-dessus, sous réserve que, dans ce produit de départ, les groupes fonctionnels libres présents se trouvent, si nécessaire, sous forme protégée, à l'exception du ou des groupe(s) hydroxy et/ou amino participant à la réaction ou un sel de celui-ci, avec un acide $R_a^1$ -COOH $R_b^1$ -COOH ou $R^2$-COOH ou un dérivé d'acide carboxylique réactif de celui-ci et en ce que l'on clive les groupes protecteurs présents ou

c) en ce que l'on prépare une liaison amide d'un composé de formule (I) par réaction d'un fragment correspondant d'un composé de formule (I) avec un groupe carboxyle libre ou d'un dérivé réactif d'acide de celui-ci avec un fragment complémentaire comportant un groupe amino libre ou avec un dérivé réactif de celui-ci comportant un groupe amino activé, les groupes fonctionnels libres présents dans les composants réactionnels se trouvant, si nécessaire, sous forme protégée, à l'exception des groupes participant à la réaction et en ce que l'on clive les groupes protecteurs présents ou

d) en ce que l'on estérifie ou met sous forme d'amide dans un composé de formule (I) dans laquelle au moins est présent un groupe carboxyle libre, le ou les groupe(s) carboxyle(s) libre(s) et/ou en ce que l'on saponifie, dans un composé de formule (I) dans laquelle au moins est présent un groupe ester, le ou les groupe(s) ester(s) ou

e) en ce que l'on fait réagir un composé de formule

III

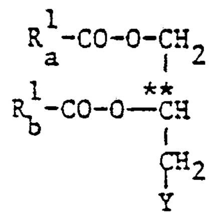

** = R ou S

(III)

où $R_a^1$ et $R_b^1$ ont les significations données ci-dessus et Y représente un groupe nucléofuge avec un composé de formule IV :

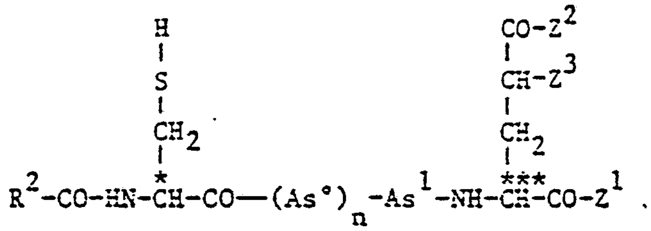

* = R

*** = R

(IV)

où les substituants ont les significations données ci-dessus, les groupes fonctionnels libres étant, si nécessaire, protégés avec des groupes protecteurs facilement clivables, à l'exception du groupe mercapto participant à la réaction, ou avec un dérivé réactif d'un composé de formule IV et en ce que l'on clive les groupes protecteurs présents ou

f) en ce que l'on fait réagir un composé de formule V

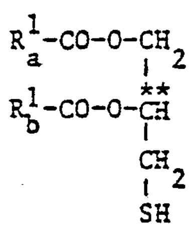

** = R ou S

(V)

où $R_a^1$ et $R_b^1$ ont les significations données ci-dessus ou un dérivé réactif de ce composé avec un composé de formule VI

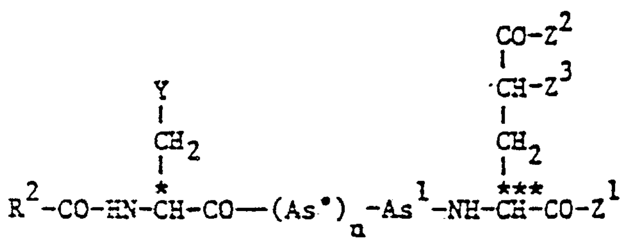

* = R

*** = R

(VI)

où Y représente un groupe nucléofuge et les autres substituants ont les significations données ci-dessus, les groupes fonctionnels libres se présentant, si nécessaire, sous forme protégée et en ce que l'on clive les groupes protecteurs présents et, si désiré, en ce que l'on transforme, après exécution de l'une des variantes a-f) du procédé le composé de formule I obtenu ayant au moins un groupe salifiable en un sel ou en un sel complexe ou en transformant le sel ou le sel complexe obtenu en un composé libre et, si désiré, en séparant les mélanges d'isomères obtenus.

**Revendications pour l'Etat Contractant suivant: AT**

1. Procédé pour la préparation d'un composé de formule I

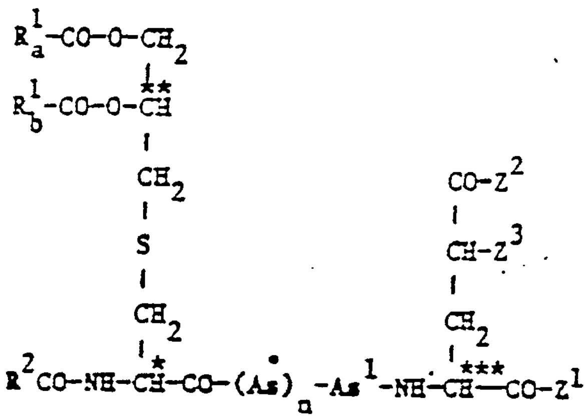

(I)

* = R

** = R ou S

*** = R

où $R_a^1$ et $R_b^1$ représentent indépendamment l'un de l'autre un reste hydrocarboné ayant 7 à 21 atomes de C, aliphatique ou cycloaliphatique-aliphatique, éventuellement substitué par des fonctions oxygène ou l'un des restes $R_a^1$ -CO et $R_b^1$ -CO représente l'hydrogène et l'autre des restes $R_a^1$ -CO et $R_b^1$ -CO représente un acyle, $R_a^1$ ou $R_b^1$ ayant la signification précitée, $R^2$ représente un reste hydrocarboné ayant 1 à 21 atomes de C, aliphatique ou cycloaliphatique-aliphatique, éventuellement substitué par des fonctions oxygène, n = 0 ou 1, $As^{\circ}$ représente un reste de formule -O-Hc-CO- ou -NH-Hc-CO-, Hc représentant un reste hydrocarboné aliphatique ayant au plus 12 atomes de C, $As^1$ représente un D- ou L- $\alpha$ -amino-acide, $Z^1$ et $Z^2$ représentent indépendamment l'un de l'autre un hydroxy ou le reste N terminal d'un D- ou L-$\alpha$-amino-acide, d'un acide amino alcane inférieur sulfonique ou d'un peptide ayant au plus 6 amino-acides du groupe des D- ou L-$\alpha$-amino-acideset des acides amino alcane inférieur sulfoniques et $Z^3$ représente l'hydrogène ou -CO-$Z^4$, $Z^4$ représentantun hydroxy ou le reste N terminal d'un D- ou L-$\alpha$-amino-acide, d'un acide amino alcane inférieur sulfonique ou d'un peptide ayant au plus 6 amino-acides du groupe des D- ou L- $\alpha$ -amino-acides et des acides amino alcane inférieur sulfoniques ou d'un amide ou d'un ester d'un tel composé présentant au moins un groupe carboxylique, les centres d'asymétrie désignés par * ou ** ou *** présentant les configurations absolues indiquées, la configuration sur un atome de C asymétrique portant le groupe $Z^3$ pouvant être R ou S, ou d'un mélange de diastéréoisomères correspondant ou d'un sel d'un tel composé comportant au moins un groupe salifiable ou éventuellement d'un sel complexe d'un tel composé, caractérisé

a) en ce que l'on clive, dans un composé correspondant à la formule (I) ou dans un sel de celui-ci, les substituants ayant les significations données ci-dessus, sous réserve que la chaîne peptidique

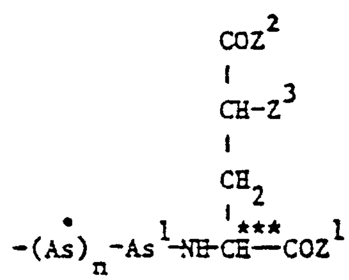

(II)

contiennent au moins un groupe fonctionnel protégé, le ou les groupe(s) protecteur(s) ou

b) en ce que l'on acyle un composé de formule I dans laquelle au moins l'un des restes $R_a^1$ -CO-, $R_b^1$ -CO- et $R^2$-CO- représente l'hydrogène et les autres substituants ont les significations données ci-dessus, sous réserve que, dans ce produit de départ, les groupes fonctionnels libres présents se trouvent, si nécessaire, sous forme protégée, à l'exception du ou des groupe(s) hydroxy et/ou amino participant à la réaction ou un sel de celui-ci, avec un acide $R_a^1$ -COOH, $R_b^1$ -COOH ou $R^2$-COOH ou un dérivé d'acide carboxylique réactif de celui-ci et en ce que l'on clive les groupes protecteurs

présents ou

c) en ce que l'on prépare une liaison amide d'un composé de formule (I) par réaction d'un fragment correspondant d'un composé de formule (I) avec un groupe carboxyle libre ou d'un dérivé réactif d'acide de celui-ci avec un fragment complémentaire comportant un groupe amino libre ou avec un dérivé réactif de celui-ci comportant un groupe amino activé, les groupes fonctionnels libres présents dans les composants réactionnels se trouvant, si nécessaire, sous forme protégée, à l'exception des groupes participant à la réaction et en ce que l'on clive les groupes protecteurs présents ou

d) en ce que l'on estérifie ou met sous forme d'amide dans un composé de formule (I) dans laquelle au moins est présent un groupe carboxyle libre, le ou les groupe(s) carboxyle(s) libre(s) et/ou en ce que l'on saponifie, dans un composé de formule (I) dans laquelle au moins est présent un groupe ester, le ou les groupe(s) ester(s) ou

e) en ce que l'on fait réagir un composé de formule III

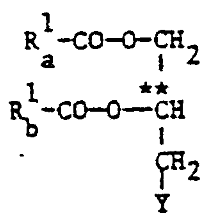

** = R ou S

(III)

où $R_a^1$ et $R_b^1$ ont les significations données ci-dessus et Y représente un groupe nucléofuge avec un composé de formule IV

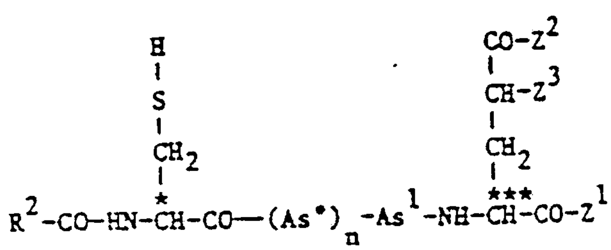

* = R

*** = R

(IV)

où les substituants ont les significations données ci-dessus, les groupes fonctionnels libres étant, si nécessaire, protégés avec des groupes protecteurs facilement clivables, à l'exception du groupe mercapto participant à la réaction, ou avec un dérivé réactif d'un composé de formule IV et en ce que l'on clive les groupes protecteurs présents ou

f) en ce que l'on fait réagir un composé de formule V

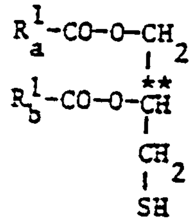

** = R ou S

(V)

où $R_a^1$ et $R_b^1$ ont les significations données ci-dessus ou un dérivé réactif de ce composé avec un composé de formule VI

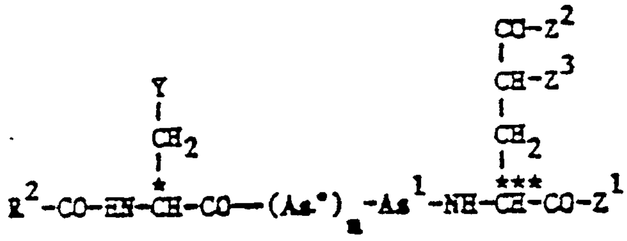

* = R

*** = R (VI)

où Y représente un groupe nucléofuge et les autres substituants ont les significations données ci-dessus, les groupes fonctionnels libres se présentant, si nécessaire, sous forme protégée et en ce que l'on clive les groupes protecteurs présents et, si désiré, en ce que l'on transforme, après exécution de l'une des variantes a-f) du procédé le composé de formule I obtenu ayant au moins un groupe salifiable en un sel ou en un sel complexe ou en transformant le sel ou le sel complexe obtenu en un composé libre et, si désiré, en séparant les mélanges d'isomères obtenus.

2. Procédé selon la revendication 1, caractérisé en ce que l'on choisit les produits de départ de telle façon que l'on prépare un composé de formule I où $R_a^1$ et $R_b^1$ ont la même signification et représentent un reste hydrocarboné ayant 7 à 21 atomes de C, aliphatique ou cycloaliphatique-aliphatique, éventuellement substitués par des fonctions oxygène, $Z^1$ et $Z^2$ représentent indépendamment l'un de l'autre un hydroxy ou le reste N terminal d'un D- ou L-$\alpha$-amino-acide ou d'un peptide ayant au plus 6 D- ou L-$\alpha$-amino-acides et $Z^3$ représente l'hydrogène ou -CO-$Z^4$, $Z^4$ représentant un hydroxy ou le reste N terminal d'un D- ou L-$\alpha$ -amino-acide ou d'un peptide contenant au plus 6 D- ou L- $\alpha$-amino-acides.

3. Préparation selon la revendication 2, caractérisé en ce que l'on choisit les produits de départ de telle façon que l'on prépare un composé de formule I où les $\alpha$-amino-acides sont les L- $\alpha$-amino-acides naturels ou leurs antipodes de la série D ou un sel d'un tel composé ayant au moins un groupe salifiable.

4. Procédé selon l'une des revendications 1 à 3, caractérisé en ce que l'on choisit les produits de départ de telle façon que l'on prépare un composé (I) où un amino-acide $As^1$ est choisi dans le groupe constitué par Gly, Ala, Ser, Abu (acide $\alpha$-amino-butyrique), Val, $\alpha$MeAla ( $\alpha$-méthyl-alanine) et Leu, un amino-acide dans le groupe $Z^1$, étant choisi dans le groupe Lys, Orn (ornithine), Dpm (acide $\alpha$, $\alpha$'-diamino-pimélique), Gly, Ala, D-Asn et D-Ala et un amino-acide $Z^2$ et/ou $Z^4$ étant choisi dans le groupe Lys, Orn, Dpm, Lan (lanthionine), Gly ou Ala, un reste peptidique $Z^1$, $Z^2$ ou $Z^4$ étant construit à partir de deux amino-acides choisis de cette façon ou un sel d'un tel composé ayant au moins un groupe salifiable.

5. Procédé selon l'une des revendications 1 à 4, caractérisé en ce que l'on choisit les produits de départ de telle façon que l'on prépare un composé de formule I, dans laquelle $Z^1$ et $Z^2$ représentent dans la formule (I) indépendamment l'un de l'autre l'hydroxy ou le reste d'un amino-acide et $Z^3$ représente l'hydrogène ou -CO$Z^4$, $Z^4$ représentant l'hydroxy ou le reste d'un amino-acide ou un sel d'un tel composé ayant au moins un groupe salifiable.

6. Procédé selon la revendication 5, caractérisé en ce que l'on choisit les produits de départ de telle façon que l'on prépare un composé de formule I dans laquelle $Z_1$ représente dans la formule (I) un reste amino-acide choisi dans le groupe constitué par -Lys, -Orn (reste ornithine), -Dpm (reste $\alpha$,$\alpha$'-diamino-pimélique), -Gly, -Ala, -D-Ala et -D-Asn, et $Z^2$ et/ou $Z^4$ représentent un reste amino-acide choisi dans le groupe constitué par -Lys, -Orn-, -Dpm, -Lan (reste lanthionine), -Gly et -Ala ou un sel d'un tel composé ayant au moins un groupe salifiable.

7. Procédé selon l'une des revendications 1 à 6, caractérisé en ce que l'on choisit les produits de départ de telle façon que l'on prépare un composé de formule I où les restes acyles $R_a^1$ -CO-, $R_b^1$ -CO- et $R^2$-CO- dérivent d'acides gras ayant 8 à 16 atomes de C ou (dans le cas de $R^2$-CO-) 2 à 16 atomes de C, saturés ou insaturés, éventuellement oxygénés ou un sel d'un tel composé ayant au moins un groupe salifiable.

**8.** Procédé selon l'une des revendications 1 à 6, caractérisé en ce que les produits de départ sont choisis de telle façon que l'on prépare un composé de formule (I) où les restes acyles $R_a^1$ -CO-, $R_b^1$ -CO- et $R^2$-CO- dérivent des acides caprylique, pélargonique, caprique, undécylique, laurique, myristique, palmitique, margarique, stéarique, arachique, behénique, oléique, élaïdique, linolique,α-ou β-élaéostéarique, stéarolique ou α-linolénique, ou, dans le cas de $R^2$-CO-, également des acides acétique, propionique, butyrique, oenanthique ou capronique ou un sel d'un tel composé ayant au moins un groupe saponifiable.

**9.** Procédé selon l'une des revendications 1 à 6, caractérisé en ce que l'on choisit les produits de départ de telle façon que l'on prépare un composé de formule I où les restes acyle $R_a^1$ -CO-, $R_b^1$ -CO- et $R^2$-CO-dérivent d'acides carboxyliques cycloaliphatiques-aliphatiques saturés ou insaturés ayant 8 à 16 atomes de C ou, dans le cas de $R^2$-CO-, 2 à 16 atomes de C dans la partie aliphatique, et étant substitués à un endroit quelconque dans la chaîne carbonée aliphatique par un noyau cycloalkyle ou cycloalkényle ayant 3 à 8 atomes de C ou interrompus par un reste cycloalkylène ou cycloalkénylène ayant 3 à 8 atomes de C ou un sel d'un tel composé ayant au moins un groupe salifiable.

**10.** Procédé selon l'une des revendications 1 à 6, caractérisé en ce que l'on choisit les produits de départ de telle façon que l'on prépare un composé de formule I dans laquelle $R_a^1$ -CO- et $R_b^1$ -CO- sont différents de $R^2$-CO- et $R_a^1$ -CO- et $R_b^1$ -CO- représentent le lauroyle, le myristoyle, le palmitoyle ou le stéaroyle et $R^2$-CO-, le stéaroyle, le myristoyle, le lauroyle, le caprinoyle ou le capryloyle ou un sel d'un tel composé ayant au moins un groupe salifiable.

**11.** Procédé selon l'une des revendications 2 et 6 à 10, caractérisé en ce que l'on choisit les produits de départ de telle façon que l'on prépare un ester de formule I, les groupes esters dérivant des alcools aliphatiques inférieurs ayant 1 à 7 atomes de C, des alcools monocycliques-aliphatiques inférieurs ayant 1 à 7 atomes de C dans la partie aliphatique, des alcoxy en $C_{1-7}$ phénols, des alkyle en $C_{1-7}$ aminophénols ou des dialkyle en $C_{1-7}$ aminophénols ou des halogénophénols ou du tétrahydrofuranol ou du tétrahydropyranol ou un sel d'un tel composé ayant au moins un groupe salifiable.

**12.** Procédé selon l'une des revendications 2 et 6 à 10, caractérisé en ce que l'on choisit les produits de départ de telle façon que l'on prépare les amides de formule I où le groupe amide est non substitué ou dérive d'une amine cyclique ou acyclique, primaire ou secondaire, le ou les reste(s) hydrocarboné(s) substituant l'atome d'azote de l'amine étant, dans le cas des amines non cycliques, des groupes alkyles ayant 1 à 7 atomes de C, et, dans le cas des amines cycliques, des groupes alkylènes ayant 2 à 6 atomes de C ou un sel d'un tel composé ayant au moins un groupe salifiable.

**13.** Procédé selon l'une des revendications 1 à 12, caractérisé en ce que l'on choisit les produits de départ de telle façon que l'on prépare un composé de formule I dans lequel dans la formule (I) de la revendication 1, le reste Hc dans le reste $As^{\circ}$ représente un reste alkylène ou alkylidène ayant 2 à 6 atomes de C ou un sel d'un tel composé ayant au moins un groupe salifiable.

**14.** Procédé selon la revendication 13, caractérisé en ce que l'on choisit les produits de départ de telle façon que l'on prépare un composé de formule I dans lequel $As^{\circ}$ représente le reste de l'acide D- ou L-lactique, de la glycine, de l'alanine, de l'acide α-amino-butyrique, de la valine, de la norvaline, de la leucine, de l'isoleucine, de la norleucine ou des amino-acides correspondants de la série D ou un sel d'un tel composé ayant au moins un groupe salifiable.

**15.** Procédé selon l'une des revendications 1 à 12, caractérisé en ce que l'on choisit les produits de départ de telle façon que l'on prépare un composé de formule I où dans la formule (I) de la revendication 1 dans la séquence peptidique n = 0 ou un sel d'un tel composé ayant au moins un groupe salifiable.

**16.** Procédé selon la revendication 15, caractérisé en ce que l'on choisit les produits de départ de telle façon que l'on prépare un composé de formule I où la séquence de formule

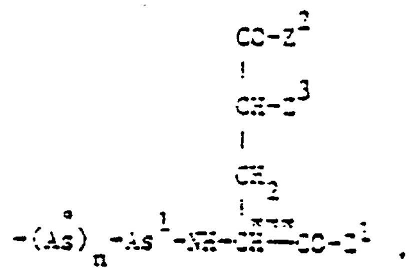

n représentant 0, dans la formule (I) de la revendication 1, est choisie dans le groupe constitué par -Ala-D-Glu, -Ala-D-Glu-$NH_2$, -Ala-D-Glu($NH_2$), -Ala-D-Glu-D-Ala-$NH_2$, -Ala-D-Glu($NH_2$)-$NH_2$, -Ala-D-Glu-(Ala), -Ala-D-Glu($NH_2$-D-Ala-$NH_2$, -Ala-D-Glu(Ala)-$NH_2$ et Ala-D-Glu(Gly-taurine-$NH_2$ ainsi que par les séquences correspondantes où figurent -Gly-, -Ser-, -Abu-, -Leu-, $\alpha$-MeAla- (reste $\alpha$-méthylalanine) ou -Val- à la place du premier reste alanine ou un sel d'un tel composé ayant au moins un groupe salifiable.

**17.** Procédé selon l'une des revendications 1 à 22, caractérisé en ce que l'on choisit les produits de départ de telle façon que l'on prépare un composé de formule (I) où la séquence de formule

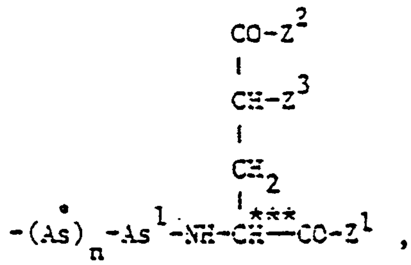

dans la formule (I) de la revendication 1, est choisie dans le groupe constitué par -As° -Ala-D-Glu, -As° -Ala-D-Glu-$NH_2$, -As° -Ala-D-Glu($NH_2$), As° -Ala-D-Glu-D-Ala-$NH_2$, As° -Ala-D-Glu($NH_2$)-$NH_2$, As° -Ala-D-Glu(Ala), As° -Ala-D-Glu($NH_2$)-D-Ala-$NH_2$ et As° -Ala-D-Glu(Ala)-$NH_2$, As° représentant le reste de la D- ou L-alanine, de l'acide D-ou L-lactique, de l'acide glycolique ou de la glycérine ou un sel d'un tel composé ayant au moins un groupe salifiable.

**18.** Procédé selon les revendications 1, 2, 7, 13 ou 14, caractérisé en ce que les produits de départ sont choisis de telle façon que l'on prépare un composé de formule I ayant la configuration R sur le centre d'asymétrie ** dans la formule (I) de la revendication 1, dans lequel les restes $R_a^1$ -CO-, $R_b^1$ -CO- et $R^2$-CO- présentent 8 à 16 atomes de C et où la séquence de formule

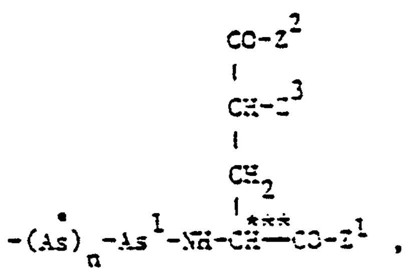

n représentant 0 ou 1, dans la formule (I) de la revendication 1, est choisie dans le groupe constitué par -Ala-D-Glu, -Ala-D-Glu-$NH_2$, -Ala-D-Glu($NH_2$), -Ala-D-Glu-D-Ala-$NH_2$, -Ala-D-Glu($NH_2$)-$NH_2$, -Ala-D-Glu-(Ala), -Ala-D-Glu($NH_2$)-D-Ala-$NH_2$, -Ala-D-Glu(Ala)-$NH_2$, -As° -Ala-D-Glu, -As° -Ala-D-Glu-$NH_2$, -As° -Ala-D-Glu($NH_2$), As° -Ala-D-Glu-D-Ala-$NH_2$, As° -Ala-D-Glu($NH_2$)-$NH_2$, As° -Ala-D-Glu(Ala), As° -Ala-D-Glu-($NH_2$)-D-Ala-$NH_2$ et As° -Ala-D-Glu(Ala)-$NH_2$ ou un sel d'un tel composé ayant au moins un groupe salifiable.

**19.** Procédé selon la revendication 18, caractérisé en ce que l'on choisit les produits de départ de telle façon que l'on prépare un composé de formule I où les restes acyles $R^1_a$ -CO- et $R^1_b$ -CO- sont différents de $R^2$-CO- et représentent le reste des acides caprylique, caprique, laurique, myristique, palmitique, stéarique ou oléique ou un sel d'un tel composé ayant au moins un groupe salifiable.

**20.** Procédé selon la revendication 2, caractérisé en ce que l'on choisit les produits de départ de telle façon que l'on prépare un composé de formule I choisi dans le groupe constitué par
le palmitoyl-Cys(2[R],3-dilauroyloxy-propyl)-Ala-D-Glu-$NH_2$,
le palmitoyl-Cys(2[R],3-dipalmitoyloxy-propyl)-Ala-D-Glu(Ala)-$NH_2$,
le palmitoyl-Cys(2[R],3-dilauroyloxy-propyl)-Ala-D-Glu ($NH_2$),
le palmitoyl-Cys(2[R],3-dilauroyloxy-propyl)-Abu-D-Glu-$NH_2$ (Abu = l'acide $\alpha$-amino-butyrique),
le palmitoyl-Cys(2[R],3-dilauroyloxy-propyl)-Ala-D-Glu ($NH_2$)-OnBu (nBu = n-butyle),
le palmitoyl-Cys(2[R],3-dilauroyloxy-propyl)-Ala-D-Glu (OnBu)-$NH_2$,
le palmitoyl-Cys(2[R],3-dilauroyloxy-propyl)-D-Ala-Ala-D-Glu-$NH_2$,

```
                                                        (D)
le palmitoyl-Cys(2[R],3-dilauroyloxy-propyl)-O-CH-CO-Ala-
                                                |
                                                CH3
    D-Glu-NH2,
```

le palmitoyl-Cys(2[R],3-dilauroyloxy-propyl)-O-$CH_2$CO-Ala-D-Glu-$NH_2$,
le palmitoyl-Cys(2[R],3-dilauroyloxy-propyl)-Ala-Do-Glu ($NH_2$)-O-$CH_2$-O-CO-C($CH_3$)$_3$,
le palmitoyl-Cys(2[R],3-dilauroyloxy-propyl)-Ser-D-Glu ($OCH_3$)-$OCH_3$,
le palmitoyl-Cys(2[R],3-dilauroyloxy-propyl)-Ala-D-Gla-$NH_2$ (Gla = l'acide $\gamma$ -carboxy-glutamique),
le palmitoyl-Cys(2[R],3-dilauroyloxy-propyl)-Abu-D-glu,
le palmitoyl-Cys(2[R],3-dilauroyloxy-propyl)-Val-D-Glu-$NH_2$,
le palmitoyl-Cys(2[R],3-dilauroyloxy-propyl)-$\alpha$MeAla-D-Glu-$NH_2$ ( $\alpha$MeAla = $\alpha$ -méthylalanine),
le palmitoyl-Cys(2[R],3-dilauroyloxy-propyl)-Ala-D-Glu-(Lys-$OCH_3$)-$NH_2$,
le palmitoyl-Cys(2[R],3-dilauroyloxy-propyl)-Ala-D-Glu(Lys-Lys-$OCH_3$)-$NH_2$,
le palmitoyl-Cys(2[R],3-dilauroyloxy-propyl)-Ala-D-Glu(Arg),
son ester mono- et diméthylique et son mono- et diamide,

```
le palmitoyl-Cys(2[R],3-dilauroyloxy-propyl)-Ala-D-
                (D)
     Glu(Lys-OCH-COOH)
              |
              CH3
```

son ester mono- et diméthylique et son mono- et diamide,
le palmitoyl-Cys(2[R],3-dilauroyloxy-propyl)-Ala-D-Glu(Tly) (Tly = 4-thia-lysine), son ester mono- et diméthylique et son mono- et diamide,
et les lipopeptides correspondants dans lesquels se trouvent, à la place du palmitoyle sur l'azote du reste cystéine, le lauroyle, le caprinoyle, le capryloyle ou le myristoyle et les composés correspondants aux lipoprotéines indiquées ci-dessus, dans lesquels se trouvent, dans le reste diacyloxypropyle à la place des restes lauroyles, les restes des acides palmitique, caprylique, caprique et myristique ainsi que les composés correspondant à tous ces lipopeptides, dans lesquels la configuration sur l'atome chiral du reste diacyloxypropyle est S au lieu de R et les mélanges de diastéréoisomères correspondants des composés R et S, ainsi qu'éventuellement leurs amides non substitués ou substitués ou un sel d'un tel composé ayant au moins un groupe salifiable.

**21.** Procédé selon la revendication 2, caractérisé en ce que l'on choisit les produits de départ de telle façon que l'on prépare un ester d'alcools aliphatiques ayant 1 à 7 atomes de C ou un ester d'alcools alcanoyle en $C_{1-7}$ oxyméthyliques, d'alcools alcanoyle en $C_{1-7}$ oxyéthyliques, d'alcools (cycloalkyles en $C_{3-8}$)-carbonyloxyméthyliques, d'alcools (cycloalkyles en $C_{3-8}$)-carbonyloxyéthyliques, du propylè-

neglycol, de la glycérine ou d'un alcoxy en $C_{1-7}$ phénol, d'un alkyle en $C_{1-7}$ aminophénol, d'un dialkyle en $C_{1-7}$ aminophénol ou d'un halogénophénol d'un lipopeptide cité dans la revendication 20.

**22.** Procédé selon la revendication 2, caractérisé en ce que l'on choisit les produits de départ de telle façon que l'on prépare un amide non substitué ou un amide d'alkyle en $C_{1-7}$ d'amines, la pyrrolidine, la pipéridine ou la pipérazine d'un des lipopeptides cités dans la revendication 20.

**23.** Procédé selon la revendication 2, caractérisé en ce que l'on choisit les produits de départ de telle façon que l'on prépare un composé de formule I, choisi dans le groupe constitué par
le lauroyl-Cys(2[R,S],3-didécanoyloxy-propyl)-Ala-D-Glu-$NH_2$,
le décanoyl-Cys(2[R,S],3-dilauroyloxy-propyl)-Ala-D-Glu-$NH_2$,
le myristoyl-Cys(2[R,S],3-dilauroyloxy-propyl)-Ala-D-Glu-$NH_2$,
le palmitoyl-Cys(2[R,S],3-didécanoyloxy-propyl)-Ala-D Glu-$NH_2$,
le palmitoyl-Cys(2[R,S],3-didécanoyloxy-propyl)-Abu-D-Glu($OCH_3$)-$OCH_3$,
et de leurs esters et amides selon l'une des revendications 21 et 23.

**24.** Procédé selon la revendication 1, caractérisé en ce que l'on choisit les produits de départ de telle façon que l'on prépare le palmitoyl-Cys(2[R],3-dilauroyloxy-propyl)-Ala-D-Glu(Gly-taurine)-$NH_2$ ou l'un de ses sels.

**25.** Procédé selon la revendication 1, caractérisé en ce que l'on choisit les produits de départ de telle façon que l'on prépare le palmitoyl-Cys(2[R],3-dilauroyloxy-propyl)-Ala-D-Glu-$NH_2$ ou l'un de ses sels.

**26.** Procédé selon la revendication 1, caractérisé en ce que l'on choisit les produits de départ de telle façon que l'on prépare un sel d'ammonium, un sel alcalin ou alcalino-terreux d'un des lipopeptides acides revendiqués dans les revendications 1 à 25 ou un sel d'addition d'acides non toxique, pharmaceutiquement utilisable, des lipopeptides basiques revendiqués dans les revendications précitées.